(19) Europäisches Patentamt
European Patent Office
Office européen des brevets

(11) Veröffentlichungsnummer: **0 089 926**
**A2**

(12) # EUROPÄISCHE PATENTANMELDUNG

(21) Anmeldenummer: **83810113.7**

(22) Anmeldetag: **18.03.83**

(51) Int. Cl.[3]: **C 07 D 471/14**
A 61 K 31/435
//(C07D471/14, 221/00, 209/00, 209/00)

(30) Priorität: **24.03.82 CH 1811/82**

(43) Veröffentlichungstag der Anmeldung:
**28.09.83 Patentblatt 83/39**

(84) Benannte Vertragsstaaten:
**AT BE CH DE FR GB IT LI LU NL SE**

(71) Anmelder: **CIBA-GEIGY AG**
**Patentabteilung Postfach**
**CH-4002 Basel(CH)**

(72) Erfinder: **Fröstl, Wolfgang, Dr.**
**St. Johanns-Vorstadt 6/2**
**CH-4056 Basel(CH)**

(72) Erfinder: **Storni, Angelo, Dr.**
**Im Feuerbusch 3**
**CH-4310 Rheinfelden(CH)**

(54) **Pharmazeutische Präparate enthaltend stickstoffhaltige Tetracyclen sowie neue Verbindungen dieser Art.**

(57) Phamazeutische Präparate enthaltend Derivate des Indolizino[8,7-b]indols der Formel

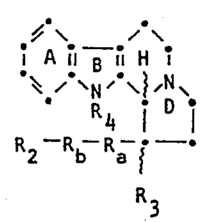

(I),

worin $R_a$ die Gruppe $-(CH_2)_m-$, worin m Null oder 1 ist, $R_b$ die Gruppe $-CH(R_1)-$ darstellt, worin $R_1$ Wasserstoff, Niederalkyl, gegebenenfalls verestertes oder veräthertes Hydroxy, Niederalkylthio, oder gegebenenfalls substituiertes Amino, $R_2$ gegebenenfalls verestertes oder veräthertes Hydroxymethyl oder gegebenenfalls funktionell abgewandeltes Carboxy, $R_3$ und $R_4$ jeweils Wasserstoff oder Niederalkyl bedeutet, oder anstelle der Gruppe $-(R_b-R_a)-$ die Gruppe $-CH=CH-$ steht, une worin der carbocyclische Ring A unsubstituiert ist oder unsubstituiertes oder substituiertes Niederalkyl, gegebenenfalls verestertes oder veräthertes Hydroxy, Nitro und/oder gegebenenfalls substituiertes Amino als Substituenten enthält, sowie N-Oxide solcher Verbindungen, und deren Salze, sowie solche Verbindungen als quaternäre Ammoniumverbindungen oder deren Salze, sowie neue Verbindungen der Formel I oder deren N-Oxide oder deren Salze, oder solche Verbindungen als quaternäre Ammoniumverbindungen oder deren Salze, erscheinen zur Behandlung von Vigilanzstörungen und zerebraler Leistungsinsuffizienz, insbesondere von Gedächtnisstörungen verschiedener Genese, wie senile Demenz, Multiinfarkt-Demenz oder Demenz vom Alzheimer-Typ, ferner von Folgezuständen von Hirntraumen oder Apoplexie geeignet.

EP 0 089 926 A2

Croydon Printing Company Ltd.

0089926



CIBA-GEIGY AG
Basel (Schweiz)

4-13860/+

# Pharmazeutische Präparate enthaltend stickstoffhaltige Tetracyclen sowie neue Verbindungen dieser Art

Die Erfindung betrifft pharmazeutische Präparate enthaltend neue Derivate des Indolizino[8,7-b]indols, und ihre Verwendung als pharmazeutisch aktive Verbindungen, ferner neue Verbindungen dieser Art und Verfahren zu ihrer Herstellung.

Die erfindungsgemässen pharmazeutischen Präparate enthalten Verbindungen der Formel

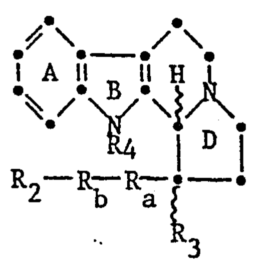

(I),

worin $R_a$ die Gruppe $-(CH_2)_m-$, worin m Null oder 1 ist, $R_b$ die Gruppe $-CH(R_1)-$ darstellt, worin $R_1$ Wasserstoff, Niederalkyl, gegebenenfalls verestertes oder veräthertes Hydroxy, Niederalkylthio, oder gegebenenfalls substituiertes Amino, $R_2$ gegebenenfalls verestertes oder veräthertes Hydroxymethyl oder gegebenenfalls funktionell abgewandeltes Carboxy, $R_3$ und $R_4$ jeweils Wasserstoff oder Niederalkyl bedeutet, oder anstelle der Gruppe $-(R_b-R_a)-$ die Gruppe $-CH=CH-$ steht, und worin der carbocyclische Ring A unsubstituiert ist, oder unsubstituiertes oder substituiertes Niederalkyl, gegebenenfalls verestertes oder veräthertes Hydroxy, Nitro und/oder gegebenenfalls substituiertes Amino als Substituenten enthält, sowie Salze oder N-Oxide davon, oder solche Verbindungen als quaternäre Ammoniumverbindungen oder deren Salze.

Den Verbindungen der Formel I liegt das Ringsystem der Formel Ia zugrunde, für welches folgende Stellungsnumerierungen verwendet werden:

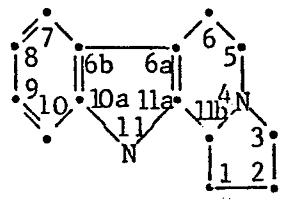

(Ia).

Das obige Ringsystem enthält demnach einen carbocyclischen und drei monoazacyclische Ringe.

Die Erfindung betrifft ebenfalls die neuen Verbindungen der Formel I, worin $R_a$, m, $R_b$, $R_2$, $R_3$ und $R_4$ die angegebenen Bedeutungen haben, und der carbocyclische Ring A wie angegeben substituiert sein kann, mit der Massgabe, dass in Verbindungen der Formel I, in welchen $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ Hydroxymethyl oder Benzyloxymethyl und $R_3$ die Aethylgruppe bedeutet, und $R_4$ Wasserstoff ist, der carbocyclische Ring A unsubstituiert ist, und mit der weiteren Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Aethyl ist, $R_2$ Hydroxymethyl oder Benzyloxymethyl bedeutet, und $R_3$ und $R_4$ jeweils Wasserstoff ist, der carbocyclische Ring A unsubstituiert ist, und mit der weiteren Massgabe, dass in Verbindungen der Formel I, worin $R_a$ eine Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ die Carbomethoxygruppe oder die Cyanogruppe bedeutet und $R_3$ und $R_4$ jeweils Wasserstoff ist, der carbocyclische Ring A unsubstituiert ist, N-Oxide oder Salze davon, oder solche Verbindungen als quaternäre Ammoniumverbindungen oder deren Salze.

Funktionell abgewandeltes Carboxy ist verestertes, etwa aliphatisch, cycloaliphatisch oder aromatische verestertes Carboxy und stellt in erster Linie Niederalkoxycarbonyl, Niederalkylthiocarbonyl oder substituiertes Niederalkoxycarbonyl, wie gegebenenfalls veräthertes oder verestertes Hydroxy oder gegebenenfalls substituiertes Amino enthaltendes Niederalkoxycarbonyl, worin Substituenten vorzugsweise durch mindestens zwei Kohlenstoffatome vom Oxy-Sauerstoff getrennt sind, z.B. Hydroxyniederalkoxycarbonyl, Aminoniederalkoxycarbonyl, Niederalkylaminoniederalkoxycarbonyl, Niederalkoxyniederalkoxycarbonyl, Diniederalkylamino-niederalkoxycarbonyl, Niederalkylenamino-niederalkoxycarbonyl, Oxaniederalkylenamino-niederalkoxycarbonyl, Thianiederalkylenamino-niederalkoxycarbonyl oder Azaniederalkylenamino-niederalkoxycarbonyl, worin das Azastickstoffatom unsubstituiert oder, z.B. durch Niederalkyl, substituiert sein kann, ferner gegebenenfalls durch Niederalkyl oder Niederalkoxy substituiertes Cycloalkyloxycarbonyl, wobei Substituenten mehrfach, insbesondere 1 bis 3 mal, vorhanden sein können, ferner gegebenenfalls partiell hydriertes Benzofuranyloxycarbonyl wie 1,3-Dihydro-1-oxo-isobenzofuranyloxycarbonyl, dar.

Funktionell abgewandeltes Carboxy kann auch amidiertes Carboxy sein, wie Carbamoyl, N-Niederalkylcarbamoyl, N,N-Diniederalkylcarbamoyl, gegebenenfalls im Phenylteil substituiertes, etwa durch Niederalkyl, Niederalkoxy, Halogen und/oder Nitro substituiertes N-Phenylniederalkylcarbamoyl, Niederalkylenaminocarbonyl, Oxaniederalkylenaminocarbonyl, Thianiederalkylenaminocarbonyl oder Azaniederalkylenaminocarbonyl, worin das Azastickstoffatom unsubstituiert oder, z.B. durch Niederalkyl, substituiert sein kann, ferner die Cyanogruppe. Amidiertes Carboxy kann ausserdem Hydrazinocarbonyl oder N'-substituiertes, wie N'-niederalkyliertes oder N'-acyliertes Hydrazinocarbonyl, z.B. N,N'-Niederalkylhydrazinocarbonyl, N,N'-Niederalkanoyl-hydrazinocarbonyl oder N,N'-Aroyl-hydrazinocarbonyl sein, wobei Aroyl insbesondere im Phenylteil gegebenenfalls durch Niederalkyl, Niederalkoxy, Halogen und/oder Nitro substituiertes Benzoyl oder Nicotinoyl darstellt.

Veräthertes Hydroxy ist z.B. Niederalkoxy, kann jedoch auch für Phenylniederalkoxy, und, als Substituent des carbocyclischen Ringes A, für Niederalkyliden- oder Niederalkylendioxy stehen, während verestertes Hydroxy acyliertes Hydroxy, z.B. Halogen oder mit einer aliphatischen oder aromatischen Carbon- oder Sulfonsäure verestertes Hydroxy, z.B. Aroyloxy, etwa Benzoyloxy, oder Benzolsulfonyloxy, dessen aromatischer Teil jeweils unsubstituiert oder z.B. durch Niederalkyl, Niederalkoxy, Halogen und/oder Nitro substituiert sein kann, wobei Substituenten der genannten Art gleich oder verschieden und jeweils mehrfach, insbesondere 1 bis 3-fach, vorhanden sein können, oder Niederalkanoyloxy, z.B. Pivaloyloxy, sein kann.

Substituiertes Amino ist z.B. Niederalkylamino, Diniederalkylamino, Niederalkylenamino, Oxaniederalkylenamino, Thianiederalkylenamino, oder Azaniederalkylenamino, worin das Azastickstoffatom unsubstituiert oder, z.B. durch Niederalkyl, substituiert sein kann, ferner acyliertes Amino, wie Niederalkanoylamino oder Niederalkoxycarbonylamino.

Substituiertes Niederalkyl enthält in erster Linie Halogen, ferner Hydroxy, Niederalkoxy oder Phenylniederalkoxy als Substituenten.

Verestertes Hydroxymethyl ist z.B. solches, worin die veresterte Hydroxygruppe obige Bedeutung hat und z.B. Halogen oder gegebenenfalls, z.B. wie angegeben, substituiertes Aroyloxy, wie Benzoyloxy, oder Benzolsulfonyloxy, ferner Niederalkanoyloxy darstellt.

Veräthertes Hydroxymethyl ist z.B. solches, worin die verätherte Hydroxygruppe beispielsweise Niederalkoxy ist.

Die für den Ring A genannten Substituenten können darin mehrfach, vorzugsweise bis zu 4 mal, vorhanden sein.

Die oben angegebenen Bedeutungen können die folgenden Bedeutungen haben, wobei mit "nieder" bezeichnete Substituenten und Verbindungen bis und mit 7, insbesondere bis und mit 4 Kohlenstoffatome enthalten

und/oder substituierte Gruppen einen oder mehrere Substituenten aufweisen können.

Niederalkoxycarbonyl ist z.B. Methoxycarbonyl, Aethoxycarbonyl, n-Propyloxycarbonyl, Isopropyloxycarbonyl, n-Butyloxycarbonyl oder tert.-Butyloxycarbonyl.

Cycloalkyloxycarbonyl hat 3-7, insbesondere 5-6, Ringglieder im Cycloalkylteil und ist z.B. Cyclohexyloxycarbonyl oder Trimethylcyclohexyloxycarbonyl.

Niederalkoxyniederalkoxycarbonyl ist z.B. 2-Methoxyäthoxycarbonyl oder Methoxymethoxycarbonyl.

In substituierten Niederalkoxycarbonylgruppen sind die Substituenten vorzugsweise durch mehr als ein Kohlenstoffatom vom Oxy-Sauerstoff getrennt und bedeuten z.B. 2-Hydroxyäthyl-oxycarbonyl, 2-Methoxyäthyl-oxycarbonyl, 2-Aethoxyäthyl-oxycarbonyl, 2-Chloräthyl-oxycarbonyl, 2-Aminoäthyl-oxycarbonyl, 2-Methylaminoäthyl-oxycarbonyl, 2-Dimethylaminoäthyl-oxycarbonyl, 2-Diäthylaminoäthyl-oxycarbonyl, 3-Dimethylaminopropyl-oxycarbonyl, 2-Pyrrolidinoäthyl-oxycarbonyl, 2-(4-Morpholino)-äthyl-oxycarbonyl, 2-(4-Thiamorpholino)-äthyl-oxycarbonyl oder 2-(4-Methly-piperazino)-äthyl-oxycarbonyl.

Niederalkylthiocarbonyl ist z.B. Methylthiocarbonyl, Aethylthiocarbonyl, n-Propylthiocarbonyl, Isopropylthiocarbonyl, n-Butylthiocarbonyl oder tert.Butylthiocarbonyl.

Niederalkoxy ist z.B. Methoxy, Aethoxy, n-Propyloxy, Isopropyloxy oder n-Butyloxy, und Phenylniederalkoxy, z.B. Benzyloxy, während Niederalkylidendioxy oder Niederalkylendioxy z.B. Methylendioxy oder 1,1-Aethylidendioxy ist.

Phenylniederalkoxy ist z.B. Benzyloxy.

Halogen ist z.B. Fluor, Chlor oder Brom, d.h. Halogen mit einer Atomnummer bis zu 35, kann jedoch auch Jod sein.

Aroyloxy ist z.B. gegebenenfalls 1-3 fach durch Niederalkyl oder Niederalkoxy, wie Methyl oder Methoxy, substituiertes Benzoyloxy, wie 3,4,5-Trimethoxybenzoyloxy oder 4-Methylbenzolsulfonyloxy.

Niederalkanoyloxy ist z.B. Acetyloxy, Propionyloxy, Pivaloyloxy oder Butyryloxy.

Niederalkyl ist z.B. Methyl, Aethyl, n-Propyl, Isopropyl, n-Butyl, Isobutyl oder tert.-Butyl, ferner n-Pentyl, Neopentyl, n-Hexyl oder n-Heptyl.

Phenylniederalkyl ist z.B. Benzyl oder 2-Phenyläthyl.

Phenoxyniederalkyl ist z.B. Phenoxymethyl oder 2-Phenoxyäthyl.

Phenylthioniederalkyl ist z.B. Phenylthiomethyl oder 2-Phenylthioäthyl.

Niederalkylthio ist z.B. Methylthio oder Aethylthio.

Niederalkylamino ist z.B. Methylamino oder Aethylamino, während Diniederalkylamino z.B. Dimethylamino, N-Aethyl-N-Methylamino oder Diäthylamino, und Niederalkylenamino z.B. Pyrrolidino oder Piperidino ist.

In Oxaniederalkylenamino, Thianiederalkylenamino und Azaniederalkylenamino ist das Heteroatom vom Aminostickstoffatom durch mindestens zwei Kohlenstoffatome getrennt; die Reste können z.B. 4-Morpholino, 4-Thiamorpholino, 4-Piperazino oder 4-Niederalkylpiperazino bedeuten.

Halogen-niederalkyl ist in erster Linie Trifluormethyl, während Hydroxyniederalkyl z.B. Hydroxymethyl, und Niederalkoxyniederalkyl z.B. 2-Methoxyäthyl sein kann.

Acyliertes Amino ist z.B. Aroylamino, wie Benzoylamino, dessen aromatischer Teil unsubstituiert sein oder die bei Aroyloxy angegebenen Substituenten, z.B. Niederalkyl, Niederalkoxy, Halogen oder funktionell abgewandeltes Carboxy, ein oder mehrfach, insbesondere 1-3 fach enthalten und demnach z.B. Benzoyl- oder 3,4,5-Trimethyloxybenzoylamino sein kann.

Niederalkanoylamino ist z.B. Acetylamino, Propionylamino oder Pivaloylamino.

1,3-Dihydro-1-oxo-isobenzofuranyloxycarbonyl ist z.B. 1,3-Dihydro-1-oxo-3-isobenzofuranyloxycarbonyl.

N-Phenylniederalkylcarbamoyl ist z.B. N-(2-Phenyläthyl)-carbamoyl.

N,N'-Niederalkylhydrazinocarbonyl ist z.B. N,N'-Methylhydrazinocarbonyl oder N,N',N'-Dimethylhydrazinocarbonyl.

N,N'-Aroylhydrazinocarbonyl ist z.B. N,N'-(4-Methoxybenzoyl)-hydrazinocarbonyl oder N,N'-(2-Nicotinoyl)-hydrazinocarbonyl.

Die Verbindungen der Formel I können als Epimerengemische, reine Epimere, Racematgemische, Racemate, optische Antipoden, Gemische von cis- und trans-Isomeren, reine cis- oder trans-Isomere, N-Oxide oder Salze, insbesondere Säureadditionssalze, vor allem pharmazeutisch verwendbare, nicht-toxische Säureadditionssalze, sowie als quaternäre Ammoniumverbindungen oder deren Salze vorliegen.

Quaternäre Ammoniumverbindungen enthalten als quaternären Substituenten gegebenenfalls z.B. wie angegeben, substituiertes Niederalkyl, z.B. Niederalkyl, Hydroxy- oder Halogeniederalkyl, Phenylniederalkyl, Phenoxyniederalkyl oder Phenylthioniederalkyl, worin der Phenylteil jeweils unsubstituiert oder durch Niederalkyl, Niederalkoxy, Halogen, Trifluormethyl und/oder Nitro substituiert sein kann. Salze von quaternären Ammoniumverbindungen entsprechen den nachfolgend definierten,

speziell den als pharmazeutisch verwendbaren, nicht-toxischen Säureadditionssalzen im Einzelnen angegebenen Salzen, insbesondere den mit Halogenwasserstoffsäuren, Schwefel- oder Phosphorsäuren, gebildeten Salzen.

Die neuen Verbindungen können in Form ihrer Salze wie ihrer Säureadditionssalze und in erster Linie ihrer pharmazeutisch verwendbaren, nicht-toxischen Säureadditionssalze vorliegen. Geeignete Salze sind z.B. solche mit anorganischen Säuren, wie Halogenwasserstoffsäuren, z.B. Chlorwasserstoffsäure oder Bromwasserstoffsäure, Schwefelsäure, oder Phosphorsäure, oder mit organischen Säuren, wie aliphatischen, cycloaliphatischen, aromatischen oder heterocyclischen Carbon- oder Sulfonsäuren, z.B. Ameisen-, Essig-, Propion-, Bernstein-, Glykol-, Milch-, Aepfel-, Wein-, Zitronen-, Malein-, Hydroxymalein-, Brenztrauben-, Fumar-, Benzoe-, 4-Aminobenzoe-, Anthranil-, 4-Hydroxybenzoe-, 2,4-, 2,5-, 2,6- oder 3,5-Dihydroxybenzoesäure, Salicyl-, Acetylsalicyl- oder Embonsäure, 4-Acetamidobuttersäure, Furan-2-carbonsäure, 3-(Theophyllin-7-yl)-propansulfonsäure, Theophyllin-7-essigsäure, Nicotinsäure, 5-Bromnicotinsäure, Indol-3-essigsäure, Methansulfon-, Aethansulfon-, 2-Hydroxyäthansulfon-, Aethylensulfon-, Toluolsulfon-, Naphthalinsulfon-, Camphersulfon-, Sulfanilsäure, oder N-Cyclohexylsulfaminsäure oder mit anderen sauren organischen Stoffen, wie Alginin-, Ascorbin- oder Gerbsäure, sauren Estern der Phosphorsäure, wie Glucose-1- oder 6-phosphat, 5-Pyridoxalphosphat, Saccharin, saure Gruppen, z.B. Carbonsäure- und/oder insbesondere Sulfonsäure-Gruppen enthaltende Kationenaustauscherharze, z.B. Sulfonierungsprodukte von Copolymeren von gegebenenfalls indifferent substituierten Styrolen und geringen Mengen Divinylbenzol als Vernetzungsmittel, oder Kondensationsprodukte von Phenolen oder Phenoläthern mit Formaldehyd und gegebenenfalls substituierten Benzolsulfonsäuren.

Die neuen Verbindungen der Formel I besitzen wertvolle pharmakologische Eigenschaften. Insbesondere wirken sie auf das Zentralnervensystem.

Beispielsweise bewirken sie einen Schutz vor der amnesiogenen Wirkung von zerebralem Elektroschock und eine Verbesserung der Gedächtnisleistung. Befunde der genannten Art können in folgendem Lerntest ermittelt werden:

Die Testvorrichtung besteht aus einer grossen Box (35 x 20 x 10 cm), die durch eine Schiebetür mit einer kleinen Box (10 x 10 x 18 cm) verbunden ist. Während die kleine Box durch eine 100 Watt Lampe von oben hell erleuchtet wird, ist die grosse Box dunkel. Der Boden beider Abteile besteht aus einem elektrisch leitenden Gitterrost. Zum Training werden Gruppen von jeweils 10 männlichen Mäusen (20-22 g) einzeln in die hell erleuchtete kleine Box gesetzt. Da Mäuse eine Spontanpräferenz für Dunkel haben, gehen die meisten innerhalb von 30 Sekunden ins dunkle Abteil. Sobald sie dieses vollständig betreten haben, wird die Schiebetür geschlossen und ein Fussschock (1 mA, 5 Sekunden) verabreicht. Die Tiere werden darauf sofort aus dem Versuchsapparat entfernt.

Zur Prüfung des Lerneffektes (Retest) werden die Mäuse nach 24 Stunden wiederum einzeln ins helle Abteil gesetzt und die Zeitspanne, bis alle sich ganz im dunklen Abteil befinden, gemessen. Ueblicherweise bleiben nun die meisten Tiere über die ganze Beobachtungszeit von 150 Sekunden im hellen Abteil. Der Lerneffekt wird weitgehend aufgehoben bzw. die Erinnerung an den Fussschock mindestens partiell ausgelöscht, wenn als amnesiogene Behandlung unmittelbar anschliessend an den Fussschock des Trainingsdurchgangs eine temporale Elektroschockbehandlung angeschlossen wird. Parameter des Elektroschocks: 50 mA, 0,4 Sekunden, 50 Hz., 0,6 Sekunden (Pulsdauer).

Zur Prüfung und zum Vergleich ihrer Schutzwirkung gegenüber der amnesiogenen Wirkung des Elektroschocks werden die Testsubstanzen 30 Minuten vor dem Training in Dosen von 0,1 mg/kg, 1 mg/kg, 10 mg/kg und 100 mg/kg (für jede Dosis werden 10 Mäuse verwendet) je nach Löslichkeit als Lösung in physiologischer NaCl-Lösung oder einer 0,5 %igen

Lösung von Methylcellulose in Wasser intraperitoneal verabreicht und die Tiere unmittelbar nach dem Training der Elektroschockbehandlung unterzogen. 24 Stunden später wird anhand der Verweildauer in der beleuchteten Box das Ausmass des noch erhalten gebliebenen Lerneffektes gemessen und mit demjenigen der anderen Testsubstanzen wie auch von Kontrolltieren mit alleiniger Verabreichung des jeweils verwendeten Lösungsmittels und Training ohne, wie auch solchen mit anschliessender Elektroschockbehandlung, verglichen. Die oben erwähnten Wirksamkeiten lassen sich an den neuen Verbindungen der Formel I, beispielsweise dem <u>erythro</u>-<u>cis</u>-α-Hydroxy-1-äthyl-2,3,5,6,11,11b-hexahydro-1H-indolizino[8,7-b]indol-1-propionsäuremethylester oder dem <u>cis</u>-1-Aethyl-2,3,5,6,11,11b-hexahydro-1H-indolizino[8,7-b]indol-1-propionsäuremethylester, in einem Dosenbereich von etwa 0,1 mg/kg bis 100 mg/kg nachweisen.

Zur unmittelbaren Messung der Verbesserung der Gedächtnisleistung wird folgende Versuchsmethodik verwendet:
Die Testvorrichtung besteht aus einer mit Tageslicht normal beleuchteten Box (50 x 50 x 50 cm) mit einem elektrisch leitenden Gitterrost, dessen Gitterstäbe (6 mm Durchmesser) in einem Abstand von jeweils 13 mm angeordnet sind. In der Mitte des Gitterrostes ist eine hölzerne Plattform (10 mm Höhe, 67 mm Durchmesser) angebracht, welche von einer Plastikröhre (20 cm hoch, 68 mm innerer Durchmesser) umgeben ist. Zur Durchführung des Trainings werden Gruppen von jeweils 30 männlichen Mäusen (20 - 25 g) verwendet, indem jeweils ein Tier auf die von den Plastikröhre umgebene Plattform gesetzt, nach 10 Sekunden die Plastikröhre entfernt und die Zeitdauer gemessen wird, welche das Tier zum Hinabsteigen und Berühren des Gitterrostes mit allen 4 Pfoten benötigt. Wenn alle 4 Pfoten den Gitterrost berühren, wird ein Fussschock (1 mA, 50 Hz) verabreicht. Innerhalb von 10 Sekunden nach Verabreichung des Fussschocks werden die Testsubstanzen in Dosen von 0,1 mg/kg, 1 mg/kg und 10 mg/kg (für jede Dosis werden 30 Mäuse verwendet) je nach Löslichkeit als Lösung in physiologischer NaCl-Lösung

oder einer 0,5%-igen Lösung von Methylcellulose in Wasser intraperitoneal verabreicht. 24 Stunden später wird anhand der Verweildauer jedes einzelnen Tieres auf der Plattform das Ausmass der Verbesserung oder Verschlechterung des Lerneffekts im Vergleich zu den Tieren, denen zur Kontrolle lediglich das Lösungsmittel appliziert worden war, ermittelt. So lässt sich an den neuen Verbindungen der Formel I, beispielsweise nach i.p.-Verabreichung von 1 mg/kg erythro-cis-α-Hydroxy-1-äthyl-2,3,5,6,11,11b-hexahydro-1H-indolizino[8,7-b]indol-1-propionsäuremethylester oder cis-1-Aethyl-2,3,5,6,11,11b-hexahydro-1H-indolizino[8,7-b]indol-1-propionsäuremethylester an Mäusen eine Verbesserung der Gedächtnisleistung zeigen.

Aufgrund dieser Eigenschaften erscheinen die neuen Verbindungen der Formel I zur Behandlung von Vigilanzstörungen und zerebraler Leistungsinsuffizienz, insbesondere von Gedächtnisstörungen verschiedener Genese, wie senile Demenz, Multiinfarkt-Demenz oder Demenz vom Alzheimer-Typ, ferner von Folgezuständen von Hirntraumen oder Apoplexie geeignet.

Die Erfindung betrifft insbesondere pharmazeutische Präparate enthaltend Verbindungen der Formel I, worin $R_a$, $R_b$ und m sowie die Gruppe der Formel $-(R_b-R_a)-$ die angegebenen Bedeutungen haben, $R_1$ Wasserstoff, Niederalkyl, Hydroxy, Halogen, Aroyloxy, Niederalkanoyloxy, Niederalkoxy, Phenylniederalkoxy, Niederalkylthio, Amino, N-Niederalkylamino, N,N-Diniederalkylamino, Niederalkylenamino, Oxaniederalkylenamino, Thianiederalkylenamino, Azaniederalkylenamino, worin das Azastickstoffatom unsubstituiert oder durch Niederalkyl substituiert sein kann, oder Niederalkanoylamino darstellt, $R_2$ für Hydroxymethyl, Aroyloxymethyl, Benzolsulfonyloxymethyl, Carboxy, Niederalkoxycarbonyl, Hydroxyniederalkoxycarbonyl, Niederalkoxy-niederalkoxycarbonyl, Halogen-niederalkoxycarbonyl, Aminoniederalkoxycarbonyl, Niederalkylaminoniederalkoxycarbonyl, Diniederalkylamino-niederalkoxycarbonyl, Niederalkylenamino-niederalkoxycarbonyl, Oxaniederalkylen-aminoniederalkoxycarbonyl, Thianiederalkylenamino-niederalkoxycarbonyl, oder Azaniederalkylenamino-niederalkoxycarbonyl, worin das Azastickstoffatom unsubstituiert oder durch Niederalkyl substituiert sein kann, Niederalkylthiocarbonyl, gegebenenfalls durch Niederalkyl oder Niederalkoxy 1 bis 3-fach substituiertes Cycloalkyloxycarbonyl, Benzofuranyloxycarbonyl, 1,3-Dihydro-1-oxo-3-isobenzofuranyloxycarbonyl, Carbamoyl, N-Niederalkyl-carbamoyl, N,N-Diniederalkyl-carbamoyl, Niederalkylenamino-carbonyl, Oxaniederalkylenamino-carbonyl, Thianiederalkylenamino-carbonyl oder Azaniederalkylenamino-carbonyl steht, worin das Azastickstoffatom unsubstituiert oder durch Niederalkyl substituiert sein kann, Hydrazinocarbonyl, N,N'-Niederalkylhydrazinocarbonyl, N,N'-Niederalkanylhydrazinocarbonyl, gegebenenfalls im Phenylteil durch Niederalkyl, Niederalkoxy, Halogen und/oder Nitro substituiertes N,N'-Benzoylhydrazinocarbonyl, N,N'-Nicotinoylhydrazinocarbonyl oder die Cyanogruppe, $R_3$ und $R_4$ jeweils Wasserstoff oder Niederalkyl bedeuten, und worin der carbocyclische Ring A unsubstituiert ist oder Niederalkyl, Halogenniederalkyl, Hydroxyniederalkyl, Niederalkoxyniederalkyl, Hydroxy, Niederalkoxy, Phenylniederalkoxy, Niederalkyliden- oder Niederalkylendioxy, Halogen, Niederalkanoyloxy, Nitro, Amino, N-Niederalkylamino, N,N-Diniederalkylamino, Niederalkylenamino, Oxaniederalkylenamino, Thianiederalkylenamino, Azaniederalkylenamino, worin das Azastickstoffatom unsubstituiert oder durch Niederalkyl substituiert sein kann, und/oder Niederalkanoylamino jeweils 1-3 fach als Substituenten enthält, als Epimerengemische, reine Epimere, Racematgemische, Racemate, optische Antipoden, Gemische von cis- und trans-Isomeren, reine cis- oder trans-Isomere, N-Oxide oder Salze, insbesondere Säureadditionssalze, vor allem pharmazeutisch verwendbare nicht-toxische Säureadditionssalze davon, oder solche Verbindungen als quaternäre Ammoniumverbindungen oder deren Salze.

Die Erfindung betrifft insbesondere pharmazeutische Präparate enthaltend Verbindungen der Formel I, worin $R_a$, $R_b$ und m sowie die Gruppe der Formel $-(R_b-R_a)-$ die angegebenen Bedeutungen haben, $R_1$ Wasserstoff, Hydroxy, Niederalkoxy, Aroyloxy, Niederalkanoyloxy, Amino, N-Niederalkylamino, N,N-Diniederalkylamino oder Niederalkanoylamino bedeutet, $R_2$ Hydroxymethyl, Aroyloxymethyl, Carboxy, Niederalkoxycarbonyl, Niederalkoxy-niederalkoxycarbonyl, Niederalkylthiocarbonyl, Diniederalkylaminoniederalkoxycarbonyl, Carbamoyl, N-Niederalkylcarbamoyl, Hydrazinocarbonyl, N,N'-Benzoylhydrazinocarbonyl, N,N'-Nicotinoylhydrazinocarbonyl oder die Cyanogruppe oder N,N-Diniederalkyl-carbamoyl darstellt, $R_3$ und $R_4$ jeweils Wasserstoff oder Niederalkyl ist, und worin der carbocyclische Ring A unsubstituiert oder durch Niederalkyl, Trifluormethyl, Hydroxy, Niederalkoxy, Phenylniederalkoxy, Niederalkylendioxy, Halogen, Niederalkanoyloxy, Nitro, Amino und/oder Niederalkanoylamino jeweils 1-3 fach substituiert ist, wobei Niederalkoxy, Niederalkyl, Niederalkanoyl und Niederalkanoylamino jeweils bis und mit 4 Kohlenstoffatome enthalten, als Epimerengemische, reine Epimere, Racematgemische, Racemate, optische Antipoden, Gemische von cis - und trans-Isomeren, reine cis- oder trans-Isomere, N-Oxide oder Salze, insbesondere Säureadditionssalze, vor allem pharmazeutisch verwendbare, nicht-toxische Säureadditionssalze davon, oder solche Verbindungen als quaternäre Ammoniumverbindungen, worin die quartäre Gruppe Niederalkyl, Hydroxyniederalkyl, Halogenniederalkyl, Phenylniederalkyl oder Phenoxyniederalkyl ist, oder deren Salze.

Die Erfindung betrifft insbesondere pharmazeutische Präparate enthaltend Verbindungen der Formel I, worin $R_a$, $R_b$ und m sowie die Gruppe der Formel $-(R_b-R_a)-$ die angegebenen Bedeutungen haben, $R_1$ Wasserstoff, Hydroxy, Benzoyloxy, Niederalkanoyloxy oder Amino bedeutet, $R_2$ für Hydroxymethyl, im aromatischen Teil gegebenenfalls durch Niederalkyl, Niederalkoxy, Halogen oder funktionell abgewandeltes Carboxy substituiertes Benzoyloxymethyl, Carboxy, Niederalkoxycarbonyl, wie Methoxycarbonyl oder Aethoxycarbonyl, Niederalkoxyniederalkoxycarbonyl wie Methoxymethoxycarbonyl, Niederalkylthiocarbonyl, wie Aethylthiocarbonyl, Carbamoyl, Hydrazinocarbonyl oder die Cyanogruppe steht, $R_3$ und $R_4$ jeweils Wasserstoff oder Niederalkyl bedeuten, und worin der carbocyclische Ring A unsubstituiert oder, vorzugsweise in den Stellungen

8 und/oder 9, durch Niederalkyl, z.B. Methyl, Hydroxy, Niederalkoxy, z.B. Methoxy, Phenylniederalkoxy, z.B. Benzyloxy, Niederalkylendioxy, z.B. Methylendioxy, und/oder Halogen, z.B. Brom, substituiert ist, wobei Niederalkyl und Niederalkoxy jeweils bis und mit vier Kohlenstoffatome enthalten, als Epimerengemische, reine Epimere, Racematgemische, Racemate, optische Antipoden, Gemische von cis- und trans-Isomeren, reine cis- oder trans-Isomere, N-Oxide oder Salze, insbesondere Säureadditionssalze, vor allem pharmazeutisch verwendbare, nichttoxische Säureadditionssalze davon, oder solche Verbindungen als quaternäre Ammoniumverbindungen, worin die quartäre Gruppe Niederalkyl oder Phenylniederalkyl ist, oder deren Salze.

Die Erfindung betrifft insbesondere pharmazeutische Präparate enthaltend Verbindungen der Formel I, worin $R_a$ die Gruppe der Formel $-(CH_2)_m-$ bedeutet, wobei m für 1 steht, $R_b$ die Gruppe der Formel $-CH(R_1)-$ darstellt, wobei $R_1$ Wasserstoff, Hydroxy, Benzoyloxy oder Acetyloxy ist, $R_2$ Hydroxymethyl, gegebenenfalls durch Niederalkyl, wie Methyl oder Niederalkoxy, wie Methoxy oder Carboxy substituiertes Benzoylmethyl, z.B. 3,4,5-Trimethoxybenzoylmethyl, Carboxy, Niederalkoxycarbonyl, insbesondere Methoxycarbonyl oder Aethoxycarbonyl, Niederalkoxy-niederalkoxycarbonyl wie Methoxy-methoxycarbonyl, Niederalkylthiocarbonyl wie Aethylthiocarbonyl, Carbamoyl oder die Cyanogruppe bedeutet, $R_3$ Wasserstoff oder Niederalkyl und $R_4$ Wasserstoff bedeutet, und worin der carbocyclische Ring A unsubstituiert oder in 8- und/oder 9-Stellung durch Hydroxy oder Niederalkoxy, z.B. Methoxy oder Methylendioxy und/oder Halogen, wie Brom, substituiert ist, wobei in diesen Verbindungen das Wasserstoffatom in 11b-Stellung und der Rest $R_3$, insbesondere Aethyl, in 1-Stellung in der trans- oder vorzugsweise in der cis-Konfiguration zueinander stehen, als Epimerengemische, reine Epimere, Racematgemische, Racemate, optische Antipoden, Gemische von cis- und trans- Isomeren, reine cis- oder trans-Isomere, N-Oxide oder Salze, insbesondere Säureadditionssalze, vor allem pharmazeutisch verwendbare Säureadditionssalze davon, oder solche Verbindungen als

quaternäre Ammoniumverbindungen, worin die quartäre Gruppe Niederalkyl, z.B. Methyl ist, oder deren Salze.

Die Erfindung betrifft in erster Linie pharmazeutische Präparate enthaltend die in den Beispielen beschriebenen neuen Verbindungen der Formel I.

Die Erfindung betrifft Verbindungen der Formel I, worin $R_a$ die Gruppe $-(CH_2)_m-$, worin m Null oder 1 ist, $R_b$ die Gruppe $-CH(R_1)-$ darstellt, worin $R_1$ Wasserstoff, Niederalkyl, gegebenenfalls verestertes oder veräthertes Hydroxy, Niederalkylthio, oder gegebenenfalls substituiertes Amino, $R_2$ gegebenenfalls verestertes oder veräthertes Hydroxymethyl oder gegebenenfalls funktionell abgewandeltes Carboxy, $R_3$ und $R_4$ jeweils Wasserstoff oder Niederalkyl bedeutet, oder anstelle der Gruppe $-(R_b-R_a)-$ die Gruppe $-CH=CH-$ steht, und worin der carbocyclische Ring A unsubstituiert ist, oder unsubstituiertes oder substituiertes Niederalkyl, gegebenenfalls verestertes oder veräthertes Hydroxy, Nitro und/oder gegebenenfalls substituiertes Amino als Substituenten enthält, mit der Massgabe, dass in Verbindungen der Formel I, in welchen $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ Hydroxymethyl, $R_3$ die Aethylgruppe und $R_4$ Wasserstoff ist, der carbocyclische Ring A unsubstituiert ist, und mit der weiteren Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Aethyl, $R_2$ Hydroxymethyl und $R_3$ und $R_4$ jeweils Wasserstoff bedeuten, der carbocyclische Ring A unsubstituiert ist, und mit der weiteren Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ die Carbomethoxygruppe oder die Cyanogruppe bedeutet, und $R_3$ und $R_4$ jeweils Wasserstoff ist, der carbocyclische Ring A unsubstituiert ist, als Epimerengemische, reine Epimere, Racematgemische, Racemate, optische Antipoden, Gemische von cis- und trans-

Isomeren, reine cis- oder trans-Isomere, N-Oxide oder Salze, insbesondere Säureadditionssalze, vor allem pharmazeutisch verwendbare, nicht-toxische Säureadditionssalze davon, oder solche Verbindungen als quaternäre Ammoniumverbindungen oder deren Salze.

Die Erfindung betrifft insbesondere Verbindungen der Formel I, worin $R_a$, $R_b$ und m sowie die Gruppe der Formel $-(R_b-R_a)-$ die angegebenen Bedeutungen haben, $R_1$ Wasserstoff, Niederalkyl, Hydroxy, Halogen, Aroyloxy, Niederalkanoyloxy, Niederalkoxy, Phenylniederalkoxy, Niederalkylthio, Amino, N-Niederalkylamino, N,N-Diniederalkylamino, Niederalkylenamino, Oxaniederalkylenamino, Thianiederalkylenamino, Azaniederalkylenamino, worin das Azastickstoffatom unsubstituiert oder durch Niederalkyl substituiert sein kann, oder Niederalkanoylamino darstellt, $R_2$ für Hydroxymethyl, Aroyloxymethyl, Benzolsulfonyloxymethyl, Carboxy, Niederalkoxycarbonyl, Hydroxyniederalkoxycarbonyl, Niederalkoxy-niederalkoxycarbonyl, Halogen-niederalkoxycarbonyl, Aminoniederalkoxycarbonyl, Niederalkylamino-niederalkoxycarbonyl, Diniederalkylamino-niederalkoxycarbonyl, Niederalkylenamino-niederalkoxycarbonyl, Oxaniederalkylenamino-niederalkoxycarbonyl, Thianiederalkylenamino-niederalkoxycarbonyl, oder Azaniederalkylenamino-niederalkoxycarbonyl, worin das Azastickstoffatom unsubstituiert oder durch Niederalkyl substituiert sein kann, Niederalkylthiocarbonyl, gegebenenfalls durch Niederalkyl oder Niederalkoxy 1 bis 3-fach substituiertes Cycloalkyloxycarbonyl, Benzofuranyloxycarbonyl, 1,3-Dihydro-1-oxo-3-isobenzofuranylcarbonyl, Carbamoyl, N-Niederalkyl-carbamoyl, N,N-Diniederalkyl-carbamoyl, Niederalkylenamino-carbonyl, Oxaniederalkylenamino-carbonyl, Thianiederalkylenamino-carbonyl oder Azaniederalkylenamino-carbonyl steht, worin das Azastickstoffatom unsubstituiert oder durch Niederalkyl substituiert sein kann, Hydrazinocarbonyl, N,N'-Niederalkylhydrazinocarbonyl, N,N'-Niederalkanoylhydrazinocarbonyl, gegebenenfalls im Phenylteil durch Niederalkyl, Niederalkoxy, Halogen und/oder Nitro substituiertes N,N'-Benzoylhydrazinocarbonyl, N,N'-Nicotinoylhydrazinocarbonyl, oder die Cyanogruppe, $R_3$ und $R_4$ jeweils Wasserstoff oder Niederalkyl bedeuten, und worin der

carbocyclische Ring A unsubstituiert ist oder Niederalkyl, Halogenniederalkyl, Hydroxyniederalkyl, Niederalkoxyniederalkyl, Hydroxy, Niederalkoxy, Phenylniederalkoxy, Niederalkyliden- oder Niederalkylendioxy, Halogen, Niederalkanoyloxy, Nitro, Amino, N-Niederalkylamino, N,N-Diniederalkylamino, Niederalkylenamino, Oxaniederalkylenamino, Thianiederalkylenamino, Azaniederalkylenamino, worin das Azastickstoffatom unsubstituiert oder durch Niederalkyl substituiert sein kann, und/oder Niederalkanoylamino jeweils 1-3-fach als Substituenten enthält, mit der Massgabe, dass in Verbindungen der Formel I, in welchen $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ Hydroxymethyl, $R_3$ die Aethylgruppe und $R_4$ Wasserstoff ist, der carbocyclische Ring A unsubstituiert ist, und mit der weiteren Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Aethyl, $R_2$ Hydroxymethyl und $R_3$ und $R_4$ jeweils Wasserstoff bedeuten, der carbocyclische Ring A unsubstituiert ist, und mit der weiteren Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ die Carbomethoxygruppe oder die Cyanogruppe bedeutet, und $R_3$ und $R_4$ jeweils Wasserstoff ist, der carbocyclische Ring A unsubstitituiert ist, als Epimerengemische, reine Epimere, Racematgemische, Racemate, optische Antipoden, Gemische von cis- und trans-Isomeren, reine cis- oder trans-Isomere, N-Oxide oder Salze, insbesondere Säureaddtionssalze, vor allem pharmazeutisch verwendbare, nicht-toxische Säureadditionssalze davon oder solche Verbindungen als quaternäre Ammoniumverbindungen oder deren Salze.

Die Erfindung betrifft insbesondere Verbindungen der Formel I, worin $R_a$, $R_b$ und m sowie die Gruppe der Formel $-(R_b-R_a)-$ die angegebenen Bedeutungen haben, $R_1$ Wasserstoff, Hydroxy, Niederalkoxy, Aroyloxy, Niederalkanoyloxy, Amino, N-Niederalkylamino, N,N-Diniederalkylamino

oder Niederalkanoylamino bedeutet, $R_2$ Hydroxymethyl, Aroyloxymethyl, Carboxy, Niederalkoxycarbonyl, Niederalkoxy-niederalkoxycarbonyl, Niederalkylthiocarbonyl, Diniederalkylaminoniederalkoxycarbonyl, Carbamoyl, N-Niederalkylcarbamoyl, Hydrazinocarbonyl, N,N'-Benzoylhydrazinocarbonyl, N,N'-Nicotinoylhydrazinocarbonyl, N,N-Diniederalkylcarbamoyl oder die Cyanogruppe darstellt, $R_3$ und $R_4$ jeweils Wasserstoff oder Niederalkyl ist, und worin der carbocyclische Ring A unsubstituiert oder durch Niederalkyl, Trifluormethyl, Hydroxy, Niederalkoxy, Phenylniederalkoxy, Niederalkylendioxy, Halogen, Niederalkanoyloxy, Nitro, Amino und/oder Niederalkanoylamino jeweils 1-3-fach substituiert ist, wobei Niederalkoxy, Niederalkyl, Niederalkanoyl und Niederalkanoylamino jeweils bis und mit 4 Kohlenstoffatome enthalten, mit der Massgabe, dass in Verbindungen der Formel I, in welchen $R_a$ die Gruppe der Formel $-(CH_2)_m-$ bedeutet, worin m für 1 steht, $R_b$ eine Gruppe der Formel $-CH(R_1)-$ darstellt, worin $R_1$ Wasserstoff ist, $R_2$ Hydroxymethyl, $R_3$ die Aethylgruppe und $R_4$ Wasserstoff ist, der carbocyclische Ring A unsubstituiert ist, und mit der weiteren Massgabe, dass in Verbindungen der Formel I, worin $R_a$ die Gruppe der Formel $-(CH_2)_m-$ bedeutet, worin m für 1 steht, $R_b$ eine Gruppe der Formel $-CH(R_1)-$ darstellt, worin $R_1$ Wasserstoff ist, $R_2$ die Carbomethoxygruppe oder die Cyanogruppe bedeutet, und $R_3$ und $R_4$ jeweils Wasserstoff ist, der carbocyclische Ring A unsubstituiert ist, als Epimerengemische, reine Epimere, Racematgemische, Racemate, optische Antipoden, Gemische von *cis*- und *trans*-Isomeren, reine *cis*- oder *trans*-Isomere, N-Oxide oder Salze, insbesondere Säureadditionssalze, vor allem pharmazeutisch verwendbare, nicht-toxische Säureadditionssalze davon, oder solche Verbindungen als quaternäre Ammoniumverbindungen, worin die quaternäre Gruppe Niederalkyl, Hydroxyniederalkyl, Halogenniederalkyl, Phenylniederalkyl oder Phenoxyniederalkyl ist, oder deren Salze.

Die Erfindung betrifft insbesondere Verbindungen der Formel I, worin $R_a$, $R_b$ und m sowie die Gruppe der Formel $-(R_b-R_a)-$ die angegebenen Bedeutungen haben, $R_1$ Wasserstoff, Hydroxy, Benzoyloxy, Niederalkanoyloxy oder Amino bedeutet, $R_2$ für Hydroxymethyl, im aromatischen Teil gegebenenfalls durch Niederalkyl, Niederalkoxy, Halogen oder funktionell abgewandeltes Carboxy substituiertes Benzoyloxymethyl, Carboxy, Niederalkoxycarbonyl, wie Methoxycarbonyl oder Aethoxycarbonyl, Niederalkoxy-niederalkoxycarbonyl wie Methoxy-methoxycarbonyl, Niederalkylthiocarbonyl, wie Aethylthiocarbonyl, Carbamoyl, Hydrazinocarbonyl oder die Cyanogruppe steht, $R_3$ und $R_4$ jeweils Wasserstoff oder Niederalkyl bedeuten, und worin der carbocyclische Ring A unsubstituiert oder, vorzugsweise in den Stellungen 8 und/oder 9, durch Niederalkyl, z.B. Methyl, Hydroxy, Niederalkoxy, z.B. Methoxy, Phenylniederalkoxy, z.B. Benzyloxy, Niederalkylendioxy, z.B. Methylendioxy, und/oder Halogen, z.B. Brom, substituiert ist, wobei Niederalkyl und Niederalkoxy jeweils bis und mit vier Kohlenstoffatome enthalten, mit der Massgabe, dass in Verbindungen der Formel I, in welchen $R_a$ die Gruppe der Formel $-(CH_2)_m-$ bedeutet, worin m für 1 steht, $R_b$ eine Gruppe der Formel $-CH(R_1)-$ darstellt, worin $R_1$ Wasserstoff ist, $R_2$ Hydroxymethyl, $R_3$ die Aethylgruppe und $R_4$ Wasserstoff ist, der carbocyclische Ring A unsubstituiert ist, und mit der weiteren Massgabe, dass in Verbindungen der Formel I, in welchen $R_a$ die Gruppe der Formel $-(CH_2)_m-$ bedeutet, worin m für 1 steht, $R_b$ eine Gruppe der Formel $-CH(R_1)-$ darstellt, worin $R_1$ Wasserstoff ist, $R_2$ die Carbomethoxygruppe oder die Cyanogruppe bedeutet, $R_3$ und $R_4$ jeweils Wasserstoff ist, der carbocyclische Ring A unsubstituiert ist, als Epimerengemische, reine Epimere, Racematgemische, Racemate, optische Antipoden, Gemische von cis- und trans-Isomeren, reine cis- oder trans-Isomere, N-Oxide oder Salze, insbesondere Säureadditionssalze, vor allem pharmazeutisch verwendbare, nicht-toxische Säureadditionssalze davon, oder solche Verbindungen als quaternäre Ammoniumverbindungen, worin die quaternäre Gruppe Niederalkyl oder Phenylniederalkyl ist, oder deren Salze.

Die Erfindung betrifft insbesondere Verbindungen der Formel I, worin

$R_a$ die Gruppe der Formel $-(CH_2)_m-$ bedeutet, wobei m für 1 steht, $R_b$ die Gruppe der Formel $-CH(R_1)-$ darstellt, wobei $R_1$ Wasserstoff oder Hydroxy, Benzoyloxy oder Acetyloxy ist, $R_2$ Hydroxymethyl, gegebenenfalls durch Niederalkyl, wie Methyl, oder Niederalkoxy, wie Methoxy, oder Carboxy substituiertes Benzoylmethyl, z.B. 3,4,5-Trimethoxybenzoylmethyl, Carboxy, Niederalkoxycarbonyl, insbesondere Methoxycarbonyl oder Aethoxycarbonyl, Niederalkoxy-niederalkoxycarbonyl wie Methoxymethoxycarbonyl, Niederalkylthiocarbonyl, wie Aethylthiocarbonyl, Carbamoyl oder die Cyanogruppe bedeutet, $R_3$ Wasserstoff oder Niederalkyl und $R_4$ Wasserstoff bedeutet, und worin der carbocyclische Ring A unsubstituiert oder in 8- und/oder 9-Stellung durch Hydroxy oder Niederalkoxy, z.B. Methoxy, oder Methylendioxy und/oder Halogen, wie Brom, substituiert ist, wobei in diesen Verbindungen das Wasserstoffatom in 11b-Stellung und der Rest $R_3$, insbesondere Aethyl, in der trans- oder vorzugsweise in der cis-Konfiguration zueinander stehen, mit der Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ Hydroxymethyl, $R_3$ die Aethylgruppe und $R_4$ Wasserstoff ist, der carbocyclische Ring A unsubstituiert ist, und mit der weiteren Massgabe, dass in Verbindungen der Formel I, in welchen $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ Methoxycarbonyl oder die Cyanogruppe bedeutet, $R_3$ und $R_4$ jeweils Wasserstoff ist, der carbocyclische Ring A unsubstituiert ist, als Epimerengemische, reine Epimere, Racematgemische, Racemate, optische Antipoden, Gemische von cis- und trans-Isomeren, reine cis- oder trans-Isomere, N-Oxide oder Salze, insbesondere Säureadditionssalze, vor allem pharmazeutisch verwendbare, nicht-toxische Säureadditionssalze davon, oder solche Verbindungen als quaternäre Ammoniumverbindungen, worin die quaternäre Gruppe Niederalkyl, z.B. Methyl ist, oder deren Salze.

Die Erfindung betrifft insbesondere Verbindungen der Formel I, worin $R_a$ die Gruppe der Formel $-(CH_2)_m-$ bedeutet, wobei m für 1 steht, $R_b$ die

Gruppe der Formel $-CH(R_1)-$ darstellt, wobei $R_1$ Wasserstoff, Hydroxy, Niederalkanoyloxy, wie Acetyloxy, oder Aroyloxy, wie Benzoyloxy ist, $R_2$ Carbamoyl, Carboxy oder Niederalkoxycarbonyl, wie Methoxycarbonyl, ist, $R_3$ Niederalkyl, wie Aethyl, und $R_4$ Wasserstoff darstellt, und worin der carbocyclische Ring A unsubstituiert oder in 8- und/oder 9-Stellung durch Halogen, wie Brom, substituiert ist, wobei in diesen Verbindungen das Wasserstoffatom in 11b-Stellung und der Rest $R_3$, insbesondere Aethyl, in der trans-, vorzugsweise jedoch in der cis-Konfiguration zueinander stehen, und eine Hydroxygruppe $R_1$ in der threo-, vorzugsweise jedoch in der erythro-Form, bezogen auf die durch das in 11b-Stellung stehende Wasserstoffatom und die Niederalkylgruppe $R_3$, insbesondere Aethyl, gebildete cis-Konfiguration steht, als Epimerengemische, reine Epimere, Racematgemische, Racemate, optische Antipoden oder Salze, insbesondere Säureadditionssalze, vor allem pharmazeutisch verwendbare nicht-toxische Säureadditionssalze davon.

Die Erfindung betrifft in erster Linie die in den Beispielen beschriebenen neuen Verbindungen der Formel I oder Salze, insbesondere Säureadditionssalze, vor allem pharmazeutisch verwendbare, nicht toxische Säureadditionssalze davon.

Die neuen Verbindungen der Formel I werden in an sich bekannter Weise hergestellt. Man kann sie z.B. erhalten, indem man in einer Verbindung der Formel

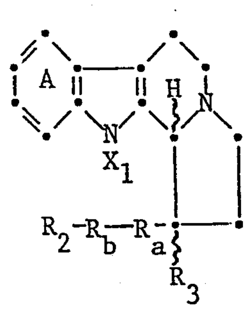

(II),

worin $X_1$ Wasserstoff oder einen abspaltbaren und durch Wasserstoff ersetzbaren Substituenten oder Niederalkyl bedeutet und gegebenenfalls Hydroxy- und/oder Aminogruppen durch einen abspaltbaren und durch Wasserstoff ersetzbaren Rest substituiert sind mit der Massgabe, dass mindestens $X_1$ einen abspaltbaren und durch Wasserstoff ersetzbaren Substituenten bedeutet, oder mindestens Hydroxy- und/oder Aminogruppen durch einen abspaltbaren und durch Wasserstoff ersetzbaren Substituenten substituiert sind, oder in einem Salz davon, die von Wasserstoff verschiedene Gruppe $X_1$ und/oder Hydroxy- und/oder Aminogruppen substituierende, abspaltbare und durch Wasserstoff ersetzbare Substituenten abspaltet und durch Wasserstoff ersetzt, und wenn erwünscht, eine erhältliche Verbindung der Formel I in eine andere Verbindung der Formel I umwandelt, und/oder, wenn erwünscht, ein erhaltenes Racematgemisch in die Racemate, oder ein erhaltenes Racemat in die optischen Antipoden, oder ein erhaltenes Gemisch von cis- und trans-Isomeren in die reinen cis- und trans-Isomeren oder ein erhaltenes Epimerengemisch in die reinen Epimeren auftrennt, und/oder wenn erwünscht, eine erhaltene Verbindung der Formel I in ein Salz oder ein erhaltenes Salz in eine freie Verbindung oder ein anderes Salz überführt.

Die Abspaltung der Gruppe $X_1$ oder von an Hydroxy- und/oder Aminogruppen stehenden Substituenten, wobei solche Hydroxy- und/oder Aminogruppen auch zusammen durch einen zweiwertigen Substituenten substituiert sein können, erfolgt mittels Solvolyse, wie Hydrolyse, Alkoholyse oder Acidolyse, oder mittels Reduktion einschliesslich Hydrogenolyse.

Eine besonders geeignete abspaltbare Gruppe $X_1$ oder eine Hydroxy- und/oder Aminoschutzgruppe ist in erster Linie eine hydrogenolytisch abspaltbare $\alpha$-Arylniederalkylgruppe, wie eine gegebenenfalls substituierte 1-Polyphenylniederalkyl- oder 1-Phenylniederalkylgruppe, worin Substituenten, insbesondere des Phenylteils, z.B. Niederalkyl wie Methyl, oder Niederalkoxy wie Methoxy, oder Halogen, wie Chlor oder Trifluormethyl sein können, und in erster Linie Benzyl. Hydroxy-und/oder Aminoschutzgruppen können auch einen solvolytisch, wie hydrolytisch oder acidolytisch ferner einen reduktiv, einschliesslich hydrogenolytisch, abspaltbaren Rest, insbesondere einen entsprechenden Acylrest, wie den Acylrest einer organischen Carbonsäure, z.B. Niederalkanoyl, wie Acetyl, oder Aroyl, wie Benzoyl, ferner den Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenyl-niederalkylgruppe, worin Substituenten, in erster Linie des Phenylteils, z.B. die oben gegebene Bedeutung haben, und in erster Linie Trityl darstellen, während $X_1$ als solvolytisch, wie hydrolytisch oder acidolytisch abspaltbarer Rest den Rest eines Halbesters oder Kohlensäure, z.B. Niederalkoxycarbonyl, Methoxy- oder Aethoxycarbonyl, oder den Rest einer organischen Sulfonsäure, etwa einen Arylsulfonylrest, wie den Benzol- oder p-Toluolsulfonylrest bedeutet. Ein Hydroxy- und/oder Aminogruppen zusammen substituierender zweiwertiger Rest ist in erster Linie eine hydrogenolytisch abspaltbare Gruppe, wie gegebenenfalls substituiertes 1-Phenyl-niederalkyliden, worin Substituenten, insbesondere des Phenylteils, z.B. Niederalkyl oder Niederalkoxy sein können, und insbesondere Benzyliden, sowie solvolytisch, insbesondere hydrolytisch spaltbare Gruppen, wie Niederalkyliden, z.B. Methylen oder Isopropyliden, oder 1-Phenyl-niederalkyliden, dessen Phenylteil gegebenenfalls durch Niederalkyl, wie Methyl oder Niederalkoxy wie Methoxy, substituiert ist, insbesondere Benzyliden, oder Cycloalkyliden, z.B. Cyclopentyliden oder Cyclohexyliden.

In der Form von Salzen verwendbare Ausgangsstoffe werden in erster Linie in der Form von Säureadditionssalzen, z.B. mit Mineralsäuren, sowie mit organischen Säuren verwendet.

Hydrogenolytisch abspaltbare Reste $X_1$ sowie für diesen Fall Hydroxy- und/oder Aminogruppen substituierende Reste dieser Art, insbesondere substituierte 1-Phenylniederalkylgruppen, ferner auch geeignete Acylgruppen, wie gegebenenfalls substituiertes 1-Phenylniederalkoxycarbonyl, sowie an Hydroxy- und/oder Aminogruppen zusammen stehende gegebenenfalls substituierte 1-Phenylniederalkylidengruppen können durch Behandeln mit katalytisch aktiviertem Wasserstoff, z.B. mit Wasserstoff in Gegenwart eines Nickelkatalysators, wie Raney-Nickel, oder eines geeigneten Edelmetallkatalysators abgespalten werden.

Hydrolytisch abspaltbare Gruppen $X_1$ sowie für diesen Fall auch Hydroxy- und/oder Aminogruppen substituierende Gruppen dieser Art, wie Acylreste von organischen Carbonsäuren, z.B. Niederalkoxycarbonyl, ferner z.B. Tritylreste sowie Hydroxy- und/oder Aminogruppen zusammen substituierende Niederalkyliden-, 1-Phenyl-niederalkyliden- oder Cycloalkylidengruppen können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxids oder -carbonats oder eines Amins, wie Isopropylamin, abgespalten werden.

Acidolytisch abspaltbare Reste $X_1$ sowie auch Hydroxy- und/oder Aminogruppen substituierende Reste dieser Art sind insbesondere gewisse Acylreste von Halbestern der Kohlensäure, z.B. tert.-Niederalkoxycarbonyl, oder, als Substituenten von Amino- und/oder Hydroxygruppen, gegebenenfalls substituierte Diphenylmethoxycarbonylreste, ferner auch ein tert.-Niederalkylrest z.B. tert.Butyl; solche Reste können durch Behandeln mit geeigneten starken organischen Carbonsäuren, wie gegebenenfalls durch Halogen, insbesondere Fluor, substituierten Niederalkylcarbonsäuren, in erster Linie mit Trifluoressigsäure (wenn notwendig, in Gegenwart eines aktivierenden Mittels, wie Anisol), sowie mit Ameisensäure abgespalten werden.

Unter reduktiv abspaltbaren, Hydroxy- und/oder Aminogruppen substituierenden Resten dieser Art werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl oder Arylmethoxycarbonyl, die z.B. beim Behandeln mit einem reduzierenden Schwermetall, wie Zink, oder mit einem reduzierenden Schwermetallsalz, wie einem Chrom(II)salz, z.B. -chlorid oder -acetat, üblicherweise in Gegenwart einer organischen Carbonsäure, wie Ameisensäure oder Essigsäure, und von Wasser abgespalten werden können.

Schutzgruppen, welche an Hydroxy- und/oder Aminogruppen stehen, entsprechen den vorhin genannten und mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmassnahme abgespalten werden.

Die obigen Reaktionen werden üblicherweise in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z.B. in einem offenen oder geschlossenen Gefäss und/oder in der Atmosphäre eines Inertgases, z.B. Stickstoff.

Ausgangsstoffe der Formel II können in an sich bekannter Weise hergestellt werden. Ausgangsstoffe der Formel II, worin anstelle der Gruppen $-(R_b-R_a)-$ der Rest der Formel -CH=CH- steht, können auf übliche Weise erhalten werden, indem man eine Verbindung der Formel

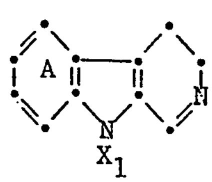

(IIa),

mit einem Aldehyd der Formel

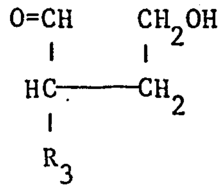

(IIb)

oder einem reaktionsfähigen Derivat davon, etwa einem solchen worin die Aldehydgruppe in silylierter Form vorliegt und demnach z.B. der Gruppe

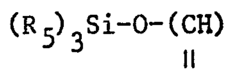

(IIc)

entspricht, worin $R_5$ Niederalkyl, z.B. Methyl ist, und die Hydroxygruppe vorzugsweise in reaktionsfähiger, veresterter Form, z.B. als Ester mit einer anorganischen Säure, etwa einer Halogenwasserstoffsäure, oder einer organischen Säure, z.B. p-Toluolsulfonsäure vorhanden ist, und demnach z.B. als Brom oder als p-Toluolsulfonyloxy vorliegt, in Gegenwart eines basischen Mittels, etwa eines Amins, wie z.B. Aethyl-diisopropylamin, in einem geeigneten Lösungsmittel, etwa einem solchen stark polaren und aprotischen Charakters, etwa Dimethylformamid, zu einer Verbindung der Formel

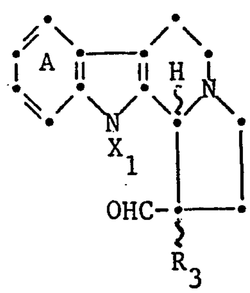

(IId)

umsetzt, und eine so erhaltene Verbindung mit einer Verbindung der Formel

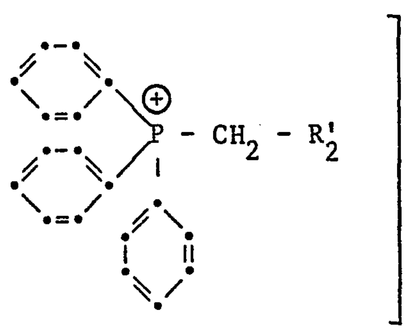

Hal$^{\ominus}$ (IIe)

worin Hal für Halogen, z.B. Chlor, steht, und $R_2'$ gegebenenfalls verestertes oder amidiertes Carboxy, z.B. Niederalkoxycarbonyl, Carbamoyl oder Cyano bedeutet, in Gegenwart einer Base, z.B. eines Alkalimetallniederalkanolats oder -hydrids, etwa Kalium-tert.butylat oder Natriumhydrid, unter den Bedingungen der Wittig-Reaktion zu einer Verbindung der Formel II umsetzt, worin anstelle der Gruppe $-(R_b-R_a)-$ der Rest der Formel -CH=CH- steht und worin $R_2'$ die angegebene Bedeutung hat.

Man kann aber auch so vorgehen, dass man einen Aldehyd der Formel IId mit einer Verbindung der Formel

$$R_2' - CH_2 - Hal \qquad (IIf),$$

worin Hal für Halogen, insbesondere für Brom steht, unter den Bedingungen der Reformatsky-Reaktion in Gegenwart von Zink in einem geeigneten inerten Lösungsmittel, z.B. Benzol oder Tetrahydrofuran, umsetzt, das Reaktionsprodukt zu einer Verbindung der Formel

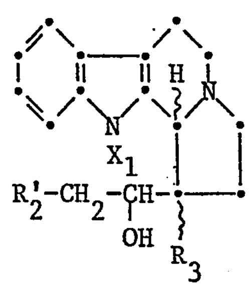

(IIg)

hydrolysiert, und hieraus unter Wasserabspaltung eine Verbindung der Formel II herstellt, worin anstelle der Gruppe $-R_b-R_a-$ der Rest -CH=CH- steht und $R_2'$ die angegebene Bedeutung hat.

Ausgangsstoffe der Formel II, worin $R_a$ und $R_b$ die angegebenen Bedeutungen mit Ausnahme der Gruppe -CH=CH- haben, können erhalten werden, indem man z.B. in einer Verbindung der Formel

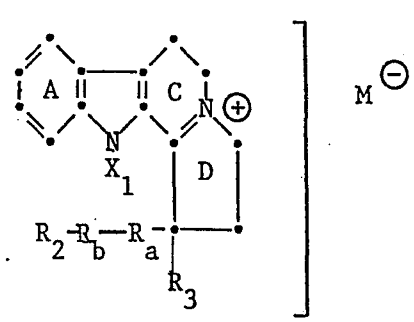

(III),

worin $M^{\ominus}$ das Anion einer Säure, z.B. das der Perchlorsäure ist, $X_1$ obige Bedeutung hat, und bezüglich vorhandener Hydroxy- und/oder Aminogruppen die angegebenen Substituenten und Massgaben gelten, die den Ringen C und D gemeinsame Doppelbindung zur Kohlenstoff-Stickstoff-Einfachbindung, z.B. mittels Wasserstoff in Gegenwart eines Hydrierkatalysators, etwa eines Palladium-auf-Kohle-Katalysators, oder mittels eines Hydrids, wie Diboran oder Natriumborhydrid, oder eines Dileichtmetallhydrids, wie Lithium-tri-tert.butoxyaluminiumhydrid, reduziert. Hierbei ist darauf zu achten, dass andere vorhandene Doppelbindungen nicht angegriffen werden, beispielsweise durch volumetrische Kontrolle des Wasserstoffverbrauchs oder entsprechende Dosierung eines der genannten Reduktionsmittel und rechtzeitigen Abbruch der Reduktion.

Ausgangsmaterialien der Formel III wiederum lassen sich beispielsweise durch folgende Umsetzungen herstellen:

Man reduziert in üblicher Weise eine Verbindung der Formel

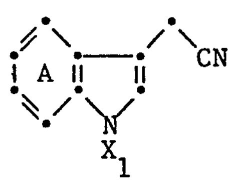

(IIIa),

z.B. mittels katalytisch aktiviertem Wasserstoff, etwa Wasserstoff in Gegenwart eines Nickelkatalysators, wie Raney-Nickel, vorzugsweise in Gegenwart von Ammoniak, zu einem Tryptaminderivat der Formel

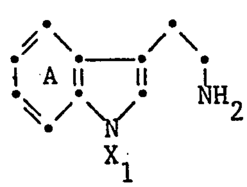

(IIIb),

setzt dieses mit einer Verbindung der Formel

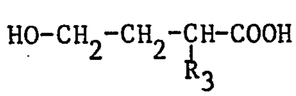

(IIIc)

oder einem reaktionsfähigen Derivat davon, beispielsweise mit dem aus einem in 2-Stellung gegebenenfalls niederalkylierten, wie äthylierten, γ-Butyrolacton der Formel

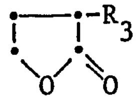

(IIId)

und Thionylchlorid in Gegenwart eines geeigneten Kondensationsmittels, etwa wasserfreiem Zinkchlorid, in üblicher Weise erhältlichen entsprechenden 4-Chlorbutyrylchlorid der Formel

$$Cl-CH_2-CH_2-\underset{R_3}{CH}-COCl \qquad (IIIe)$$

zu einer Verbindung der Formel

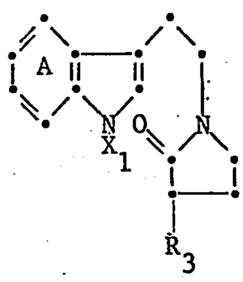

(IIIf),

um, wandelt eine solche Verbindung durch auf übliche Weise, z.B. in Gegenwart eines Kondensationsmittels, etwa eines solchen sauren Charakters, wie Phosphoroxychlorid, durchzuführende ringschliessende Kondensation in eine Verbindung der Formel

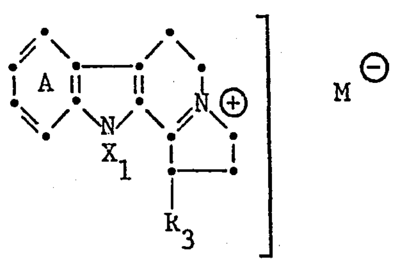

(IIIg)

um, stellt hieraus durch Behandeln mittels einer Base, etwa eines Alkalihydroxids, wie Natriumhydroxid, eine Verbindung der Formel

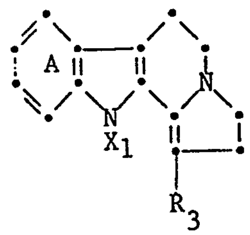

(IIIh)

her, und setzt eine solche Verbindung mit einer Acrylverbindung der Formel

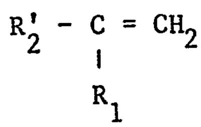

. (IIIi)

zu einer Verbindung der Formel III, worin $R_a$ die Gruppe $-CH_2-$ bedeutet, $R_1$ und $R_2'$ die angegebenen Bedeutungen haben, um.

Diese Umsetzungen werden in an sich bekannter Weise vorgenommen.

Die neuen Verbindungen der Formel I können ebenfalls erhalten werden, indem man in einer Verbindung der Formel

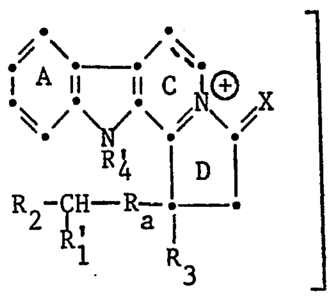

$M^{\ominus}$ (IV),

worin $M^{\ominus}$ das Anion einer Säure, z.B. das der Perchlorsäure ist, die Gruppe $>C=X$ für die Gruppe $>C=S$ oder $>CH_2$ steht, $R_1'$ die Bedeutung von $R_1$ hat, oder für eine durch eine Schutzgruppe geschützte, mittels Reduktion in eine freie Hydroxygruppe umwandelbare Hydroxygruppe steht, oder $R_1'$ einen Niederalkenylrest darstellt, oder gegebenenfalls anstelle der Gruppe $-\underset{R_1'}{\underset{|}{CH}}-$ der Rest der Formel $>C=CHR_1''$ (IVa), steht, worin $R_1''$ Wasserstoff oder einen ein Kohlenstoffatom weniger aufweisenden Niederalkylrest bedeutet, oder anstelle der Gruppe $-\underset{R_1'}{\underset{|}{CH}}-R_a-$

die Gruppe -CH=CH- (IVb) steht, und $R_4'$ Wasserstoff oder einen Niederalkenylrest bedeutet, die den Ringen C und D gemeinsame Doppelbindung zur Kohlenstoff-Stickstoff-Einfachbindung, einen gegebenenfalls vorhandenen Niederalkenylrest $R_1'$ und/oder $R_4'$ zur Niederalkylgruppe $R_1$ bzw. $R_4$ und/oder gegebenenfalls die Gruppe IVa zur Gruppe $-\underset{R_1}{CH}-$, und/oder gegebenenfalls die Gruppe IVb zur Gruppe $-CH_2-CH_2-$ (IVc), und/oder eine im Ring C gegebenenfalls vorhandene Doppelbindung zur Kohlenstoff-Kohlenstoff-Einfachbindung und/oder eine gegebenenfalls vorhandene Gruppe $>C=S$ zur Gruppe $>CH_2$ reduziert, und an Hydroxy- und/oder Aminogruppen gegebenenfalls stehende, unter den Bedingungen der Reduktion abspaltbare und durch Wasserstoff ersetzbare Schutzgruppen abspaltet und durch Wasserstoff ersetzt, und, wenn erwünscht, die anschliessend an das erste Verfahren beschriebenen Verfahrensschritte durchführt.

Ein Niederalkenylrest $R_1'$ bzw. $R_4'$ hat bis zu 7, vorzugsweise bis zu 4 Kohlenstoffatome und ist z.B. ein Vinyl-, 2-Methylvinyl-, Allyl- oder 2-Methylallylrest.

An Hydroxy- und/oder Aminogruppen gegebenenfalls stehende unter den Bedingungen dieses Reduktionsverfahrens abspaltbare und durch Wasserstoff ersetzbare Gruppen sind z.B. die für $X_1$ und für Hydroxy- und/oder Aminogruppen genannten, reduktiv abspaltbaren und durch Wasserstoff ersetzbaren Gruppen, z.B. eine α-Arylniederalkylgruppe, z.B. eine 1-Phenylniederalkyl-, wie die Benzylgruppe, während die Hydroxy- und/oder Aminogruppen zusammen substituierenden Reste, etwa Phenyläthylidenreste, solche der beschriebenen Art sind und z.B. den Benzylidenrest darstellen.

Die Reduktion der den Ringen C und D gemeinsamen Doppelbindung und/oder einer Niederalkenylgruppe $R_1'$ und/oder $R_4'$ bzw. einer Gruppe IVa oder IVb sowie einer im Ring C gegebenenfalls vorhandenen Doppelbindung, und/oder die Abspaltung gegebenenfalls an Hydroxy- und/oder Aminogruppen

einschliesslich dem Indolstickstoffatom stehender Schutzgruppen für den Fall, dass die Gruppe $>C=X$ die Gruppe $>CH_2$ darstellt, kann in an sich bekannter Weise, z.B. durch Behandeln mit katalytisch aktiviertem Wasserstoff, wie Wasserstoff in Gegenwart eines geeigneten Hydrierkatalysators, z.B. eines Nickel-, Platin- oder Palladiumkatalysators, erfolgen, wobei gegebenenfalls vorhandene hydrogenolytisch abspaltbare Schutzgruppen zugleich abgespalten und durch Wasserstoff ersetzt werden; oder man arbeitet mit einem geeigneten Hydridreduktionsmittel, wie z.B. Diboran, oder einem Alkalimetallborhydrid, z.B. Natriumborhydrid. Für die Reduktion der den Ringen C und D gemeinsamen Doppelbindung, wobei die Gruppe $>C=X$ für die Gruppe $>CH_2$ steht, kommen ferner Dileichtmetallhydride, etwa Lithiumaluminiumhydrid, insbesondere Lithium-tri-tert.-butoxyaluminiumhydrid in Betracht.

Bei der Anwendung von Hydridreduktionsmitteln können auch an Sauerstoff gebundene Acylreste von Carbonsäuren, wie z.B. der der Essigsäure, im gleichen Arbeitsgang abgespalten werden. Bei der Durchführung dieser, insbesondere der katalytischen, Reduktionen, ist darauf zu achten, dass andere Gruppen nicht angegriffen werden, z.B. durch volumetrische Kontrolle des Wasserstoffverbrauchs und rechtzeitigen Abbruch der Reduktion, oder durch entsprechende Dosierung des Reduktionsmittels.

Für den Fall, dass in Ausgangsstoffen der Formel IV die Gruppe $>C=X$ für die Gruppe $>C=S$ steht, erfolgt deren reduktive Umwandlung in die Gruppe $>CH_2$ sowie die gleichzeitige Reduktion der den Ringen C und D gemeinsamen Doppelbindung zur Kohlenstoff-Stickstoff-Einfachbindung und gegebenenfalls vorhandener Gruppen IVa oder IVb sowie einer gegebenenfalls vorhandenen Niederalkylengruppe $R_1'$ und/oder $R_4'$ und einer gegebenenfalls vorhandenen Doppelbindung im Ring C in üblicher Weise mittels Wasserstoff in Gegenwart eines sowohl hydrierend wie auch desulfierend wirkenden Katalysators, etwa Raney-Nickel. Diese Reduktionen werden in üblicher Weise, etwa in Gegenwart eines geeigneten Lösungsmittels und in einem Temperaturbereich von etwa -10° bis

etwa +100° in einem offenen oder geschlossenen Gefäss gegebenenfalls in einer Inertgasatmosphäre, z.B. unter Stickstoff, vorgenommen.

Ausgangsstoffe der Formel IV können in an sich bekannter Weise hergestellt werden. So kann man z.B. analog den zur Herstellung von Verbindungen der Formel III beschriebenen Reaktionsschritten IIIb-IIIh, wobei die Gruppe >C=X für die Gruppe >$CH_2$ steht, wie folgt vorgehen:

Man setzt beispielsweise eine Tryptaminverbindung der Formel

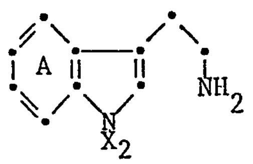

(IVd),

worin $X_2$ Niederalkyl oder eine geeignete mittels Solvolyse, wie Hydrolyse abspaltbare Gruppe ist, die sich von einer organischen Sulfonsäure ableitet und z.B. Arylsulfonyl, wie p-Toluolsulfonyl ist, oder einen Rest eines Halbesters der Kohlensäure, z.B. Niederalkoxycarbonyl, wie etwa tert.-Butoxycarbonyl darstellt, z.B. mit dem aus einem in 2-Stellung gegebenenfalls niederalkylierten, wie äthylierten, γ-Butyrolacton der Formel IIId und Thionylchlorid in Gegenwart von wasserfreiem Zinkchlorid in üblicher Weise erhältlichen entsprechenden 4-Chlorbutyrylchlorid der Formel

$$Cl-CH_2-CH_2-\underset{R_3}{CH}-COCl \quad \text{(IVe)}$$

zu einer Verbindung der Formel

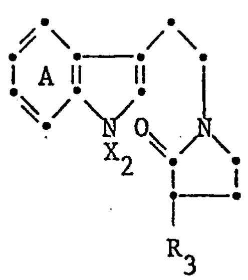

(IVf)

um, unterwirft eine solche Verbindung der ringschliessenden, auf übliche Weise, z.B. in Gegenwart eines Kondensationsmittels, z.B. eines

solchen sauren Charakters wie Phosphoroxychlorid, durchzuführenden Kondensation zu einer Verbindung der Formel

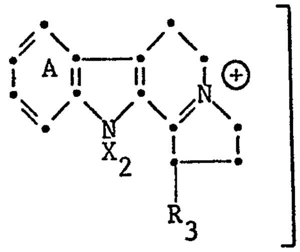 $M^{\ominus}$ (IVg),

wandelt eine solche Verbindung mittels einer Base, etwa eines Alkalihydroxids, wie Natriumhydroxid, in eine Verbindung der Formel

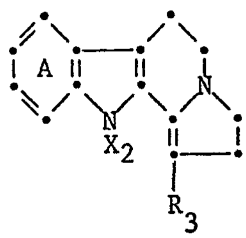 (IVh)

um, und setzt eine solche Verbindung, gegebenenfalls nach Abspaltung der Gruppe $X_2$, z.B. mittels Wasser in basischem Medium, z.B. in Gegenwart eines Alkalihydroxids, wie Natriumhydroxid, mit einer Verbindung der Formel

$$R'_2 - \underset{\underset{R'_1}{|}}{C} = CH_2 \qquad \text{(IVi)}$$

und gegebenenfalls nachfolgender Abspaltung der Gruppe $X_2$, z.B. wie angegeben, und deren Ersatz durch Wasserstoff zu einer Verbindung der Formel IV um. Durch Behandeln einer solcher Verbindung mit einem dehydrierend wirkenden Agens, etwa in gepufferter wässriger Lösung, z.B. mit Luftsauerstoff, kann man nach Entfernung der gegebenenfalls vorhandenen abspaltbaren und durch Wasserstoff ersetzbaren Gruppe $X_2$, etwa wie angegeben, eine Verbindung der Formel IV mit einer zusätzlichen Doppelbindung im Ring C erhalten.

Eine abgeänderte Verfahrensweise zur Herstellung von Ausgangsstoffen der Formel IV besteht darin, dass man eine Verbindung der Formel

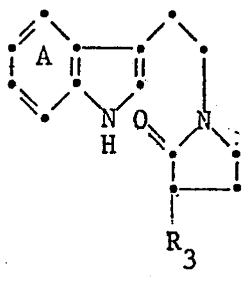

(IVk)

worin den carbocyclischen Ring A gegebenenfalls substituierende Hydroxy- und/oder Aminogruppen zweckmässigerweise in geschützter, z.B. aralkylierter, wie 1-Phenylniederalkylierter, z.B. benzylierter, Form vorliegen, in eine entsprechende als Dianion vorliegende Verbindung der Formel

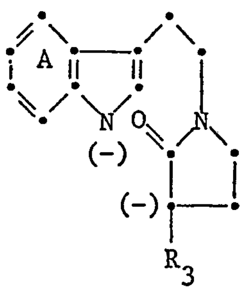

(IVk')

beispielsweise durch Umsetzung mit einer geeigneten metallorganischen Verbindung, etwa einem organischen Alkalimetallamid, z.B. Lithiumdiisopropylamid, in üblicher Weise überführt, eine solche Verbindung mit einer Verbindung der Formel IVi zu einer Verbindung der Formel

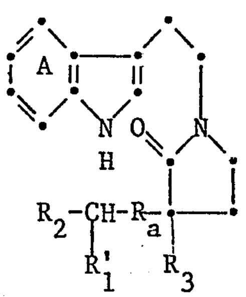

(IVl)

umsetzt, und anschliessend eine solche Verbindung durch ringschliessende Kondensation mittels eines geeigneten Kondensationsmittels, etwa wie vorhin beschrieben, z.B. eines solchen sauren Charakters, wie etwa Phosphoroxychlorid,und nachfolgender Abspaltung gegebenenfalls vorhandener Schutzgruppen, etwa mittels Hydrolyse, in eine Verbindung der Formel IV umwandelt. Diese Reaktionen werden in an sich bekannter Weise durchgeführt. Ausgangsstoffe der Formel IV, welche die Gruppe IVa enthalten, können erhalten werden, indem man z.B. eine Verbindung der Formel

$$\begin{array}{l} R_2' - \underset{\displaystyle CH_2OH}{\underset{|}{C}} = CHR_1'' \end{array} \qquad (IVm),$$

worin die primäre Hydroxygruppe vorzugsweise in reaktionsfähiger veresterter Form, z.B. als Ester mit einer anorganischen oder organischen Säure, wie einer Halogenwasserstoffsäure oder einer organischen Sulfonsäure, etwa p-Toluolsulfonsäure vorliegt, und demnach beispielsweise p-Tosyloxy oder insbesondere Brom darstellt, mit einer Verbindung der Formel IVh in üblicher Weise, z.B. in schwach saurer gepufferter Lösung, etwa bei einem pH von 6,0-6,8, umsetzt.

Ausgangsstoffe der Formel IV, worin die Gruppe $>C=X$ die Gruppe $>C=S$ darstellt, können in an sich bekannter Weise hergestellt werden, indem man z.B. eine Tryptaminverbindung der Formel IVd mit einer Carbonsäure der Formel

$$\begin{array}{l} \overset{\displaystyle CHO}{\overset{|}{R_3-CH}}-CH_2-COOH \end{array} \qquad (IVn)$$

oder einem reaktionsfähigen Derivat davon, etwa einem Ester mit einem Niederalkanol, z.B. Methanol, zu einer Verbindung der Formel

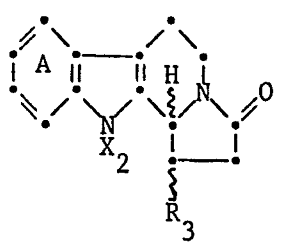

(IVo)

umsetzt. Die Umsetzung wird in an sich bekannter Weise, üblicherweise in Gegenwart eines geeigneten Lösungsmittels, z.B. in einem Temperaturbereich von etwa -10° bis etwa +100°C in einem offenen oder geschlossenen Gefäss durchgeführt.

Anschliessend wird eine erhaltene Verbindung der Formel IVo, worin Hydroxy- und/oder Aminogruppen gegebenenfalls in geschützter, z.B. wie oben beschrieben, in acylierter, wie etwa niederalkanoylierter, z.B. acetylierter, oder aralkylierter z.B. benzylierter, Form vorliegen, mittels Oxidation in eine Verbindung der Formel

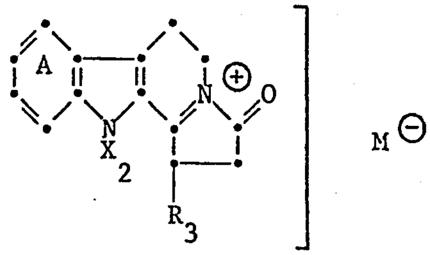

(IVp),

oder ein Salz davon umgewandelt und gegebenenfalls vorhandene Schutzgruppen hydrolytisch, z.B. wie beschrieben, abgespalten.

Geeignete Oxidationsmittel sind beispielsweise Hg(II)-salze, z.B. Hg(II)-acetat, ferner unterchlorige Säure, zweckmässigerweise in Form eines geeigneten Derivats, etwa tert.-Butylhypochlorit.

Eine so erhaltene Verbindung der Formel IVp wird anschliessend durch Behandeln mit einer Base, z.B. mit einem Alkalimetallhydroxid, etwa Natriumhydroxid, in eine Verbindung der Formel

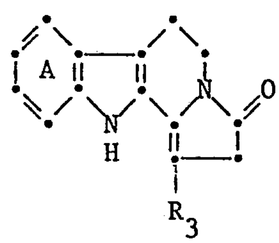

(IVq)

umgewandelt. Hieran schliesst sich die Umsetzung einer so erhaltenen Verbindung mit einer Verbindung der Formel IVi, etwa analog, wie vorhin beschrieben, zu einer Verbindung der Formel

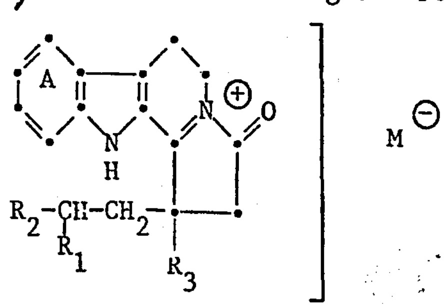

(IVr)

an, welche dann durch Umsetzung mit einer geeigneten Schwefelverbindung, z.B. 2,4—bis-(4-Methoxyphenyl)-2,4-dithioxo-1,3,2,4-dithiadiphosphetan (Lawesson-Reagenz), in eine Verbindung der Formel IV umgewandelt wird, worin die Gruppe >C=X für die Gruppe >C=S steht. In Abänderung dieser Reaktionsfolge kann man auch so vorgehen, dass man eine Verbindung der Formel IVq mit einer geeigneten Schwefelverbindung, etwa wie beschrieben, z.B. mit dem oben definierten Lawesson-Reagenz, in eine Verbindung der Formel

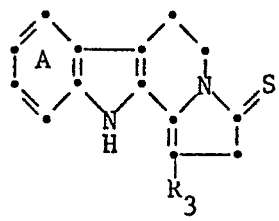

(IVs)

umwandelt, und aus einer solchen Verbindung durch Umsetzung mit einer Verbindung der Formel IVi, z.B. analog wie vorhin beschrieben, eine Verbindung der Formel IV erhält, worin die Gruppe >C=X die Gruppe >C=S darstellt.

Ausgangsstoffe der Formel IV, die eine Gruppe IVb enthalten, können auf übliche Weise, z.B. durch Umsetzung einer Tryptaminverbindung der Formel IVd, worin Hydroxy- und/oder Aminogruppen zweckmässigerweise in geschützter Form, etwa wie beschrieben, z.B. durch α-Arylniederalkyl, wie Benzyl, geschützt vorliegen, mit einer Verbindung der Formel

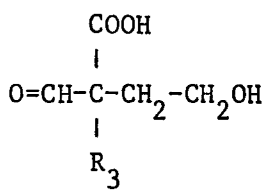

(IVt),

worin die Aldehydgruppe zweckmässig in geschützter, wie z.B. acetalisierter Form, z.B. als Diniederalkyl-, wie Dimethylacetal,vorliegt, die Carboxylgruppe in veresterter Form, z.B. als Ester, etwa mit einem Niederalkanol, z.B. Aethanol, und die primäre Hydroxylgruppe in reaktionsfähiger veresterter Form, z.B. als Ester mit einer anorganischen Säure, z.B. Salzsäure, oder mit einer organischen Säure, etwa

mit p-Toluolsulfonsäure, vorliegt, und demnach z.B. Chlor, oder p-Tosyloxy bedeutet, in eine Verbindung der Formel

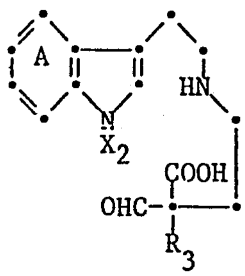

(IVu)

umgewandelt werden, die anschliessend der cyclisierenden Kondensation zu einer Verbindung der Formel

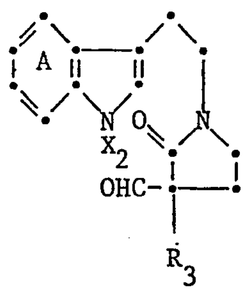

(IVv)

unterworfen wird.

Die Reaktion erfolgt zweckmässigerweise in Gegenwart eines Kondensationsmittels, z.B. einer Base, etwa einer heterocyclischen Stickstoffbase, wie Imidazol, bei erhöhter Temperatur. Anschliessend wird die gegebenenfalls geschützte, wie acetalisierte, Aldehydgruppe im sauren Medium, etwa in Essigsäure, in die freie Aldehydgruppe umgewandelt. Eine so erhaltene Verbindung der Formel IVv wird anschliessend mit einer Verbindung der Formel

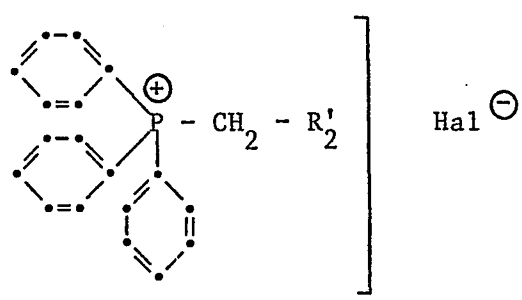

(IVw),

worin Hal für Halogen, z.B. Chlor, steht, und $R_2'$ gegebenenfalls verestertes oder amidiertes Carboxy, z.B. Niederalkoxycarbonyl oder Carbamoyl, bedeutet, in Gegenwart einer Base, z.B. eines Alkalimetall-

niederalkanolats, etwa Kalium-tert.-butylat, unter den Bedingungen der Wittig-Reaktion zu einer Verbindung der Formel

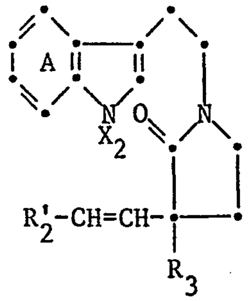

(IVx)

umgesetzt, eine so erhaltene Verbindung mittels cyclisierender Kondensation, etwa in Gegenwart eines geeigneten Kondensationsmittels, z.B. eines solchen sauren Charakters, wie etwa Phosphoroxychlorid, und Abspaltung der Gruppe $X_2$ sowie gegebenenfalls vorhandener Hydroxy- und/ oder Aminoschutzgruppen und deren Ersatz durch Wasserstoff, etwa wie beschrieben, in eine Verbindung der Formel IV umgewandelt, die eine Gruppe IVb enthalten.

Diese Umsetzungen werden in üblicher Weise in einem geeigneten Lösungsmittel und in einem Temperaturbereich von etwa -10 bis etwa +150°C, gegebenenfalls in einer Inertgasatmosphäre, z.B. unter Stickstoff, vorgenommen.

Ausgangsstoffe der Formel IV, worin m in der Gruppe $R_a$ Null ist, können auf übliche Weise erhalten werden, indem man eine Verbindung der Formel

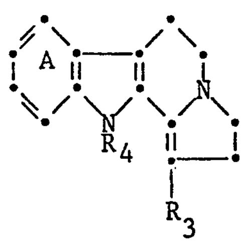

(IVy)

mit einer Verbindung der Formel

$R_2 - CH_2OH$ (IVz)

zu einer Verbindung der Formel IV umsetzt.

Die Hydroxygruppe in einer Verbindung der Formel (IVz) liegt zweckmässigerweise in reaktionsfähiger, veresterter Form, z.B. als Ester mit einer starken organischen Sulfonsäure, wie einer aliphatischen oder aromatischen Sulfonsäure, oder einer Halogenwasserstoffsäure vor und bedeutet demnach z.B. die Methansulfonyloxy- oder die p-Tosyloxygruppe, ferner Halogen, insbesondere Brom und speziell Jod. Eine Gruppe $R_2$ ist insbesondere eine veresterte Carboxygruppe der angegebenen Art, z.B. eine mit einem Niederalkanol, z.B. Methanol, veresterte Carboxygruppe.

Die Umsetzung wird in üblicher Weise in einem geeigneten Lösungsmittel, z.B. einem inerten und gegebenenfalls aprotischen Lösungsmittel, z.B. Dimethylsulfoxid, gegebenenfalls mit einem weiteren Lösungsmittel inerten Charakters, z.B. einem aromatischen Lösungsmittel wie Toluol, bei erhöhter Temperatur, etwa in einem Bereich von +30 - +180°, vorzugsweise etwa +70 - +150° und zweckmässigerweise in einer Inertgasatmosphäre, etwa unter Stickstoff, vorgenommen.

Ausgangsstoffe der Formel IVy sind beispielsweise aus einer Verbindung der Formel IVh durch Abspaltung der Gruppe $X_2$ z.B. mittels Hydrolyse, und deren Ersatz durch Wasserstoff erhältlich. Diese Umsetzungen werden in an sich bekannter Weise vorgenommen.

Die neuen Verbindungen der Formel I können ebenfalls erhalten werden, indem man eine Verbindung der Formel

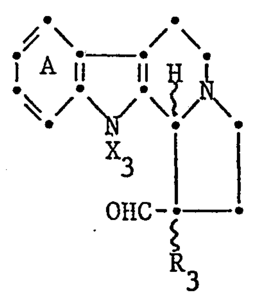

(V),

worin $X_3$ Niederalkyl oder eine unter den Bedingungen des Verfahrens abspaltbare und durch Wasserstoff ersetzbare Gruppe bedeutet, und

Hydroxy- und/oder Aminogruppen gegebenenfalls in geschützter, z.B. mittels Solvolyse, wie Hydrolyse, spaltbarer Form vorliegen, mit einer Verbindung der Formel

$$X_o - CH_2 - R_2' \qquad (Va)$$

worin $X_o$ den Rest der Formel 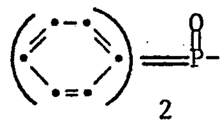 (Vb), oder den Rest der Formel 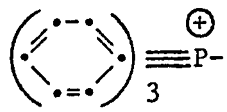 (Vc) bedeutet, der in quaternärer Form, z.B. als quaternäres Halogenid, z.B. als Chlorid, vorliegt, oder $X_o$ den Rest der Formel $(R_5O)_2{=}\overset{O}{\overset{\|}{P}}-$ (Vd) darstellt, worin $R_5$ Niederalkyl, z.B. Aethyl ist, oder $X_o$ Halogen bedeutet, und $R_2'$ gegebenenfalls verestertes oder amidiertes Carboxy darstellt, umsetzt, und eine erhaltene Verbindung durch gleichzeitige oder nachfolgende Abspaltung der abspaltbaren und durch Wasserstoff ersetzbaren Gruppe $X_3$ und gegebenenfalls vorhandene Hydroxy- und/oder Aminoschutzgruppen in eine entsprechende Verbindung der Formel I umwandelt, worin anstelle der Gruppe $-(R_b-R_a)-$ der Rest -CH=CH- steht, und wenn erwünscht, die im Anschluss an das erste Verfahren genannten Verfahrensschritte durchführt.

Eine unter den Bedingungen des Verfahrens abspaltbare Gruppe $X_3$ ist z.B. eine mittels Wasser im basischen oder sauren Medium abspaltbare Gruppe und stellt z.B. den Rest einer organischen Sulfonsäure, etwa Aryl-sulfonyl, z.B. p-Toluolsulfonyl, oder der Rest eines Halbesters der Kohlensäure, wie Niederalkoxycarbonyl, z.B. tert.-Butoxycarbonyl, dar.

Hydroxy- und/oder Aminoschutzgruppen sind z.B. Acylreste von organischen Carbonsäuren, z.B. Niederalkanoyl, wie Acetyl, oder Halbester der Kohlensäure, z.B. Niederalkoxycarbonyl, ferner beispielsweise Trityl- oder Silylreste, wie z.B. Trimethylsilyl. Solche Schutzgruppen werden mittels Wasser, gegebenenfalls in Gegenwart von basischen Mitteln oder schwachen Säuren abgespalten.

Verestertes Carboxy ist z.B. ein mit einem Niederalkanol, z.B. Methanol oder Aethanol, oder einem Niederalkanthiol, wie Aethylmercaptan, verestertes Carboxy, und stellt demnach z.B. Niederalkoxycarbonyl, wie Methoxy- oder Aethoxycarbonyl oder Niederalkylthiocarbonyl, z.B. Aethylthiocarbonyl dar.

Amidiertes Carboxy, worin das Stickstoffatom an zwei von Wasserstoff verschiedene Reste gebunden ist, entspricht der Formel $-CONR_6R_7$ (Ve), worin $R_6$ und $R_7$ jeweils z.B. Niederalkyl, wie Methyl ist, oder $R_6$ und $R_7$ zusammen z.B. Niederalkylen, Oxa-, Thia-, gegebenenfalls N'-substituiertes, wie N'-niederalkyliertes Azaniederalkylen bedeuten und demnach zusammen mit dem N-Atom beispielsweise Pyrrolidino, Morpholino, Piperazino oder N'-Methylpiperazino darstellen. Die Umsetzung mit einer Verbindung der Formel Va, worin $X_o$ eine der Gruppen Vb, Vc oder Vd bedeuten, erfolgt üblicherweise in Gegenwart eines basischen Mittels, z.B. eines Alkalimetallniederalkanolats, wie Kalium-tert.butylat, oder eines Alkalimetallhydrids, wie Natriumhydrid.

Die Umsetzung einer Verbindung der Formel V mit einer solchen der Formel Va, worin $X_o$ für Halogen, beispielsweise Brom, und $R_2''$ vorzugsweise für verestertes Carboxy steht, sowie die gleichzeitige oder nachfolgende Abspaltung der Gruppe $X_3$ und gegebenenfalls vorhandener Hydroxy- und/oder Aminoschutzgruppen erfolgt in üblicher Weise, z.B. unter den Bedingungen der Reformatsky-Reaktion, in Gegenwart von Zink in einem geeigneten, inerten Lösungsmittel, etwa einem aromatischen Lösungsmittel, wie Benzol, oder einer ätherartigen Flüssigkeit, wie Diäthyläther. Hierbei erhält man unter Wasserabspaltung eine entsprechende Verbindung der Formel I, worin anstelle der Gruppe $-(R_b-R_a)-$ der Rest $-CH=CH-$ steht.

Diese Reaktionen werden üblicherweise, z.B. in Gegenwart eines Lösungsmittels oder Lösungsmittelgemisches durchgeführt, wenn notwendig, unter Kühlen oder Erwärmen, z.B. in einem offenen oder geschlossenen Gefäss und/oder in einer Inertgasatmosphäre, z.B. Stickstoff.

Ausgangsstoffe der Formel V können in an sich bekannter Weise hergestellt werden.

So kann man z.B. eine Verbindung der Formel

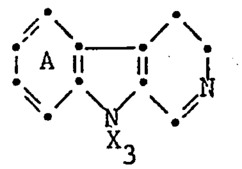

(Vf),

mit einem Aldehyd der Formel IIb oder einem reaktionsfähigen Derivat davon, etwa einem solchen, worin die Aldehydgruppe in geschützter, z.B. silylierter Form vorliegt, und demnach z.B der Gruppe IIc entspricht, und die Hydroxygruppe in reaktionsfähiger, veresterter Form vorliegt, zu einer Verbindung der Formel V umsetzen, und anschliessend eine gegebenenfalls vorhandene Aldehydschutzgruppe abspalten.

Eine reaktionsfähige veresterte Hydroxygruppe ist eine, durch eine starke Säure, insbesondere eine starke anorganische Säure, wie eine Halogenwasserstoffsäure, insbesondere Chlor-, Brom- oder Jodwasserstoffsäure, oder Schwefelsäure, oder eine starke organische Säure, insbesondere eine starke organische Sulfonsäure, wie eine aliphatische oder aromatische Sulfonsäure, z.B. Methansulfonsäure, 4-Methylphenylsulfonsäure oder 4-Bromphenylsulfonsäure, veresterte Hydroxygruppe, und stellt in erster Linie Halogen, z.B. Brom, oder aliphatisch oder aromatisch substituiertes Sulfonyloxy, z.B. Methylsulfonyloxy oder 4-Methylphenylsulfonyloxy dar.

Die obige Reaktion wird in an sich bekannter Weise durchgeführt, wobei man, besonders bei Verwendung eines Ausgangsmaterials mit einer reaktionsfähigen veresterten Hydroxygruppe, vorteilhafterweise in Gegenwart eines basischen Mittels, wie eines organischen basischen Mittels, etwa eines Amins, z.B. Aethyldiisopropylamin, üblicherweise in einem geeigneten Lösungsmittel, etwa einem solchen stark polaren, aprotischen Charakters, z.B. Dimethylformamid, in einem Temperaturbereich von etwa -5° bis +150°, vorzugsweise von +10° bis +100°, in

einem offenen oder geschlossenen Gefäss und/oder in einer Inertgasatmosphäre, z.B. Stickstoff, arbeitet.

Ausgangsstoffe der Formel Va sind bekannt oder können nach üblichen Methoden erhalten werden. So kann man z.B. zur Herstellung eines Ausgangsstoffs der Formel Va, worin $X_o$ für die Gruppe Vc steht, Triphenylphosphin mit einer Verbindung der Formel Va umsetzen, worin $X_o$ für Halogen, etwa Chlor steht. Ausgangsstoffe der Formel Va, worin $X_o$ für die Gruppe Vd steht, können durch Umsetzung eines Trialkylphosphits der Formel

$$(R_5O)_2 = P - OR_5 \qquad (Vh)$$

mit einer Verbindung der Formel Va, worin $X_o$ für Halogen, etwa Chlor, steht, erhalten werden.

Diese Umsetzungen werden in üblicher Weise durchgeführt.

Die neuen Verbindungen der Formel I können ebenfalls erhalten werden, indem man in einer Verbindung der Formel

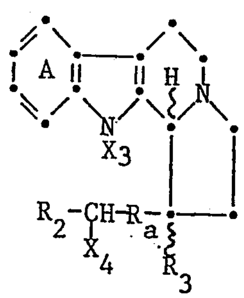

(VI),

worin $X_3$ Niederalkyl oder eine unter den Bedingungen des Verfahrens abspaltbare und durch Wasserstoff ersetzbare Gruppe bedeutet, und $X_4$ Halogen oder eine mit einer organischen Sulfonsäure veresterte Hydroxygruppe bedeutet, die Gruppe $X_4$ abspaltet und durch der Bedeutung von $R_1$ entsprechendes, gegebenenfalls substituiertes Amino, gegebenenfalls verestertes oder veräthertes Hydroxy oder Niederalkylthio ersetzt und gleichzeitig oder nachfolgend eine abspaltbare und durch Wasserstoff ersetzbare Gruppe $X_3$ abspaltet und durch Wasserstoff ersetzt, und, wenn

erwünscht, die im Anschluss an das erste Verfahren beschriebenen Verfahrensschritte durchführt. Eine unter den Bedingungen des Verfahrens abspaltbare und durch Wasserstoff ersetzbare Gruppe $X_3$ ist eine mittels Wasser im basischen oder sauren Medium oder mittels Ammonolyse abspaltbare Gruppe und stellt z.B. den Rest einer organischen Sulfonsäure, z.B. p-Toluolsulfonyl oder den Rest eines Halbesters der Kohlensäure, z.B. Aethoxycarbonyl oder tert.Butoxycarbonyl, dar.

Die Abspaltung der Gruppe $X_4$ und deren Austausch z.B. gegen die gegebenenfalls substituierte Aminogruppe erfolgt durch Umsetzung mit einer die Einführung einer solchen Aminogruppe erlaubenden Verbindung. So kann man z.B. eine Verbindung der Formel VI, worin $X_4$ beispielsweise Halogen, etwa Chlor, ist, mit Hexamethylentetramin in üblicher Weise, z.B. in einem geeigneten inerten Lösungsmittel, wie Chloroform, umsetzen, und das erhaltene Addukt im sauren Medium, z.B. in Gegenwart einer Halogenwasserstoffsäure, etwa wässriger Salzsäure, in eine Verbindung der Formel VI umwandeln, worin anstelle von $X_4$ die primäre Aminogruppe steht, und gleichzeitig eine Schutzgruppe $X_3$, z.B. die tert.-Butoxygruppe, abspalten und durch Wasserstoff ersetzen. Man kann aber auch so vorgehen, dass man den Austausch der Gruppe $X_4$ gegen Amino mittels Ammoniak, z.B. als Lösung in einem geeigneten Lösungsmittel, etwa eines solchen inerten, z.B. aromatischen Charakters, wie Benzol, oder einer ätherartigen Flüssigkeit, wie Dioxan, oder mittels eines primären oder sekundären Amins, gegebenenfalls in Gegenwart eines säurebindenden Mittels, etwa eines basischen Mittels, z.B. Kaliumcarbonat, oder eines Ueberschusses des der einzuführenden Aminogruppe entsprechenden Amins, vornimmt, wobei gleichzeitig eine geeignete Schutzgruppe $X_3$, z.B. die genannte tert.-Butoxygruppe, abgespalten wird.

Der Austausch einer Gruppe $X_4$, beispielsweise Chlor, gegen der Bedeutung von $R_1$ entsprechendes Hydroxy kann in üblicher Weise, z.B. mittels Wasser, gegebenenfalls in Gegenwart einer Säure oder einer Base, z.B. eines Hydroxids oder Carbonats eines Alkalimetall- oder

Erdalkalimetalls, z.B. Natriumhydroxid, Natriumcarbonat oder Magnesiumcarbonat, oder einer wässrigen Mineralsäure, etwa wässriger Schwefelsäure unter gleichzeitiger Abspaltung einer Schutzgruppe $X_3$, z.B. Aethoxycarbonyl oder n-Butoxycarbonyl, vorgenommen werden.

Der Austausch einer Gruppe $X_4$ gegen verestertes Hydroxy, beispielsweise Aroyloxy, Benzolsulfonyloxy oder Niederalkanoyloxy, kann z.B. durch Umsetzung mit einem geeigneten Salz, etwa einem Alkalimetallsalz, z.B. Natrium- oder Kaliumsalz einer der einzuführenden veresterten Hydroxygruppe entsprechenden Säure, etwa einer gegebenenfalls, etwa wie angegeben, substituierten aromatischen Carbon- oder Sulfonsäure oder einer Niederalkancarbonsäure, zweckmässigerweise in einem geeigneten Lösungsmittel, etwa einem solchen polaren Charakters, etwa einem Alkohol, wie einem Niederalkanol, etwa Aethanol, oder einem Carbonsäureamid, wie etwa N-Methylacetamid, N,N-Dimethylacetamid, N,N,N',N'-Tetramethylharnstoff, oder Sulfolan, erfolgen.

Die Abspaltung einer Gruppe $X_4$, etwa Chlor, und deren Austausch gegen der Bedeutung von $R_1$ entsprechendes veräthertes Hydroxy, z.B. Niederalkoxy, oder Niederalkylthio erfolgt in üblicher Weise durch Umsetzung mit einem der verätherten Hydroxygruppe bzw. Niederalkylthiogruppe entsprechenden Alkanol bzw. Niederalkanthiol, zweckmässigerweise in Form einer Metallverbindung. So kann man beispielsweise eine Alkalimetallverbindung eines Niederalkanols, bzw. Niederalkanthiols, etwa die des Aethanols, wie Natriumäthoxid, oder des Aethylmercaptans, wie dessen Natriumverbindung, mit einer Verbindung der Formel VI umsetzen, worin $X_4$ beispielsweise Halogen, wie Chlor, oder Methansulfonyloxy ist, wobei die Gruppe $X_4$ gegen entsprechendes Niederalkoxy bzw. Niederalkylthio ersetzt und eine Schutzgruppe $X_3$ beispielsweise Benzolsulfonyl oder Aethoxycarbonyl, abgespalten und durch Wasserstoff ersetzt wird.

Diese Umsetzungen werden in üblicher Weise in Gegenwart eines Lösungsmittels unter Kühlen oder Erwärmen in einem Temperaturbereich von etwa -20 bis etwa +120° gegebenenfalls in einer Schutzgasatmosphäre, etwa unter Stickstoff, durchgeführt.

Ausgangsstoffe der Formel VI können, soweit sie neu sind, auf an sich bekannte Weise erhalten werden, indem man z.B. eine Verbindung der Formel

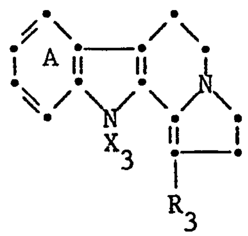

(VIa)

mit einer Verbindung der Formel

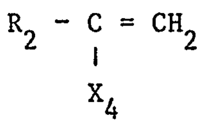

(VIb)

und anschliessender Salzbildung, z.B. mit Perchlorsäure, zu einer Verbindung der Formel

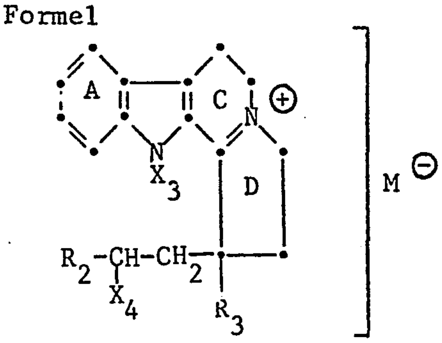

(VIc)

umsetzt.

Diese Umsetzung wird üblicherweise in Gegenwart eines geeigneten Lösungsmittels inerten Charakters, z.B. eines halogenierten Kohlenwasserstoffs, etwa Methylenchlorid, vorgenommen.

Hierin schliesst sich die Reduktion der den Ringen C und D gemeinsamen Doppelbindung zu einer Verbindung der Formel VI an, die in üblicher Weise, z.B. mittels Lithium-tri-tert.butoxy-aluminiumhydrid, erfolgen kann.

Ausgangsstoffe der Formel VIa wiederum können analog der für die Herstellung einer Verbindung der Formel IIIe beschriebenen Reaktionsfolge IIIa-IIIe wie folgt erhalten werden:

Man setzt eine Tryptaminverbindung der Formel

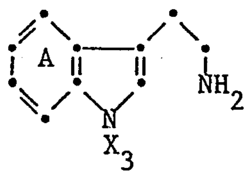

(VId)

beispielsweise mit einer Verbindung der Formel

$$Cl-CH_2-CH_2-\underset{R_3}{\underset{|}{CH}}-COCl$$

(VIe)

zu einer Verbindung der Formel

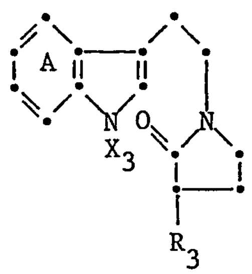

(VIf)

um, die anschliessend in üblicher Weise durch ringschliessende Kondensation, z.B. in Gegenwart eines geeigneten Kondensationsmittels, etwa eines solchen sauren Charakters, etwa eines Säurehalogenids, z.B. Phosphoroxychlorid, in eine Verbindung der Formel

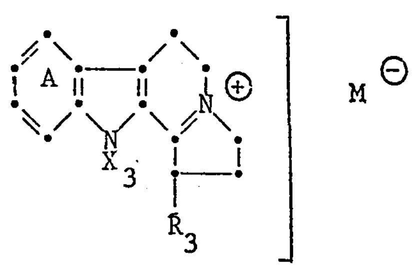

(VIg)

umgewandelt wird, aus welcher durch Behandeln mit einem basischen Mittel, z.B. einer anorganischen Base, etwa einem Alkalihydroxid wie z.B. Natriumhydroxid, ein Ausgangsmaterial der

Formel VIa erhalten wird. Ausgangsstoffe der Formel VIe wiederum können auf übliche Weise durch Umsetzung eines in 2-Stellung gegebenenfalls niederalkylierten, z.B. äthylierten, γ-Butyrolacton der Formel IIId mit Thionylchlorid in Gegenwart eines geeigneten Kondensationsmittels, etwa wasserfreiem Zinkchlorid, erhalten werden.

Die neuen Verbindungen der Formel I, worin $R_1$ für Amino steht, können ebenfalls erhalten werden, indem man in einer Verbindung der Formel

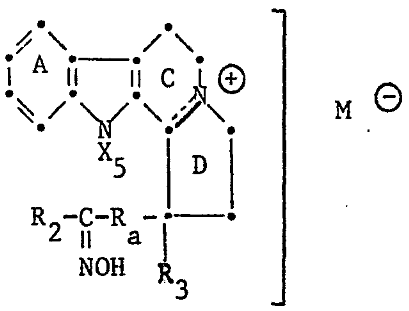

(VII),

worin $X_5$ Niederalkyl, Niederalkenyl oder eine unter den Bedingungen der Reduktion abspaltbare und durch Wasserstoff ersetzbare Gruppe bedeutet, die Gruppe $>C=NOH$ (VIIa) zur Gruppe $>CHNH_2$ (VIIb), und eine gegebenenfalls vorhandene den Ringen C und D gemeinsame Doppelbindung, wobei in einem solchen Falle das den Ringen C und D gemeinsame N-Atom eine positive Ladung trägt und das Anion M die angegebene Bedeutung hat, zur Kohlenstoff-Stickstoff-Einfachbindung, und/oder eine gegebenenfalls vorhandene Niederalkenylgruppe $X_5$ zur Niederalkylgruppe reduziert, und/oder eine gegebenenfalls vorhandene, unter den Bedingungen der Reduktion abspaltbare und durch Wasserstoff ersetzbare Gruppe $X_5$, und an Hydroxy- und/oder Aminogruppen gegebenenfalls stehende unter den Bedingungen der Reduktion abspaltbare und durch Wasserstoff ersetzbare Schutzgruppen abspaltet und durch Wasserstoff ersetzt, und, wenn erwünscht, die im Anschlusss an das erste Verfahren beschriebenen Verfahrensschritte durchführt.

Eine besonders geeignete abspaltbare Gruppe $X_5$ sowie an Hydroxy- und/oder Aminogruppen gegebenenfalls gebundene Schutzgruppen sind in erster Linie eine hydrogenolytisch abspaltbare α-Arylniederalkylgruppe, wie eine gegebenenfalls substituierte 1-Polyphenylniederalkyl-

oder 1-Phenylniederalkylgruppe, worin Substituenten, insbesondere des Phenylteils, z.B. Niederalkyl, wie Methyl, oder Niederalkoxy, wie Methoxy sein können, und in erster Linie Benzyl.

In Betracht kommen ferner geeignete Acylgruppen, wie im Phenylteil gegebenenfalls,z,B. wie oben angegeben, substituiertes 1-Phenylniederalkoxycarbonyl.

Die Reduktion wird in üblicher Weise durchgeführt, beispielsweise durch Behandeln mit katalytisch aktiviertem Wasserstoff, wie Wasserstoff in Gegenwart eines Hydrierkatalysators, z.B. eines Nickelkatalysators, wie Raney-Nickel, oder eines Edelmetallkatalysators, wie Platin oder Palladium, wobei die den Ringen C und D gemeinsame Doppelbindung zur Kohlenstoff-Stickstoff-Einfachbindung, die Gruppe VIIa zur Gruppe VIIb, eine Niederalkenylgruppe $X_5$ zur Niederalkylgruppe reduziert oder eine abspaltbare Schutzgruppe $X_5$ sowie gegebenenfalls an Hydroxy- und/oder Aminogruppen gebundene Schutzgruppen abgespalten und durch Wasserstoff ersetzt werden. Man kann die Reduktion auch stufenweise durchführen, indem man die den Ringen C und D gemeinsame Doppelbindung zur Kohlenstoff-Stickstoff-Einfachbindung reduziert und in einer so erhaltenen, der Formel VII analogen Verbindung die Gruppen VIIa zur Gruppe VIIb, eine Niederalkenylgruppe $X_5$ zur Niederalkylgruppe reduziert oder eine abspaltbare und durch Wasserstoff ersetzbare Schutzgruppe $X_5$ sowie gegebenenfalls vorhandene, an Hydroxy- und/oder Aminogruppen stehende, Schutzgruppen abspaltet und durch Wasserstoff ersetzt. Die Reduktion der den Ringen C und D gemeinsamen Doppelbindung kann z.B. mittels eines geeigneten Hydridreduktionsmittels, etwa einem Dileichtmetallhydridreduktionsmittel, z.B. Lithium-tri-tert.butoxyaluminiumhydrid, erfolgen, während die anschliessende Reduktion der Gruppe VIIa zur Gruppe VIIb und die Abspaltung der Schutzgruppen z.B. mittels aktiviertem Wasserstoff, etwa wie angegeben, oder mittels eines Alkalimetalls, etwa Natrium, in einem Alkohol, etwa einem Niederalkanol, wie Aethanol, durchgeführt werden kann.

Ausgangsstoffe der Formel VII können auf an sich bekannte Weise hergestellt werden. Man kann sie z.B. erhalten, indem man eine Verbindung der Formel

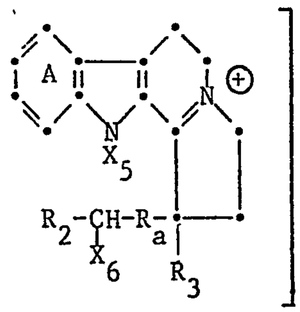

$M^{\ominus}$ (VIIc)

worin $X_6$ für Wasserstoff oder eine geeignete Abgangsgruppe, z.B. Chlor Brom oder Jod ist,mit einem nitrosierend wirkenden Agens umsetzt. Als solches kann man z.B. salpetrige Säure verwenden die man zweckmässigerweise aus einem Salz der salpetrigen Säure, beispielsweise einem Alkalimetallsalz, z.B. dem Natriumsalz, im sauren Medium, etwa in Essigsäure, in situ erhält.

Ausgangsstoffe der Formel VIIc wiederum können hergestellt werden, indem man eine Tryptaminverbindung der Formel

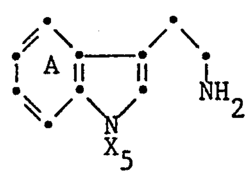

(VIId)

z.B. mit einem 4-Chlorbutyrylchlorid der Formel

$$Cl - CH_2 - CH_2 - \underset{R_3}{CH} - COCl \qquad (VIIe)$$

zu einer Verbindung der Formel

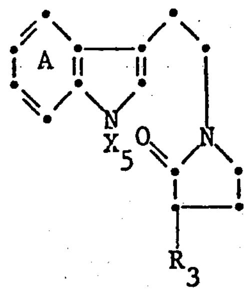

(VIIf)

umsetzt, und hieraus durch ringschliessende Kondensation, z.B. in Gegenwart eines geeigneten Kondensationsmittels, etwa eines solchen sauren Charakters, z.B. eines Säurehalogenids, wie Phosphoroxychlorid, eine Verbindung der Formel

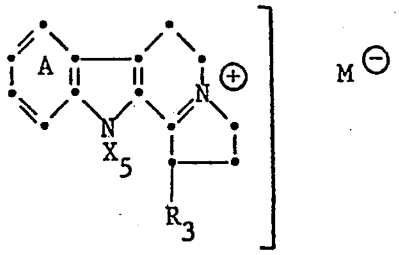

(VIIg)

herstellt, aus welcher durch Behandeln mit einem basischen Mittel, z.B. einer anorganischen Base, etwa einem Alkalihydroxid, wie Natriumhydroxid, eine Verbindung der Formel

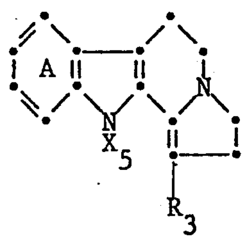

(VIIh)

erhalten wird. Durch Umsetzung einer solchen Verbindung z.B. mit einer Verbindung der Formel

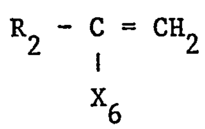

(VIIi)

in Gegenwart eines geeigneten Lösungsmittels, etwa eines halogenierten Kohlenwasserstoffs, wie Dichlormethan, und nachfolgende Salzbildung, etwa mit Perchlorsäure, kann eine Verbindung der Formel VIIc erhalten werden, worin m der Gruppe $R_a$ 1 ist. Diese Umsetzungen werden in an sich bekannter Weise vorgenommen.

Die neuen Verbindungen der Formel I, worin $R_2$ für gegebenenfalls verestertes oder amidiertes Carboxy und $R_4$ für Wasserstoff steht, können ebenfalls erhalten werden, indem man in einer Verbindung der Formel

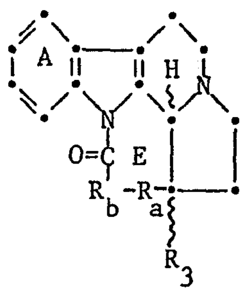

(VIII)

den Ring E mittels Alkoholyse, Aminolyse einschliesslich Ammonolyse oder Hydrolyse unter Bildung einer gegebenenfalls veresterten oder amidierten Carboxygruppe $R_2$ öffnet und, wenn erwünscht, die im Anschluss an das erste Verfahren beschriebenen Verfahrensschritte durchführt. Die Alkoholyse kann in Ab- oder Anwesenheit beschleunigend wirkender Mittel, z.B. basischer Substanzen, durchgeführt werden. Zweckmässigerweise führt man die Umsetzung jedoch im basischen Medium, z.B. in Gegenwart geeigneter Basen, etwa einer Metallverbindung des betreffenden Alkohols durch, wobei letzterer auch als Lösungsmittel dienen kann. Metallverbindungen dieser Art sind z.B. solche der Erdalkalimetalle, insbesondere aber der Alkalimetalle und stellen demnach beispielsweise ein Magnesiumniederalkanolat, etwa Magnesiumäthylat, oder ein Alkalimetallniederlakoholat, etwa Natriummethoxid, dar.

Die Aminolyse einschliesslich Ammonolyse wird zweckmässigerweise in einem geeigneten, beispielsweise inerten, Lösungsmittel durchgeführt. Solche sind z.B. Flüssigkeiten ätherartigen Charakters, etwa Diäthyl- oder Di-n-butyläther, Tetrahydrofuran oder Dioxan, oder es handelt sich um aromatische Lösungsmittel,wie z.B. Benzol, Toluol oder Xylol. Für die Ammonolyse kann eine Lösung von Ammoniak in einem der genannten Lösungsmittel, jedoch auch eine wässrige Lösung eingesetzt werden.

Die Hydrolyse wird mittels Wasser, zweckmässigerweise im sauren, vorteilhafterweise jedoch im basischen Medium durchgeführt. Als Säuren kommen z.B. Mineralsäuren, wie Schwefelsäure, oder organische Säuren, z.B. Essigsäure oder Trifluoressigsäure, in Betracht, während basische Mittel beispielsweise anorganische Basen, wie Hydroxide von Erdalkali- oder Alkalimetallen, z.B. Magnesiumhydroxid oder Natriumhydroxid sind.

Die obigen Umsetzungen werden in an sich bekannter Weise und üblicherweise in Gegenwart eines Lösungsmittels, z.B. eines der genannten, oder eines Lösungsmittelgemisches und, wenn notwendig, unter Kühlen oder Erwärmen, z.B. in einem Temperaturbereich von etwa -20° bis etwa +150° in einem offenen oder geschlossenen Gefäss und/oder in einer Inertgasatmosphäre, z.B. in einer Stickstoffatmosphäre durchgeführt.

Ausgangsstoffe der Formel VIII können in an sich bekannter Weise hergestellt werden. So kann man z.B. in einer Verbindung der Formel

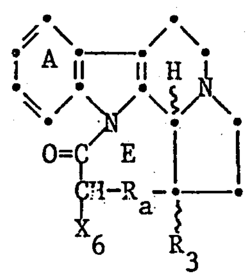

(VIIIb)

worin $X_6$ die oben genannte Bedeutung als Wasserstoff oder einer geeigneten Abgangsgruppe, z.B. Chlor, Brom oder Jod hat, ferner aber auch eine mit einer Sulfonsäure veresterte Hydroxygruppe, z.B. die Methansulfonylgruppe, darstellt, die Gruppe $X_6$ durch der Bedeutung von $R_1$ in der Gruppe $R_b$ entsprechendes gegebenenfalls verestertes Hydroxy, Niederalkylthio oder gegebenenfalls substituiertes Amino oder Niederalkyl ersetzen. So kann man eine Verbindung der Formel VIIIb, worin $X_6$

verschieden von Wasserstoff ist und etwa Chlor oder Brom bedeutet, dieses durch veräthertes Hydroxy, beispielsweise Niederalkoxy oder Phenylniederalkoxy, oder durch Niederalkylthio ersetzen, indem man einen diesen Gruppen entsprechenden Alkohol oder Niederalkenthiol, zweckmässigerweise in Gegenwart eines basischen Mittels, z.B. eines Metallniederalkanolats, etwa Natriumniederalkanolats, mit einer solchen Verbindung der Formel VIIIb umsetzt und gegebenenfalls vorhandene Hydroxy- und/oder Aminoschutzgruppen, die unter den Reaktionsbedingungen abspaltbar sind, abspaltet und durch Wasserstoff ersetzt.Oder man setzt den verwendeten Alkohol oder das entsprechende Niederalkanthiol als Metallverbindung, z.B. als solches mit einem Alkalimetall, z.B. Natrium, für diese Umsetzung ein, die man in üblicher Weise in einem geeigneten Lösungsmittel, etwa einem Niederalkanol, wie Aethanol, durchführt.

Zum Einführen von gegebenenfalls substituiertem, etwa niederalkyliertem Amino, ferner Niederalkylenamino, Oxaniederalkylenamino, Thianiederalkylenamino oder Azaniederalkylenamino, kann man eine Verbindung der Formel VIIIb, worin $X_6$ verschieden von Wasserstoff ist, z.B. mit Ammoniak oder einer solches abgebenden Verbindung, etwa Hexamethylentetramin, oder einer Phthalimid-Verbindung, z.B. Phthalimidkalium, oder einem die genannten Substituenten enthaltenden primären oder sekundären Amin in üblicher Weise, in Ab- oder Anwesenheit eines geeigneten Lösungsmittels, und/oder in Gegenwart eines säurebindenden Mittels, etwa eines basischen Mittels, wie Kaliumcarbonat, oder eines Ueberschusses an dem der einzuführenden Aminogruppe entsprechendem Amin, gegebenenfalls bei erhöhter oder erniedrigter Temperatur, in einem offenen oder geschlossenen Gefäss umsetzen. Erfolgt die Umsetzung mit Hexamethylentetramin, so schliesst sich hieran die Zusetzung des intermediär gebildeten Addukts im sauren Medium, etwa mittels verdünnter Salzsäure, an, während aus dem durch Umsetzung z.B. mittels Phthalimidkalium erhaltenen Zwischenprodukt die Phthaloylgruppe, etwa mittels Hydrazinhydrat, abgespalten werden muss. In den letztgenannten Fällen wird auf die beschriebene Weise die primäre Aminogruppe eingeführt und gegebenenfalls vorhandene, unter den beschriebenen Reaktionsbedingungen abspaltbare Hydroxy- und/oder Aminoschutzgruppen abgespalten und durch Wasserstoff ersetzt.

Die Einführung einer primären Aminogruppe kann auch so erfolgen, indem man eine Verbindung der Formel VIIIb, worin $X_6$ die angegebene Bedeutung hat, und worin insbesondere Aminogruppen durch Schutzgruppen geschützt sind, beispielsweise solche, die mittels Reduktion, wie Hydrogenolyse, abspaltbar und durch Wasserstoff ersetzbar sind, wie z.B. α-Aralkylgruppen wie 1-Phenylniederalkylgruppen, z.B. Benzyl, anschliessend mit einem nitrosierend wirkenden Agens, etwa salpeteriger Säure, die man zweckmässigerweise in situ im Reaktionsgemisch bildet, etwa aus einem Salz, z.B. Alkalimetallsalz wie Natriumnitrit, oder einem Ester, z.B. tert.-Butylnitrit, in Gegenwart einer Säure, zu einer Verbindung der Formel VIIIb umsetzt, worin die Gruppe $-CHX_6-$ der Gruppe $-C(=NOH)-$ entspricht, wobei die gebildete Hydroximinogruppe in der syn- oder anti-Form oder als Gemisch dieser beiden Formen vorliegen kann, und die Hydroximinogruppe zur primären Aminogruppe reduziert. Die Reduktion der Hydroximinogruppe zur primären Aminogruppe kann beispielsweise mit aktiviertem Wasserstoff, z.B. Wasserstoff in Gegenwart eines Hydrierkatalysators, wie Raney-Nickel, oder eines Edelmetallkatalysators, wie Platin oder Palladium, erfolgen, wobei gegebenenfalls vorhandene, unter den Bedingungen dieser Reduktion abspaltbare an Hydroxy-und insbesondere an Aminogruppen stehende Schutzgruppen, z.B. α-Aryl-niederalkyl-, z.B. 1-Phenylniederalkylgruppen, etwa Benzyl, abgespalten und durch Wasserstoff ersetzt werden; oder man reduziert beispielsweise mittels nascierendem Wasserstoff, z.B. mittels einer Säure, etwa einer Mineralsäure, wie Schwefelsäure, in Gegenwart eines geeigneten Metalls, etwa Zink, oder mittels eines Amalgams,z.B. eines Alkalimetallamalgams, wie Natriumamalgam, in Wasser, oder mittels eines Alkalimetalls, z.B. Natrium in einem Niederalkanol, wie Aethanol, wobei gegebenenfalls an Hydroxy- und/oder Aminogruppen stehende, unter den Bedingungen der Reduktion abspaltbare Schutzgruppen, beispielsweise Silylgruppen, etwa die Trimethylsilylgruppe, oder eine Niederalkoxycarbonylgruppe, etwa tert.-Butoxycarbonyl, abgespalten und durch Wasserstoff ersetzt werden.

Der Austausch einer Gruppe $X_6$, z.B. Chlor, gegen der Bedeutung von $R_1$ entsprechendes Hydroxy kann in üblicher Weise mittels Hydrolyse, gegebenenfalls in Gegenwart einer Säure oder einer Base, z.B. eines Hydroxids oder Carbonats eines Alkali- oder Erdalkalimetalls, z.B. Kaliumhydroxid, Natriumbicarbonat oder Magnesiumcarbonat erfolgen, wobei unter diesen Bedingungen abspaltbare gegebenenfalls vorhandene Hydroxy- und/oder Aminoschutzgruppen abgespalten und durch Wasserstoff ersetzt werden.

Auf die vorstehend beschriebene Weise eingeführte Hydroxy- oder Aminogruppen können in verestertes Hydroxy bzw. acyliertes Amino überführt werden. Beispielsweise setzt man eine erhaltene Verbindung der Formel VIIIb, worin anstelle von $X_6$ Hydroxy steht, mit einer aromatischen Carbon- oder Sulfonsäure oder einer Niederalkancarbonsäure, oder einem reaktionsfähigen Derivat davon, etwa einem Anhydrid, gemischten Anhydrid, etwa einem Halogenid, wie Säurechlorid, oder einem reaktionsfähigen Ester, beispielsweise einem solchen, dessen alkoholische Komponente durch geeignete Gruppen oder Substituenten aktiviert ist, auf übliche Weise um. In analoger Weise kann eine freie Aminogruppe in acyliertes Amino, etwa Niederalkanoylamino, z.B. durch Umsetzung mit einem entsprechenden Säureanhydrid, umgewandelt werden. Mit der Einführung der

vorstehend beschriebenen gegebenenfalls verätherten Hydroxygruppen oder von gegebenenfalls substituierten Aminogruppen in eine Verbindung der Formel VIIIb kann zu gleicher Zeit die beschriebene Oeffnung des Ringes E bewirkt werden, die dann zu Verbindungen der Formel I führt, worin $R_2$ gegebenenfalls verestertes oder amidiertes Carboxy darstellt.

Der Austausch einer Gruppe $X_6$ als Wasserstoff gegen der Bedeutung von $R_1$ entsprechendes Niederalkyl kann z.B. in der Weise erfolgen, dass man eine Verbindung der Formel VIIIb, worin $X_6$ Wasserstoff ist, dieses Wasserstoffatom gegen ein geeignetes Metall, welches seinerseits weiteren Umsetzungen zugänglich ist, z.B. ein Alkalimetall, ersetzt etwa durch Umsetzung mit einer reaktionsfähigen Alkalimetallverbindung wie Lithiumisopropylamid, und die erhaltene Verbindung mit einem reaktionsfähigen Ester eines Niederalkanols, beispielsweise einem Halogenid, wie Chlorid, Bromid oder Jodid, oder einem Sulfonsäureester, wie etwa einem aliphatischen oder aromatischen Charakters, wie einem Methansulfonsäure- oder p-Toluolsulfonsäureester, umsetzt. Diese Umsetzungen werden in üblicher Weise in einem geeigneten, z.B. inerten, Lösungsmittel unter Kühlen oder Erwärmen im offenen oder geschlossenen Gefäss, gegebenenfalls in einer Schutzgasatmosphäre, wie Stickstoff, vorgenommen.

Ausgangsstoffe der Formel VIIIb wiederum, worin $R_a$ die Gruppe $-CH_2-$ bedeutet, können erhalten werden, indem man z.B. eine Tryptaminverbindung der Formel

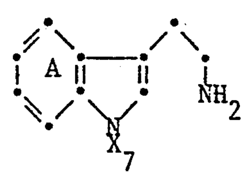

(VIIIc),

die ihrerseits z.B. analog der für die Umwandlung einer Verbindung der Formel IIIa in eine solche der Formel IIIb beschriebenen Verfahrensweise zugänglich ist, worin $X_7$ Niederalkyl oder den Rest einer organischen Sulfonsäure, z.B. den p-Tosylrest, oder den Rest eines Halbesters der Kohlensäure, z.B. ein Niederalkoxycarbonyl, wie Methoxy- oder Aethoxycarbonyl darstellt, z.B. mit einem 4-Chlorbutyrylchlorid der oben erläuterten Formel IIIe zu einer Verbindung der Formel

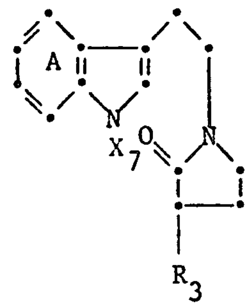

(VIIId)

umsetzt, und hieraus durch ringschliessende Kondensation, z.B. in Gegenwart eines geeigneten Kondensationsmittels, etwa eines solchen sauren Charakters, z.B. eines Säurehalogenids, wie Phosphoroxychlorid, eine Verbindung der Formel

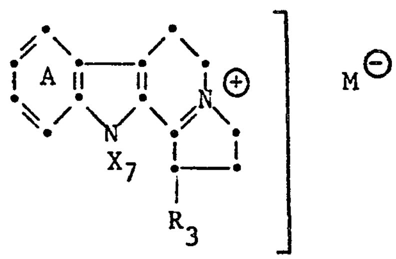

(VIIIe)

herstellt, aus welcher durch Behandeln mit einem basischen Mittel, z.B. einer anorganischen Base, etwa einem Alkalihydroxid, wie Natriumhydroxid, und gleichzeitiger oder nachfolgender Abspaltung einer abspaltbaren und durch Wasserstoff ersetzbaren Gruppe $X_7$ und deren Ersatz durch Wasserstoff eine Verbindung der Formel

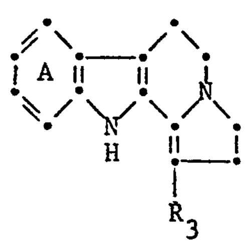

(VIIIf)

erhalten werden kann. Durch anschliessende Umsetzung einer solchen Verbindung z.B. mit einer Acrylverbindung der Formel

$$R_2 - \underset{X_6}{C} = CH_2 \qquad \text{(VIIIg)}$$

und nachfolgende Reduktion der in einer schliesslich erhaltenen Ver-

bindung der Formel

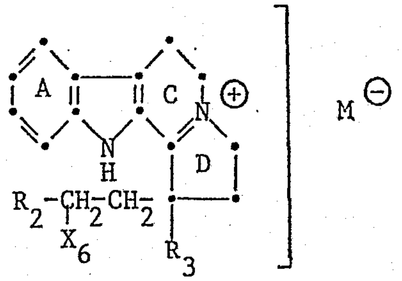

(VIIIh)

vorhandenen, den Ringen C und D gemeinsamen Doppelbindung zur Kohlenstoff-Stickstoff-Einfachbindung, etwa mittels Wasserstoff in Gegenwart eines Hydrierkatalysators, wie eines Palladium-auf-Kohle-Katalysators, erhält man eine Verbindung der Formel

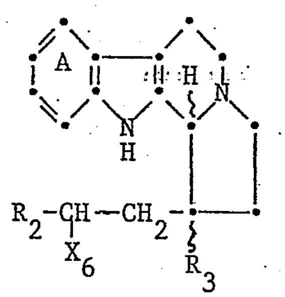

(VIIIi),

aus welcher, insbesondere wenn $R_2$ eine veresterte Carboxygruppe, etwa Niederalkoxycarbonyl darstellt, und $X_6$ Wasserstoff ist, durch ringschliessende Kondensation in üblicher Weise, z.B. im basischen Medium, etwa in Gegenwart eines Alkalimetallniederalkanolats, z.B. Kalium-tert. butylat, vorzugsweise in Gegenwart eines zur Enolatbildung befähigten Stoffes, beispielsweise einer Carbonylverbindung, an deren einem der Carbonylgruppe benachbarten Kohlenstoffatom ein bewegliches, zur Enolbildung befähigtes Wasserstoffatom gebunden ist, z.B. eines Arylniederalkylketons, wie Acetophenon, oder eines 1,3-Diketons, z.B. eines Acylacetons, etwa eines Niederalkanoylacetons wie Acetylaceton, eine Verbindung der Formel VIIIb erhalten werden kann, worin $X_6$ Wasserstoff bedeutet.

Diese Umsetzungen werden in üblicher Weise vorgenommen.

Ausgangsstoffe der Formel VIIIb, worin m in der Gruppe $R_a$ Null ist, und $X_6$ Wasserstoff bedeutet, können z.B. durch Umsetzung einer Verbindung der Formel VIIIf mit einer Verbindung der oben erläuterten Formel

IVz auf die im Anschluss daran dort beschriebene Weise und anschliessende Cyclisierung einer so erhaltenen Verbindung, etwa wie vorstehend beschrieben, in üblicher Weise erhalten werden.

In den Ausgangsstoffen der Formel VIIIb, worin $X_6$ Wasserstoff bedeutet, kann ein an einem der Carbonylgruppe benachbart stehenden Kohlenstoffatom gebundenes Wasserstoffatom durch z.B. der Gruppe $X_6$ entsprechendes Halogen, etwa Chlor, Brom oder Jod ersetzt werden, indem man eine, z.B. wie vorstehend beschrieben, erhaltene Verbindung der Formel VIIIb, worin $X_6$ Wasserstoff ist, in eine entsprechende ein Carbanion enthaltende Verbindung umwandelt, z.B. durch Umsetzung mit Lithiumdiisopropylamid in einem geeigneten Lösungsmittel, etwa Tetrahydrofuran, anschliessend das Reaktionsprodukt mit einem Kohlenstofftetrahalogenid, etwa Tetrachlor- oder Tetrabrommethan oder einer Lösung von Jod in Tetrahydrofuran, umsetzt und mittels saurer Mittel, etwa wässriger Salzsäure hydrolysiert. (Vgl. hierzu: R.T. Arnold et al.: J. Org. Chem. 43, 3687-89 (1978). Der Ersatz einer Gruppe $X_6$ als Wasserstoff in einer Verbindung der Formel VIIIb gegen entsprechendes Hydroxy kann aber auch so erfolgen, indem man z.B. ein Umsetzungsprodukt mit Lithiumdiisopropylamid z.B., ein solches wie vorstehend beschrieben, durch Umsetzung mit Trimethylchlorsilan in eine Verbindung der Formel

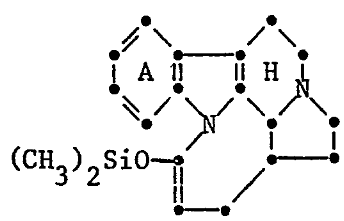

(VIIIj)

umwandelt, aus einer solchen Verbindung mittels eines geeigneten Oxidationsmittels, etwa eines Peroxids, wie m-Chlorperbenzoesäure ein entsprechendes Epoxid herstellt und hieraus in Gegenwart sauer reagierender Mittel, z.B. Triäthylammoniumfluorid, eine Verbindung der Formel VIIIb herstellt, worin anstelle der Gruppe $X_6$ als Wasserstoff eine Hydroxygruppe steht.

Eine Modifikation der Umwandlung der Gruppe $X_6$ als Wasserstoff in entsprechendes Hydroxy besteht darin, dass man das vorhin beschriebene Umsetzungsprodukt einer Verbindung der Formel VIIIb mit Lithiumdiisopropylamid mittels des Komplexes $MO_5$•Pyridin•Hexamethylphosphorsäuretriamid in Anlehnung an die Methode von Vedejs et al.; J. Org. Chem. 43, 188 (1978) oxidiert.

Die so gebildete Hydroxygruppe kann dann z.B. mit einer der Bedeutung von $X_6$ entsprechenden Halogenwasserstoffsäure oder einer Sulfonsäure, etwa Methansulfonsäure, oder einem reaktionsfähigen Derivat davon, z.B. dem Halogenid, in eine entsprechend veresterte Hydroxygruppe $X_6$ umgewandelt werden. Die Umwandlung der Hydroxygruppe in Halogen kann auch durch Einwirkung eines halogenierend wirkenden Mittels, etwa Phosphoroxychlorid, auf übliche Weise, z.B. in einem geeigneten Lösungsmittel, wie Chlorbenzol, erfolgen. Die vorstehend beschriebenen Oxidations- und Veresterungs- bzw. Halonenierungsmethoden erfolgen in üblicher Weise und zweckmässigerweise in solchen Verbindungen der Formel IIIb, worin im carbocyclischen Ring A stehende Hydroxy- und/oder Aminogruppen in geeigneter Weise geschützt sind, wobei die Abspaltung solcher Schutzgruppen, soweit zweckmässig und erwünscht, im Verlaufe der mit Verbindungen der Formel VIIIb durchzuführenden beschriebenen Umsetzungen erfolgen kann.

Die neuen Verbindungen der Formel I können ebenfalls erhalten werden, indem man in einer Verbindung der Formel

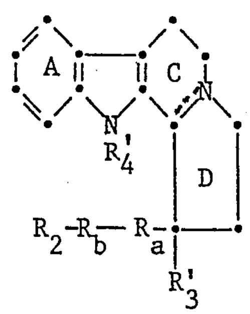

(IX),

worin $R_3'$ eine mittels Reduktion in eine Niederalkylgruppe $R_3$ überführbare Gruppe und $R_4'$ Wasserstoff oder Niederalkenyl darstellt, gegebenenfalls den Ringen C und D eine Doppelbindung gemeinsam ist, und in diesem Falle das dazugehörende N-Atom eine positive Ladung trägt und eine solche Verbindung als Salz vorliegt, und Hydroxy- und/oder Aminogruppen gegebenenfalls durch mittels Reduktion abspaltbare und durch Wasserstoff ersetzbare Gruppen geschützt sind, die Gruppe $R_3'$ zur Niederalkylgruppe $R_3$, eine gegebenenfalls vorhandene Niederalkenylgruppe $R_4'$ zur Niederalkylgruppe, eine gegebenenfalls vorhandene den Ringen C und D gemeinsame Doppelbindung zur Kohlenstoff-Stickstoff-Einfachbindung reduziert, und gegebenenfalls an Hydroxy- und/oder Aminogruppen stehende, unter den Bedingungen des Reduktionsverfahrens abspaltbare und durch Wasserstoff ersetzbare Schutzgruppen abspaltet und durch Wasserstoff ersetzt, und, wenn erwünscht, die im Anschluss an das erste Verfahren beschriebenen Verfahrensschritte durchführt.

Eine Niederalkenylgruppe $R_4'$ hat bis zu 7, vorzugsweise bis zu 4 Kohlenstoffatome und stellt z.B. den Vinyl-, 2-Methylvinyl- oder Allylrest dar.

$R_3'$ ist z.B. ein ungesättigter aliphatischer Kohlenwasserstoffrest mit bis zu 7, vorzugsweise bis zu 4 Kohlenstoffatomen, enthält Doppel- oder Dreifachbindungen und stellt demnach beispielsweise den Vinyl-, 2-Methylvinyl-, Allyl-, 2-Methallyl-, 3,3-Dimethylallyl-, 1-Butenyl-, den Aethinyl- oder Propargylrest dar. $R_3'$ ist ferner der Rest eines gegebenenfalls, z.B. mit einer organischen Sulfonsäure veresterten primären Niederalkanols, z.B. eines p-Toluolsulfonyloxyniederalkans oder eines diesem entsprechenden Niederalkylcarboxaldehyds mit bis zu 7, vorzugsweise 4 Kohlenstoffatomen und stellt demnach beispielsweise 2-Hydroxyäthyl, 3-Hydroxypropyl, 2-(p-Toluolsulfonyloxy)-äthyl, oder den Rest des Acetaldehyds oder 3-Propionaldehyds dar.

Eine an Hydroxy- und/oder Aminogruppen gegebenenfalls stehende, unter den Bedingungen dieses Reduktionsverfahrens abspaltbare und durch Wasserstoff ersetzbare Schutzgruppe ist in erster Linie eine hydro-

genolytisch abspaltbare α-Arylniederalkylgruppe, wie eine gegebenenfalls substituierte 1-Polyphenylniederalkyl- oder 1-Phenylniederalkylgruppe, worin Substituenten, insbesondere des Phenylteils, z.B. Niederalkyl wie Methyl, oder Niederalkoxy wie Methoxy, oder Halogen, wie Chlor, oder Trifluormethyl sein können, und in erster Linie Benzyl. Ein hydrogenolytisch abspaltbarer Rest ist ferner ein entsprechender Acylrest, wie der Acylrest einer organischen Carbonsäure, z.B. Niederalkanoyl, wie Acetyl, oder Aroyl, wie Benzoyl, ferner der Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenyl-niederalkylgruppe, worin Substituenten, in erster Linie des Phenylteils, z.B. die oben gegebene Bedeutung haben, und in erster Linie Trityl darstellen. Ein Hydroxy- und/oder Aminogruppen zusammen substituierender zweiwertiger Rest ist in erster Linie eine hydrogenolytisch abspaltbare Gruppe, wie gegebenenfalls substituiertes 1-Phenyl-niederalkyliden, worin Substituenten, insbesondere des Phenylteils, z.B. Niederalkyl oder Niederalkoxy sein können, und insbesondere Benzyliden.

Die Reduktion eines ungesättigten Kohlenwasserstoffrestes $R_3'$ zum entsprechenden Niederalkylrest $R_3$ sowie einer gegebenenfalls vorhandenen Niederalkenylgruppe $R_4'$ zur Niederalkylgruppe und der gegebenenfalls vorhandenen, den Ringen C und D gemeinsamen Doppelbindung zur Kohlenstoff-Stickstoff-Einfachbindung und die Abspaltung gegebenenfalls an Hydroxy- und/oder Aminogruppen stehender Schutzgruppen kann in an sich bekannter Weise, z.B. durch Behandeln mit katalytisch aktiviertem Wasserstoff, wie Wasserstoff in Gegenwart eines geeigneten Hydrierkatalysators, z.B. eines Nickel-, Platin- oder Palladiumkatalysators, erfolgen, wobei vorhandene hydrogenolytisch abspaltbare Schutzgruppen zugleich abgespalten und durch Wasserstoff ersetzt werden; oder man arbeitet mit einem geeigneten Hydridreduktionsmittel, wie z.B. Diboran, oder einem Alkalimetallborhydrid, z.B. Natriumborhydrid.

Die Reduktion kann auch stufenweise erfolgen, indem man z.B. zuerst eine gegebenenfalls vorhandene, den Ringen C und D gemeinsame Doppelbindung zur Kohlenstoff-Stickstoff-Einfachbindung, z.B. mittels Lithium-tri-tert.-butoxyaluminiumhydrid reduziert, und anschliessend den ungesättigten Rest $R_3'$ reduktiv, z.B. wie angegeben, in einen Niederalkylrest $R_3$ umwandelt.

Bei der Anwendung von Hydridreduktionsmitteln können auch an Sauerstoff gebundene Acylreste von Carbonsäuren, wie z.B. der der Essigsäure, im gleichen Arbeitsgang abgespalten werden. Die Reduktion eines der Gruppe $R_3'$ entsprechenden Restes eines primären Niederalkanols, z.B. 2-Hydroxyäthyl, welches zweckmässigerweise in reaktionsfähiger veresterter Form, z.B. als 2-p-Toluolsulfonyloxy-äthyl vorliegt, kann in üblicher Weise mittels eines Dileichtmetallhydrids, etwa Lithiumaluminiumhydrid in einem geegneten Lösungsmittel, wie Tetrahydrofuran erfolgen. Die Reduktion eines dem Rest $R_3'$ entsprechenden Niederalkylcarboxaldehyds kann z.B. mittels Hydrazinhydrat in Gegenwart eines geeigneten Lösungsmittels, z.B. Diäthylenglycol, durchgeführt werden. Bei der Durchführung dieser, insbesondere der katalytischen, Reduktionen ist darauf zu achten, dass andere Gruppen nicht angegriffen werden, z.B. durch volumetrische Kontrolle des Wasserstoffverbrauchs und rechtzeitigen Abbruch der Reduktion, oder durch entsprechende Dosierung des Reduktionsmittels. Diese Reduktionen werden üblicherweise in Gegenwart eines Lösungsmittels oder Lösungsmittelgemisches durchgeführt, wobei geeignete Reaktionsteilnehmer gleichzeitig auch als solche funktionieren können, und, wenn notwendig, unter Kühlen oder Erwärmen, z.B. in einem offenen oder geschlossenen Gefäss und/oder in der Atmosphäre eines Inertgases.

Ausgangsstoffe der Formel IX können in üblicher Weise z.B. wie folgt erhalten werden:

Man setzt eine Tryptaminverbindung der Formel

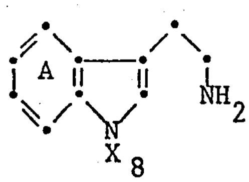

(IXa),

worin $X_8$ Wasserstoff, Niederalkenyl oder eine mittels Reduktion einschliesslich Hydrolyse oder Solvolyse, wie Hydrolyse abspaltbare und durch Wasserstoff ersetzbare Gruppe, z.B. eine α-Arylniederalkylgruppe, wie eine gegebenenfalls substituierte 1-Phenylniederalkylgruppe darstellt, worin Substituenten des Phenylteils z.B. Niederalkyl, wie Methyl oder Niederalkoxy, wie Methoxy, oder Halogen, wie Chlor, oder Trifluormethyl sein können, und insbesondere Benzyl ist, oder als mittels Solvolyse, wie Hydrolyse, abspaltbare und durch Wasserstoff ersetzbare Gruppe, z.B. einen Sulfonyl-, wie p-Toluolsulfonyl- oder Methansulfonylrest bedeutet, oder den Rest eines Halbesters der Kohlensäure, beispielsweise Niederalkoxycarbonyl, wie tert.-Butoxycarbonyl, darstellt, mit einer Verbindung der Formel

$$HO - CH_2 - CH_2 - \underset{\substack{|\\ R'_3}}{CH} - COOH \qquad (IXb)$$

oder einem reaktionsfähigen Derivat davon, beispielsweise dem aus einem in 2-Stellung durch einen Rest $R'_3$, z.B. den Vinyl- oder Allylrest, substituierten γ-Butyrolacton der Formel

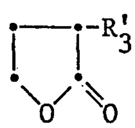

(IXc)

und Thionylchlorid in Gegenwart eines Kondensationsmittels, etwa wasserfreiem Zinkchlorid, erhältlichen entsprechenden 4-Chlorbutyrylchlorid der Formel

$$Cl - CH_2 - CH_2 - \underset{\substack{|\\ R'_3}}{CH} - COCl \qquad (IXd)$$

zu einer Verbindung der Formel

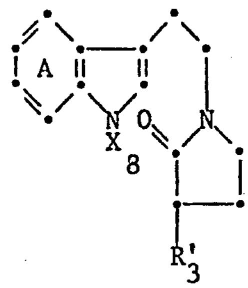

(IXe)

um. Hieraus stellt man mittels ringschliessender Kondensation, zweckmässigerweise in Gegenwart eines geeigneten Kondensationsmittels, etwa eines solchen sauren Charakters, wie Phosphoroxychlorid, eine Verbindung der Formel

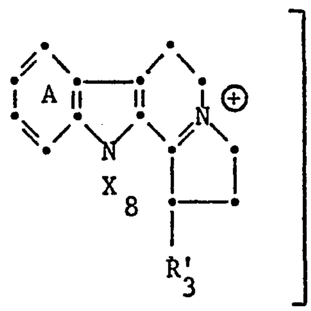

$M^{\ominus}$ (IXf)

worin M das Anion einer Säure, z.B. das der Perchlorsäure bedeutet, her, erhält hieraus durch Behandeln mit einer Base etwa einem Alkalihydroxid, z.B. Natriumhydroxid, eine Verbindung der Formel

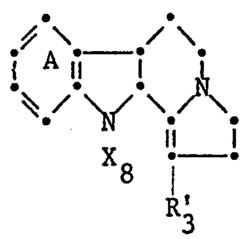

(IXg)

und setzt eine solche Verbindung mit einer Verbindung der oben erläuterten Formel

$$R_2 - \underset{\displaystyle R_1}{\underset{|}{C}} = CH_2 \qquad \text{(IIIi)}$$

um, und erhält nach anschliessender Salzbildung, z.B. mit Perchlorsäure, eine Verbindung der Formel IX, worin $R_a$ die Gruppe $-CH_2-$ bedeutet, und $R_1$ und $R_2$ die angegebenen Bedeutungen haben, den Ringen C und D eine Doppelbindung gemeinsam ist und das dazugehörende N-Atom eine positive Ladung trägt.

Zur Herstellung von Ausgangsstoffen der Formel IX, worin m in der Gruppe $R_a$ Null ist, kann man eine Verbindung der oben erläuterten Formel IXg mit einer Verbindung der Formel

$$R_2 - CH_2OH \qquad \text{(IXh)}$$

umsetzen.

Die Hydroxygruppe in einer Verbindung der Formel IXh liegt zweckmässigerweise in reaktionsfähiger, veresterter Form, z.B. als Ester mit einer starken organischen Sulfonsäure, wie einer aliphatischen oder aromatischen Sulfonsäure, oder einer Halogenwasserstoffsäure, vor und bedeutet demnach z.B. die Methansulfonyloxy- oder die p-Tosyloxygruppe, ferner Halogen, insbesondere Brom und speziell Jod. Eine Gruppe $R_2$ ist insbesondere eine veresterte Carboxygruppe der angegebenen Art, z.B. eine mit einem Niederalkanol, z.B. Methanol, veresterte Carboxygruppe.

Die Umsetzung wird in üblicher Weise in einem geeigneten Lösungsmittel, z.B. einem inerten und gegebenenfalls aprotischen Lösungsmittel, z.B. Dimethylsulfoxid, gegebenenfalls mit einem weiteren Lösungsmittel inerten Charakters, z.B. einem aromatischen Lösungsmittel wie Toluol bei erhöhter Temperatur, etwa in einem Bereich von +30 - +180°, vorzugsweise etwa +70 - +150° und zweckmässigerweise in einer Inertgasatmosphäre, etwa unter Stickstoff, vorgenommen.

Eine Variante zur Herstellung von Ausgangsstoffen der Formel IX besteht darin, dass man die entsprechend den Formeln IXa-IXg beschriebene Reaktionsfolge mit solchen Verbindungen durchführt, in denen anstelle der Gruppe $R_3'$ ein Wasserstoffatom steht, und eine auf diese Weise schliesslich erhaltene Verbindung der Formel

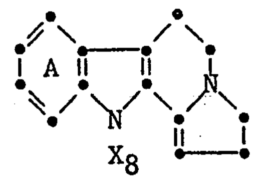

(IXi)

mit einer Verbindung der Formel IIIi bzw. der Formel IXh und anschliessender Salzbildung, z.B. mit Perchlorsäure, zu einer Verbindung der Formel

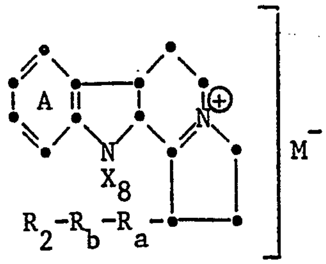

(IXj)

umsetzt, worin $R_a$ die Gruppe $-CH_2-$ bedeutet, bzw. m in der Gruppe $R_a$ Null ist. Durch Behandeln einer solchen Verbindung mit einer Base, etwa einem Alkalihydroxid wie Natriumhydroxid, wird eine Verbindung der Formel

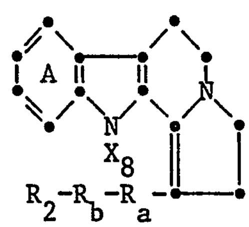

(IXk)

erhalten, in deren 1-Stellung anschliessend die Gruppe $R_3'$ eingeführt wird, z.B. durch Umsetzung mit einem die Einführung der Gruppe $R_3'$ erlaubenden reaktionsfähigen Derivat, etwa mit einer Verbindung der Formel

$$R_3' - OH \qquad (IXl),$$

worin die Hydroxygruppe zweckmässigerweise in reaktionsfähiger Form, etwa wie für Verbindungen der Formel IXh beschrieben, vorliegt, und demnach z.B. Halogen, etwa Brom, bedeuten kann. Nach anschliessender Salzbildung, z.B. mit Perchlorsäure, erhält man eine Verbindung der Formel IX, worin die Ringe C und D eine gemeinsame Doppelbindung haben. Diese Umsetzungen werden in üblicher Weise, etwa in Gegenwart

eines geeigneten Lösungsmittels und gegebenenfalls bei erhöhter Temperatur im offenen oder geschlossenen Gefäss und in einer Schutzgasatmosphäre, etwa Stickstoff, durchgeführt.

Nach Abspaltung einer vorhandenen abspaltbaren und durch Wasserstoff ersetzbaren Gruppe $X_g$, die, sofern es sich um eine reduktiv, wie hydrogenolytisch abspaltbare Gruppe, z.B. wie beschrieben, handelt, auch im Zuge der nachfolgenden Reduktion abgespalten werden kann, erhält man eine Verbindung der Formel IX, worin den Ringen C und D eine Doppelbindung gemeinsam ist. Diese kann z.B. mittels Lithium-tri-tert.-butoxyaluminiumhydrid zur Kohlenstoff-Stickstoff-Einfachbindung entsprechend einer Verbindung der Formel IX reduziert werden.

Die neuen Verbindungen der Formel I, worin die Gruppe $-(R_b-R_a)-$ für die Gruppe -CH=CH- steht, können ebenfalls erhalten werden, indem man in einer Verbindung der Formel

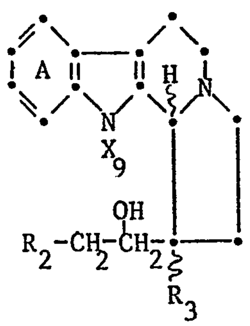

(X),

worin $X_9$ Wasserstoff, Niederalkyl oder eine unter den Bedingungen des Verfahrens abspaltbare und durch Wasserstoff ersetzbare Gruppe ist, die Gruppe der Formel $-CH_2-\overset{OH}{CH}-$ mittels Wasserabspaltung in die Gruppe -CH=CH- umwandelt, eine gegebenenfalls vorhandene unter den Bedingungen des Verfahrens abspaltbare und durch Wasserstoff ersetzbare Gruppe $X_9$ abspaltet und durch Wasserstoff ersetzt, gegebenenfalls vorhandene Hydroxy- und/oder Aminoschutzgruppen abspaltet und durch Wasserstoff ersetzt, und, wenn erwünscht, die im Anschluss an das erste Verfahren beschriebenen Verfahrensschritte durchführt.

Eine unter den Bedingungen des Verfahrens abspaltbare und durch Wasserstoff ersetzbare Gruppe $X_9$ ist eine mittels Solvolyse, wie Hydrolyse, Alkoholyse oder Acidolyse abspaltbare Gruppe, die sich z.B. von einer organischen Sulfonsäure, z.B. einer Arylsulfonsäure ableitet, und beispielsweise p-Toluolsulfonyl ist, oder den Rest eines Halbesters der Kohlensäure bedeutet und dementsprechend z.B. Niederalkoxycarbonyl, etwa tert.-Butoxycarbonyl darstellt.

Hydroxy- und/oder Aminoschutzgruppen sind solche, die unter den Bedingungen des Verfahrens mittels Hydrolyse, Alkoholyse oder Acidolyse, abgespalten und durch Wasserstoff ersetzt werden. Dementsprechend stellt eine Schutzgruppe dieser Art einen solvolytisch, hydrolytisch oder acidolytisch abspaltbaren Rest, insbesondere einen entsprechenden Acylrest, wie den Acylrest einer organischen Carbonsäure, z.B. Niederalkanoyl, wie Acetyl, oder Aroyl, wie Benzoyl,

ferner den Acylrest eines Halbesters der Kohlensäure, wie Niederalkoxycarbonyl, z.B. Methoxycarbonyl, Aethoxycarbonyl oder tert.-Butyloxy--arbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenyl-niederalkylgruppe, worin Substituenten, in erster Linie des Phenylteils, z.B. die oben gegebene Bedeutung haben, und in erster Linie Trityl, dar.

Ein Hydroxy- und/oder Aminogruppen zusammen substituierender zweiwertiger Rest ist eine solvolytisch, insbesondere hydrolytisch spaltbare Gruppe, wie Niederalkyliden, z.B. Methylen oder Isopropyliden, oder 1-Phenyl-niederalkyliden, dessen Phenylteil gegebenenfalls durch Niederalkyl, wie Methyl, oder Niederalkoxy, wie Methoxy, substituiert ist, insbesondere Benzyliden, oder Cycloalkyliden, z.B. Cyclopentyliden oder Cyclohexyliden.

Die erfindungsgemässe Wasserabspaltung erfolgt in üblicher Weise in Gegenwart saurer Stoffe gegebenenfalls in Gegenwart eines Lösungsmittels und bei erhöhter Temperatur im offenen und geschlossenen Gefäss, gewünschtenfalls in einer Schutzgasatmosphäre, etwa unter Stickstoff.

Saure Stoffe sind z.B. Säuren, wie etwa Mineralsäuren oder Carbonsäuren oder deren sauer reagierende funktionelle Derivate oder saure Salze davon, beispielsweise Schwefelsäure, Kaliumbisulfat, Thionylchlorid, gegebenenfalls in Gegenwart einer Stickstoffbase, wie Pyridin, ferner Phosphorsäure, Phosphoroxychlorid, Ameisensäure, Essigsäure oder deren Halogenide, wie Acetylchlorid, oder dessen Anhydrid, ferner Sulfonsäuren wie z.B. Methansulfonsäure oder

p-Toluolsulfonsäure.

Hydroxy- und/oder Aminoschutzgruppen der genannten Art werden unter den Bedingungen der Wasserabspaltung oder beim nachfolgenden Aufarbeiten abgespalten und durch Wasserstoff ersetzt. Diese Reaktionen werden in an sich bekannter Weise durchgeführt.

Ausgangsstoffe der Formel X sind auf übliche Weise erhältlich und können z.B. auf folgende Weise hergestellt werden:

Man setzt eine Verbindung der Formel

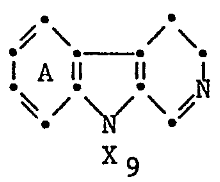

(Xa)

mit einem Aldehyd der Formel

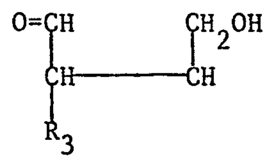

(Xb)

oder einem reaktionsfähigen Derivat davon, etwa einem solchen,worin die Aldehydgruppe in silylierter Form voliegt und demnach z.B. der Gruppe

$$(R_5)_3Si-O-(CH)$$ (Xc)

entspricht, worin $R_5$ Niederalkyl, z.B. Methyl ist, und die Hydroxygruppe vorzugsweise in reaktionsfähiger, veresterter Form, z.B. als Ester mit einer anorganischen Säure, etwa einer Halogenwasserstoffsäure, oder einer organischen Säure, z.B. p-Toluolsulfonsäure vorhanden ist, und demnach z.B. als Brom oder als p-Toluolsulfonyloxy vorliegt, in Gegenwart eines basischen Mittels, etwa eines Amins, wie

z.B. Aethyl-diisopropylamin, in einem geeigneten Lösungsmittel, etwa einem solchen stark polaren und aprotischen Charakters, etwa Dimethylformamid, zu einer Verbindung der Formel

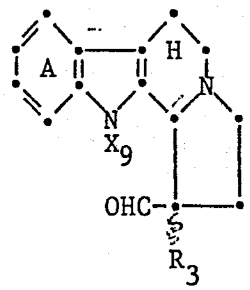

(Xd)

um, und stellt aus einer so erhaltenen Verbindung durch Umsetzung mit einer Verbindung der Formel

$$R_2 - CH_2 - Hal \qquad (Xe),$$

worin Hal für Halogen, insbesondere Brom, steht, unter den Bedingungen der Reformatsky-Reaktion eine Verbindung der Formel X her. Diese Umsetzungen werden in üblicher Weise vorgenommen.

Die neuen Verbindungen der Formel I, worin $R_2$ die Cyanogruppe und $R_1$ in der Gruppe $R_b$ Hydroxy bedeutet, können ebenfalls erhalten werden, indem man an eine Verbindung der Formel

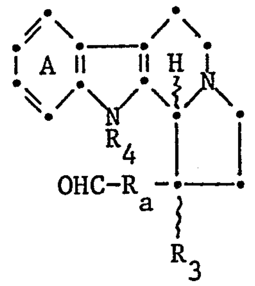

(XI)

Cyanwasserstoff oder eine in gleicher Weise reagierende, die zu bildende sekundäre Hydroxygruppe $R_1$ gegebenenfalls in geschützter Form liefernde Cyanverbindung, anlagert und die gebildete, gegebenenfalls in geschützter Form vorliegende sekundäre Hydroxygruppe in die freie Hydroxygruppe $R_1$ umwandelt, und, wenn erwünscht, die im Anschluss an das erste Verfahren beschriebenen Verfahrensschritte durchführt.Diese Umsetzung wird in an sich bekannter Weise, gegebenenfalls unter Kühlen oder Erwärmen im offenen oder geschlossenen Gefäss, z.B. unter Druck und/oder in einer Inertgasatmosphäre, etwa unter Stickstoff, vorgenommen.

Eine in gleicher Weise wie Cyanwasserstoff reagierende, die zu bildende sekundäre Hydroxygruppe $R_1$ gegebenenfalls in geschützter Form liefernde Verbindung ist z.B. eine geeignete Silylcyanid-Verbindung, etwa Trimethylsilylcyanid, welches sich an einen Ausgangsstoff der Formel XI in üblicher Weise, z.B. in einem inerten Lösungsmittel, etwa einem solchen aromatischen oder ätherartigen Charakters, wie Toluol oder Tetrahydrofuran bei erhöhter oder erniedrigter Temperatur unter Bildung einer durch die Trimethylsilylgruppe geschützten sekundären Hydroxygruppe anlagert, worin die Schutzgruppe mit sauren Mitteln, z.B. Chlorwasserstoff, abgespalten und unter Bildung eines entsprechenden Endstoffes der Formel I, worin $R_1$ Hydroxy ist, durch Wasserstoff ersetzt wird. Im Verlaufe dieser Umsetzung kann die gebildete Hydroxygruppe $R_1$ mit einem an der benachbarten Methylengruppe stehenden Wasserstoffatom unter Bildung von Wasser abgespalten werden, wobei eine Verbindung der Formel I gebildet wird, worin die Gruppe $-(R_b-R_a)-$ für die Gruppe $-CH=CH-$ steht.

Ausgangsstoffe der Formel XI wiederum, worin $R_a$ die Gruppe $-(CH_2)_m$ darstellt, sind durch Umsetzung von Verbindungen der Formel

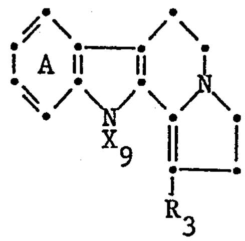

(XIa),

worin $X_9$ obige Bedeutung hat und insbesondere eine von Wasserstoff verschiedene Bedeutung hat, mit einer Verbindung der oben erläuterten Formel IXh und anschliessender Salzbildung, z.B. mit Perchlorsäure, zu einer Verbindung der Formel

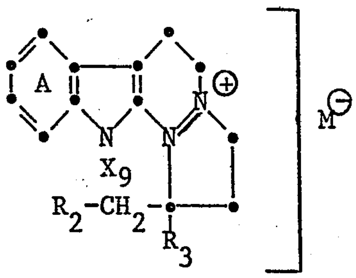

(XIb),

und deren anschliessende Reduktion, z.B. mittels Lithiumaluminiumhydrid in Tetrahydrofuran, zu einer Verbindung der Formel

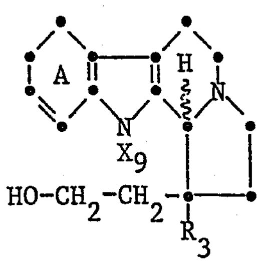

(IXc)

erhältlich, aus welcher durch Oxidation mittels eines geeigneten Oxidationsmittels, z.B. Pyridinium-chlorchromat, gegebenenfalls nach Abspaltung von an Hydroxy- und/oder Aminogruppen stehenden Schutzgruppen sowie einer gegebenenfalls vorhandenen Schutzgruppe $X_9$, Aldehyde der Formel XI hergestellt werden können. Diese Umsetzungen werden in üblicher Weise vorgenommen.

Ausgangsstoffe der Formel XI, worin $R_a$ die Gruppe $-(CH_2)_m-$ darstellt, deren Wert m für Null steht, stellen Analoge der oben erläuterten Ausgangsstoffe der Formel V dar, deren Herstellung, ausgehend von Verbindungen der Formel Vf, wie angegeben beschrieben ist.

Bei der Auswahl der geeigneten obigen Verfahren zur Herstellung von Verbindungen der Formel I muss darauf geachtet werden, dass vorhandene Substituenten nicht umgewandelt oder abgespalten werden, falls solche

Umwandlungen bzw. Abspaltungen nicht erwünscht sind, So können insbesondere veresterte oder amidierte Carboxylgruppen während Solvolysen, insbesondere Hydrolysen, ferner auch bei Reduktionen an der Reaktion beteiligt sein und umgewandelt werden. Andererseits können gleichzeitige Umwandlungen von Substituenten erwünscht sein; z.B. kann die Gruppe -CH=CH- unter den Bedingungen eines erfindungsgemäss eingesetzten Reduktionsverfahrens zur Gruppe $-CH_2-CH_2$ reduziert werden.

In erhaltenen Verbindungen kann man im Rahmen der Definition der Verbindungen der Formel I in üblicher Weise verfahrensgemäss erhaltene Verbindungen in andere Endstoffe überführen, z.B. indem man geeignete Substituenten abwandelt, einführt oder abspaltet.

Ferner kann man in erhaltenen Verbindungen mit im carbocyclischen Ring A aromatisch gebundenem Halogen, z.B. durch Behandeln mit Wasserstoff in Gegenwart eines üblichen Hydrierkatalysators, wie Raney-Nickel oder Palladium auf Kohle, solches Halogen abspalten und durch Wasserstoff ersetzen.

In erhaltenen Verbindungen kann man andererseits in den carbocyclischen Ring A Halogenatome einführen. Dieses kann in üblicher Weise geschehen, insbesondere bei nicht erhöhter Temperatur bzw. unter Kühlung und in Gegenwart eines Katalysators wie Eisen, Jod, Eisen-IIIchlorid, Aluminiumchlorid bzw. der entsprechenden Bromide.

In erhaltenen Verbindungen, worin $R_4$ Wasserstoff ist, kann man durch Umsetzung mit einem Niederalkanol, dessen Hydroxygruppe vorzugsweise in reaktionsfähiger, veresterter Form vorliegt, z.B. als Ester mit einer organischen Sulfonsäure, oder mit einer Halogenwasserstoffsäure, und die somit z.B. als Methansulfonyloxy- oder p-Toluolsulfonyloxygruppe, oder als Chlor, Brom oder insbesondere Jod vorliegt , in üblicher Weise, z.B. in Gegenwart eines basischen Kondensationsmittels, wie eines Alkalicarbonats, -bicarbonats oder -hydroxids, wie Natriumcarbonat oder -bicarbonat, oder Natriumhydroxid, eine Niederalkylgrup-

pe $R_4$ einführen.

Eine freie Carboxylgruppe lässt sich in üblicher Weise verestern, beispielsweise durch Umsetzen mit einem entsprechenden Alkohol, vorteilhaft in Gegenwart einer Säure, wie einer Mineralsäure, z.B. Schwefelsäure oder Chlorwasserstoffsäure, oder in Gegenwart eines wasserbindenden Mittels, wie Dicyclohexylcarbodiimid, oder durch Umsetzen mit einer entsprechenden Diazoverbindung, z.B. Diazomethan. Die Veresterung kann auch durch Umsetzen eines Salzes, vorzugsweise eines Alkalimetallsalzes der Säure mit einem reaktionsfähigen veresterten Alkohol, z.B. einem entsprechenden Halogenid, wie Chlorid, durchgeführt werden.

Eine freie Carboxylgruppe lässt sich in üblicher Weise amidieren, beispielsweise durch Umsetzen mit Ammoniak, oder mit einem primären oder sekundären Amin, vorteilhaft in Gegenwart eines wasserbindenden Mittels, wie Dicyclohexylcarbodiimid, oder durch Ueberführen der Carboxylgruppe in eine Halogencarbonyl-, z.B. Chlorcarbonylgruppe, und anschliessendem Umsetzen mit Ammoniak oder mit einem primären oder sedundären Amin.

In Verbindungen, die eine veresterte Carboxylgruppe enthalten, kann diese in üblicher Weise, z.B. durch Hydrolyse, vorzugsweise in Gegenwart von starken Basen, wie einem Alkalimetallhydroxyd, z.B. Natrium- oder Kaliumhydroxyd, oder starken Säuren, z.B. einer starken Mineralsäure, wie einer Halogenwasserstoffsäure, z.B. Chlorwasserstoffsäure, oder Schwefelsäure, in eine freie Carboxylgruppe übergeführt werden.

In Verbindungen mit einer veresterten Carboxylgruppe als Substituenten kann diese in üblicher Weise, z.B. durch Ammonolyse oder Aminolyse mit Ammoniak oder einem primären oder sekundären Amin in die entsprechende Carbamoylgruppe übergeführt werden.

In Verbindungen mit einer veresterten Carboxylgruppe kann man diese durch Umesterung in andere veresterte Carboxylgruppen umwandeln, z.B. durch Umsetzung mit einem Alkohol, dessen Siedepunkt deutlich über dem des in der umzuwandelnden veresterten Carboxylgruppe vorliegenden Alkohols liegt, und führt die Reaktion z.B. in einem Ueberschuss des eingesetzten Alkohols und/oder einem inerten organischen, vorzugsweise ebenfalls deutlich über dem Alkohol der umzuwandelnden veresterten Carboxylgruppe siedenden Lösungsmittel, vorzugsweise in Gegenwart eines Katalysators, z.B. eines Alkalimetall-niederalkanolats, bei erhöhter Temperatur und üblicherweise unter Abdestillieren des freigesetzten Alkohols durch.

In Verbindungen mit einer Carbamoylgruppe kann diese in eine Nitrilgruppe umgewandelt werden, z.B. mittels geeigneter wasserabspaltender Mittel, etwa N,N'-Dicyclohexylcarbodiimid.

In Verbindungen mit einer veresterten Carboxylgruppe kann diese in üblicher Weise durch Reduktion, z.B. mittels eines Alkalimetalls in einem Niederalkanol, etwa Natrium in Aethanol, in eine Hydroxymethylgruppe umgewandelt werden.

In Verbindungen mit einer Hydroxygruppe kann diese in üblicher Weise in eine Acyloxygruppe umgewandelt werden, z.B. durch Umsetzung mit einer dem Acylrest entsprechenden Carbonsäure oder Sulfonsäure, oder einem reaktionsfähigen Derivat davon, z.B. einem Säurehalogenid, oder einem Ester, gegebenenfalls in Anwesenheit eines säurebindenden oder Kondensationsmittels.

In Verbindungen, die eine Acyloxygruppe enthalten, kann diese in üblicher Weise, z.B. durch Hydrolyse, vorzugsweise in Gegenwart einer starken Base, wie einem Alkalimetallhydroxid, etwa Natriumhydroxid, oder einer starken Säure, etwa einer Mineralsäure, wie einer Halogenwasserstoffsäure oder Schwefelsäure, in die freie Hydroxygruppe umgewandelt werden.

In Verbindungen mit einer Aminogruppe kann diese in üblicher Weise in eine Acylaminogruppe umgewandelt werden, z.B. durch Umsetzung mit einer dem Acylrest entsprechenden Carbonsäure oder einem reaktionsfähigen Derivat davon, z.B. einem Säurehalogenid, oder einem Ester gegebenenfalls in Anwesenheit eines säurebindenden oder Kondensationsmittels.

In Verbindungen, die eine Acylaminogruppe enthalten, kann diese in üblicher Weise, z.B. durch Hydrolyse, vorzugsweise in Gegenwart einer starken Base, wie einem Alkalimetallhydroxid, etwa Natriumhydroxid, oder einer starken Säure, etwa einer Mineralsäure, wie einer Halogenwasserstoffsäure oder Schwefelsäure, in die freie Aminogruppe umgewandelt werden.

In Verbindungen mit einer Cyanogruppe kann diese mittels Hydrolyse, etwa mittels Wasser im sauren oder basischen Medium, z.B. in Gegenwart einer Mineralsäure, wie Schwefelsäure, oder eines Alkali- oder Erdalkalihydroxids, wie Natrium- oder Calciumhydroxid, in eine Carboxygruppe umgewandelt werden.

In Verbindungen mit einer Cyanogruppe kann diese mittels partieller Hydrolyse, z.B. mittels Wasser im sauren oder basischen Medium, etwa mittels eines Hydroxids oder Bicarbonats eines Alkalimetalls, gegebenenfalls in Gegenwart eines Peroxids, wie Wasserstoffperoxid, in die Carbamoylgruppe umgewandelt werden.

In Verbindungen mit einer Cyanogruppe kann diese durch Umsetzung mit einem Alkohol, etwa einem Niederalkanol, im sauren Medium, etwa Chlorwasserstoff, in den entsprechenden Iminoäther umgewandelt und hieraus mittels hydrolytischer Abspaltung der Iminogruppe in eine veresterte Carboxygruppe umgewandelt werden.

In Verbindungen mit einer Gruppe $R_a$, worin m 1 ist und einer Gruppe $R_b$, worin $R_1$ Hydroxy ist, kann durch Wasserabspaltung in üblicher Weise, z.B. durch Erwärmen in Gegenwart eines geeigneten Lösungsmittels, etwa eines solchen aromatischen Charakters, wie z.B. Toluol, vorzugsweise in Gegenwart eines die Wasserabspaltung beschleunigenden Mittels, z.B. eines sauren Katalysators, etwa p-Toluolsulfonsäure, eine Verbindung erhalten werden, worin anstelle der Gruppe $-(R_b-R_a)-$ die Gruppe -CH=CH- steht.

Nicht -N-oxidierte Verbindungen der Formel I können durch Einwirkung geeigneter Oxidationsmittel, z.B. einer Peroxyverbindung, z.B. einer Peroxycarbonsäure wie m-Chlor-perbenzoesäure, oder Peressigsäure, oder vom Wasserstoffperoxid, in die entsprechenden N-oxidierten Verbindungen umgewandelt werden.

N-oxidierte Verbindungen können mittels geeigneter Reduktionsmittel, z.B. aktiviertem Wasserstoff, wie Wasserstoff in Gegenwart eines Hydrierkatalysators, etwa eines Nickel-, Platin- oder Palladiumkatalysators, oder mittels schwefliger Säure in die nicht-N-oxidierten Verbindungen umgewandelt werden.

Nicht quaternisierte Verbindungen der Formel I können durch Umsetzung mit einer den genannten quartären Gruppen entsprechenden Verbindung, z.B. gegebenenfalls substituiertes Niederalkyl, wie Methyl, welches als reaktionsfähiges, verestertes Derivat des der betreffenden quartären Gruppe entsprechenden Alkohols, z.B. als Halogenid, wie Chlorid, Bromid oder Jodid oder als Ester mit Schwefelsäure oder einer organischen Sulfonsäure, z.B. einer aliphatischen oder aromatischen Sulfonsäure, wie Methansulfonsäure, Benzolsulfonsäure oder p-Toluolsulfonsäure, vorliegt, in Ab- oder Anwesenheit eines geeigneten Lösungsmittels, gegebenenfalls unter Kühlen oder Erwärmen im offenen oder geschlossenen Gefäss, in die entsprechenden quaternisierten Verbindungen umgewandelt werden.

Wie bei den Herstellungsverfahren muss auch bei der Durchführung der Zusatzschritte darauf geachtet werden, dass unerwünschte Nebenreaktionen, welche die Umwandlung zusätzlicher Gruppierungen zur Folge haben können, nicht eintreten.

Die oben beschriebenen Reaktionen können gegebenenfalls gleichzeitig oder nacheinander, ferner in beliebiger Reihenfolge durchgeführt werden. Falls notwendig, erfolgen sie in Anwesenheit von Verdünnungsmitteln, Kondensationsmitteln und/oder katalytisch wirkenden Mitteln, bei erniedrigter oder erhöhter Temperatur, im geschlossenen Gefäss unter Druck und/oder in einer Inertgasatmosphäre.

Epimerengemische als auch Gemische von cis- und trans-Isomeren lassen sich üblicherweise durch fraktioniertes Kristallisieren aus geeigneten Lösungsmitteln und/oder Chromatographie mittels geeigneter die chromatographische Trennung bewirkender Materialien, z.B. Kieselgel oder Stärke, unter Anwendung geeigneter Lösungsmittel oder deren Gemische in die reinen Epimeren bezw. reinen cis- oder trans-Isomeren trennen. Vorteilhafterweise isoliert man das wirksamere der Epimeren bzw. der cis- oder trans-Isomeren.

Erhaltene Racematgemische können auf Grund der physikalisch-chemischen Unterschiede der Diastereoisomeren in bekannter Weise, z.B. durch Chromatographie und/oder fraktionierte Kristallisation, in die beiden stereoisomeren (diastereomeren) Racemate aufgetrennt werden.

Erhaltene Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Substanz, insbesondere Säuren, und Trennen des auf diese Weise erhaltenen Salzgemisches, z.B. auf Grund von verschiedenen Löslichkeiten, in die diastereomeren Salze, aus denen die

freien Antipoden durch Einwirkung geeigneter Mittel freigesetzt werden können. Besonders gebräuchliche, optisch aktive Säuren sind z.B. die D- und L-Formen von Weinsäure, Di-O-O'-(p-Toluoyl)-weinsäure, Aepfelsäure, Mandelsäure, Camphersulfonsäure, Glutaminsäure, Asparaginsäure oder Chinasäure. Vorteilhafterweise isoliert man den wirksameren der beiden Antipoden.

Je nach den Verfahrensbedingungen und Ausgangsstoffen erhält man die neuen Verbindungen in freier Form oder in der ebenfalls von der Erfindung umfassten Form ihrer Salze, wobei die neuen Verbindungen oder Salze davon auch als Hemi-, Mono-, Sesqui- oder Polyhydrate davon vorliegen können. Säureadditionssalze der neuen Verbindungen können in an sich bekannter Weise, z.B. durch Behandeln mit basischen Mitteln, wie Alkalimetallhydroxiden, -carbonaten oder -hydrogencarbonaten oder Ionenaustauschern, in die freien Verbindungen übergeführt werden. Andererseits können erhaltene freie Basen mit organischen oder anorganischen Säuren, z.B. mit den genannten Säuren, Säureadditionssalze bilden, wobei zu deren Herstellung insbesondere solche Säuren verwendet werden, die sich zur Bildung von pharmazeutisch verwendbaren, nicht-toxischen Salzen eignen.

Diese oder andere Salze, insbesondere Säureadditionssalze der neuen Verbindungen, wie z.B. Oxalate oder Perchlorate, können auch zur Reinigung der erhaltenen freien Basen dienen, indem man die freien Basen in Salze überführt, diese abtrennt und reinigt, und aus den Salzen wiederum die Basen freisetzt.

Die neuen Verbindungen können je nach der Wahl der Ausgangsstoffe und Arbeitsweisen als Epimerengemische, reine Epimeren, Racematgemische, Racemate, optische Antipoden, Gemische von cis- oder trans-Isomeren, oder als reine cis- oder trans-Isomere vorliegen. Die Ausgangsstoffe können auch als reine Epimere, reine cis- oder trans-Isomere oder als bestimmte optische Antipoden, oder sofern es sich um Oxime handelt, auch als solche in der syn- oder anti-Form eingesetzt werden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Die Ausgangsstoffe sind bekannt oder können, falls sie neu sind, nach an sich bekannten Methoden, z.B. wie oben beschrieben und analog wie in den Beispielen illustriert, erhalten werden. Neue Ausgangsstoffe, insbesondere solche der Formeln II, III, IV, V, VI, VII, VIII, IX, X und XI, sind zur Ausführung der vorstehend beschriebenen Verfahren besonders entwickelt worden und stellen ebenso wie Verfahren zur deren Herstellung ebenfalls einen Gegenstand der Erfindung dar. Die Erfindung betrifft auch verfahrensgemäss erhältliche neue Zwischenprodukte sowie Verfahren zu deren Herstellung.

Die Erfindung betrifft ebenfalls die Verwendung der Verbindungen der Formel I oder von pharmazeutisch verwendbaren, nicht-toxischen Säureadditionssalzen solcher Verbindungen, insbesondere als pharmakologisch, in erster Linie auf das Zentralnervensystem wirkende, verwendbare Verbindungen. Dabei kann man sie, vorzugsweise in Form von pharmazeutischen Präparaten, in einem Verfahren zur prophylaktischen und/oder therapeutischen Behandlung des tierischen oder menschlichen Körpers,

insbesondere zur Behandlung von Vigilanzstörungen und zerebraler Leistungsinsuffizienz, insbesondere von Gedächtnisstörungen verschiedener Genese und von Folgezuständen von Hirntraumen oder Apoplexie verwenden.

Die Erfindung betrifft weiterhin pharmazeutische Präparate, die Verbindungen der Formel I oder pharmazeutisch verwendbare, nicht-toxische Säureadditionssalze von solchen Verbindungen als Wirkstoffe enthalten, sowie Verfahren zu ihrer Herstellung.

Die neuen Verbindungen können z.B. in Form pharmazeutischer Präparate Verwendung finden, welche eine pharmakologisch wirksame Menge der Aktivsubstanz, gegebenenfalls zusammen mit pharmazeutisch verwendbaren Trägerstoffen enthalten, die sich zur enteralen, z.B. oralen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycerin und/oder Schmiermitteln, z.B. Kieselerde, Talk, Stearinsäure oder Salze davon, wie Magnesium oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die neuen pharmakologisch wirksamen Verbindungen in Form von parenteral verabreichbaren Präparaten oder von Infusionslösungen verwenden. Solche Lösungen sind vorzugsweise isotonische wässerige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-,

Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffes.

Die Dosierung des Wirkstoffs, der allein oder zusammen mit üblichen Träger- und Hilfsstoffen verabreicht wird, kann von verschiedenen Faktoren, wie Applikationsweise, Spezies, Alter und/oder individuellem Zustand abhängen. So liegt die tägliche in einer oder mehreren, vorzugsweise höchstens 4 Einzeldosen an Warmblüter von etwa 70 kg Körpergewicht zu verabreichende Gesamtdosis in einem Bereich von etwa 10 mg bis 500 mg, vorzugsweise von etwa 25 mg bis 250 mg.

Die folgenden Beispiele dienen zur Illustration der Erfindung; Temperaturen werden in Celsiusgraden angegeben.

<u>Beispiel 1</u>: In eine Lösung von 384 g (1 Mol) des nach Beispiel 2 erhaltenen Gemisches der stereoisomeren α-Acetoxy-1-äthyl-2,3,5,6,11,11b-hexahydro-1H-indolizino[8,7-b]indol-1-propionsäuremethylester in 10 Litern Methanol leitet man bei Rückflusstemperatur während 3 Stunden Chlorwasserstoffgas ein. Nach Eindampfen des Reaktionsgemisches löst man den Rückstand in 4 Litern Eis/Wassergemisch, neutralisiert mit Natriumbicarbonat, extrahiert dreimal mit je 2 Litern Methylenchlorid, wäscht die vereinigten organischen Phasen mit Wasser, trocknet über Natriumsulfat, und dampft das Filtrat im Vakuum ein. Der erhaltene Schaum wird in 3 Liter Methanol gelöst und mit 3 Liter Aether versetzt. Nach dem Abkühlen kristallisiert das Racemat des <u>threo</u>-<u>cis</u>-α-Hydroxy-1-äthyl-2,3,5,6,11,11b-hexahydro-1H-indolizino[8,7-b]indol-1-propionsäuremethylesters, dessen optische Antipoden der Formel

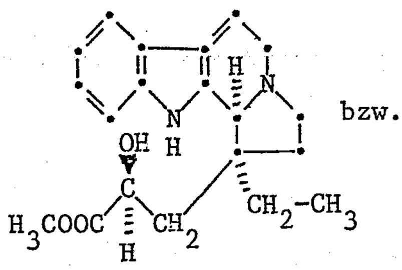 bzw. 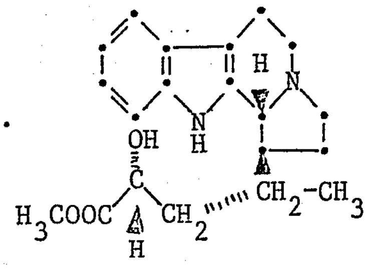

entsprechen, aus; Smp. 219-220°; des Hydrochlorids 230-233° (aus Methanol/Aether).

Die Mutterlauge wird im Vakuum auf 2 Liter eingeengt und mit methanolischer Salzsäurelösung auf pH 5 angesäuert, im Vakuum eingedampft und der Rückstand in 3 Liter Aceton gelöst. Beim Abkühlen kristallisiert das Hydrochlorid des Racemats der erythro-Form der obigen cis-Verbindung, dessen optische Antipoden der Formel

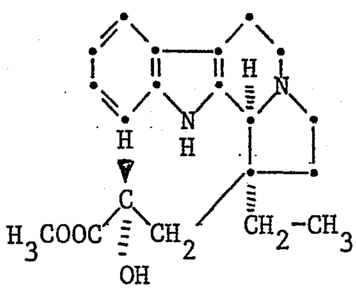 bzw. 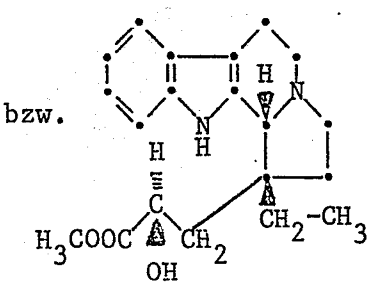

entsprechen, aus; Smp. 232-234°; Smp. der freien Base: 183-185° (aus Methylenchlorid/Aether).

Die hierbei erhaltene Mutterlauge wird eingedampft, der Rückstand in 1 Liter Eis/Wassergemisch gelöst, die Lösung mit 25% wässriger Ammoniaklösung alkalisch eingestellt, dreimal mit je 1,5 Litern Methylenchlorid extrahiert, die vereinigten organischen Phasen mit 1 Liter Wasser gewaschen, über Natriumsulfat getrocknet und das Filtrat im Vakuum eingedampft. Das hierbei erhaltene Gemisch der 3 stereoisomeren Formen der Verbindung der angegebenen Formel wird über 4 kg Kieselgel 60 (70-230 mesh, Merck) unter Verwendung eines Gemisches von Toluol/Essigsäureäthylester mit steigenden Mengen Essigsäureäthylester chromatographiert. Man isoliert auf diese Weise sowohl eine weitere

Menge des threo-Isomeren der cis-Verbindung als auch eine weitere Menge des erythro-Isomeren der cis-Verbindung vom Smp. 183-185°. Ausserdem wird ein Isomeres des trans-α-Hydroxy-1-äthyl-2,3,5,6,11,11b-hexahydro-1H-indolizino[8,7-b]indol-1-propionsäuremethylesters der Formel

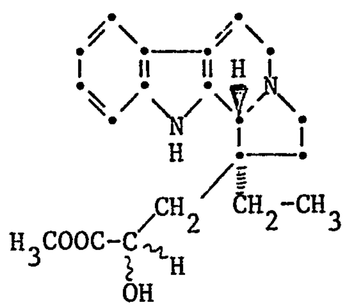

erhalten; Smp. 153-154°; Smp. des sauren Oxalats: 132-134° (aus Aceton); Smp. des sauren Sulfats als Hemihydrat 220-221° (aus Methanol/Aceton).

Eine Variante dieses Verfahrens wird wie folgt beschrieben:
Man verwendet als Ausgangsmaterial 384 g (1 Mol) des nach Beispiel 2 durch Reduktion mittels Natriumborhydrid erhaltenen Gemisches der stereoisomeren α-Acetoxy-1-äthyl-2,3,5,6,11,11b-tetrahydro-1H-indolizino[8,7-b]indol-1-propionsäuremethylester. Nach dem Aufarbeiten, wie beschrieben, und Abtrennen des erhaltenen threo-cis-α-Hydroxy-1-äthyl-2,3,5,6,11,11b-hexahydro-1H-indolizino[8,7-b]indol-1-propionsäuremethylesters erhält man eine Mutterlauge, die im Vakuum auf ein Volumen von 1 Liter eingeengt und unter Rühren mit einer Lösung von 75 g wasserfreier Oxalsäure in 250 ml Methanol versetzt wird. Nach dem Eindampfen im Vakuum löst man den Rückstand in 1,5 Liter Aceton, woraus beim Abkühlen das saure Oxalat des trans-α-Hydroxy-1-äthyl-2,3,5,6,11,11b-hexahydro-1H-indolizino[8,7-b]indol-1-propionsäuremethylesters der angegebenen Formel vom Smp. 132-134° auskristallisiert. Smp. der freien Base: 153-154° (aus Aether); Smp. des sauren Sulfats (als Hemihydrat): 220-221° (aus Methanol/Aceton).

Beispiel 2: Man rührt unter einer Stickstoffatmosphäre ein Gemisch von 300 g (1,25 Mol) 1-Aethyl-2,3,6,11-tetrahydro-5H-indolizino[8,7-b]-indol, 4 Litern Methylenchlorid, 460 g 2-Acetoxyacrylsäuremethylester (hergestellt nach: J. Wolinski, R. Novak: J.Org.Chem. 29, 3596 (1964)) und 50 ml Methanol bei Raumtemperatur. Nach 65 Stunden dampft man die Lösung unter Vakuum ein, digeriert den öligen Rückstand zur Entfernung überschüssigen 2-Acetoxyacrylsäuremethylesters dreimal mit je 500 ml n-Hexan, löst den Rückstand in 6 Litern Methanol und tropft unter Eiskühlung eine 70%-ige wässrige Perchlorsäurelösung bis zum Erreichen von pH 6,5 zu. Die erhaltene Lösung von α-Acetoxy-1-äthyl-1,2,3,5,6,11-hexahydro-indolizino[8,7-b]indol-4-ium-1-propionsäuremethylester-perchlorat wird in Gegenwart von 150 g Palladium-auf-Kohle-Katalysator (10%) während 24 Stunden bei normalem Druck hydriert, das Reaktionsgemisch filtriert, das Filtrat im Vakuum eingedampft, der Rückstand in 2 Litern Methylenchlorid gelöst und mit 2-n.Natronlauge bis pH 7,5 versetzt. Die organische Phase wird abgetrennt, über Natriumsulfat getrocknet, filtriert und im Vakuum eingedampft, wonach man ein Gemisch der stereoisomeren α-Acetoxy-1-äthyl-2,3,5,6,11,11b-hexahydro-1H-indolizino[8,7-b]indol-1-propionsäuremethylester der Formel

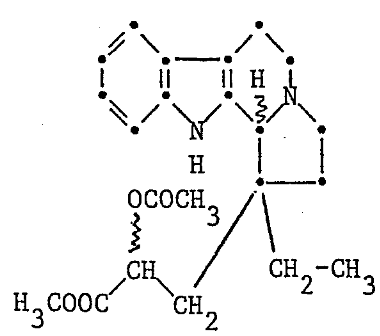

als gelbes Oel erhält.

Eine Variante dieses Verfahrens wird wie folgt beschrieben:

Das durch Umsetzung von 300 g (1,25 Mol) 1-Aethyl-2,3,6,11-tetrahydro-5H-indolizino[8,7-b]-indol und 460 g 2-Acetoxyacrylsäuremethylester und anschliessende dreimalige Behandlung mit 500 ml n-Hexan, wie oben

beschrieben, erhaltene rohe amorphe Reaktionsprodukt vom Smp. 76-80° kann auch wie folgt reduziert werden: Man löst das erhaltene Reaktionsprodukt in 10 Litern Methanol und versetzt die Lösung unter Rühren bei Raumtemperatur portionenweise mit 53 g Natriumborhydrid. Nach beendeter Zugabe rührt man noch weitere 30 Minuten, säuert die Lösung mit Eisessig auf pH 6 an und dampft im Vakuum völlig ein. Der Rückstand wird mit 5% wässriger Natriumbicarbonatlösung neutralisiert und viermal mit je 3 Litern Aether extrahiert. Die vereinigten organischen Phasen wäscht man mit Wasser, trocknet über Natriumsulfat und dampft das Filtrat im Vakuum ein, worauf man ein Gemisch des stereoisomeren α-Acetoxy-1-äthyl-2,3,5,6,11,11b-hexahydro-1H-indolizino[8,7-b]indol-1-propionsäuremethylesters der obigen Formel als gelben Schaum erhält.

Das Ausgangsmaterial kann auf folgende Weise hergestellt werden:

a) 1,14 kg (10 Mol) 2-Aethyl-γ-butyrolacton (hergestellt nach: R.F.Borne, H.Y.Aboul-Enein, I.W.Waters, J.Hicks: J.Med.Chem. 16, (3), 245, (1973) werden innerhalb von 30 Minuten bei 40°C in eine gut gerührte Lösung von 162 g wasserfreiem Zinkchlorid in 2,5 Litern Thionylchlorid eingetragen und das Gemisch während 18 Stunden unter Rückfluss gekocht. Nach Abdestillieren des Lösungsmittels unter Normaldruck wird der schwarze Rückstand über eine Vigreux-Kolonne bei Wasserstrahlvakuum destilliert, wonach man das 2-Aethyl-4-chlor-butyrylchlorid vom Kp. 105-110°/20 mm erhält.

b) 900 g einer 55%-igen Natriumhydridsuspension in Paraffinöl werden zwecks Entfernung des Paraffinöls mit 1,5 Litern n-Hexan gewaschen und der Rückstand in 4 Litern Tetrahydrofuran suspendiert. Unter Stickstoff und Kühlung auf 0 bis 5° tropft man eine Lösung von 800 g (5 Mol) Tryptamin in 4 Litern Tetrahydrofuran während 1 Stunde zu, kühlt die dunkelgrüne Suspension auf -10° und versetzt während 3 Stunden mit einer Lösung von 850 g 2-Aethyl-4-chlor-butyrylchlorid in 1 Liter Tetrahydrofuran. Nach weiterem Rühren des Reaktionsgemisches während 18 Stunden bei Raumtemperatur wird auf 0 bis 5° gekühlt, überschüssiges Natriumhydrid durch langsames Zutropfen von 2 Litern Wasser zersetzt und mit 15 Litern

Toluol verdünnt. Man extrahiert zweimal mit je 2 Litern 1-n.Salzsäure, dreimal mit je 20 Litern Wasser und einmal mit 4 Litern gesättigter wässriger Natriumchloridlösung, trocknet die organische Phase über Natriumsulfat und engt nach Filtration auf ein Volumen von 3 Litern ein. Nach Zufügen von 200 g Aktivkohle erhitzt man zum Sieden, filtriert über Celite® und wäscht den Filterrückstand mit 2 Litern heissem Toluol nach, wobei nach dem Abkühlen das 3-Aethyl-N-[2-(3'-indolyl)-äthyl]-2-pyrrolidon auskristallisiert; Smp. 98 -100°.

c) Ein Gemisch von 640 g (2,5 Mol) des erhaltenen 3-Aethyl-N-[2-(3'-indolyl)-äthyl]-2-pyrrolidon und 2 Litern Phosphoroxychlorid wird 4 Stunden unter Rückfluss gerührt, dann abgekühlt, auf 15 Liter Eis/Wassergemisch gegossen, mit weiteren 10 Litern Wasser verdünnt und auf 3° gekühlt. Unter Rühren werden während 30 Minuten 875 g Natriumperchlorat eingetragen und weitere 5 Stunden bei Raumtemperatur gerührt. Nach dem Abfiltrieren kristallisiert man den Filterrückstand aus Methanol um, wonach man das 1-Aethyl-1,2,3,5,6,11-hexahydro-indolizino[8,7-b]indol-4-ium-perchlorat der Formel

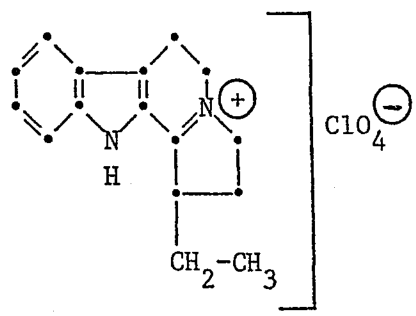

erhält; Smp. 168 - 171°.

d) In eine Lösung von 510 g (1,5 Mol) der erhaltenen Verbindung in 1,5 Litern Dimethylsulfoxid und 3 Litern Toluol werden unter Stickstoff bei guter Rührung 2 Liter 1-n.Natronlauge eingetragen. Nach Verdünnen mit 6 Litern Toluol wird die organische Phase abgetrennt, mit 1 Liter Wasser gewaschen, über Pottasche getrocknet, filtriert

und das Lösungsmittel unter Vakuum eindampft, wonach man das 1-Aethyl-2,3,6,11-tetrahydro-5H-indolizino[8,7-b]indol als orangerotes Oel erhält.

Beispiel 3: Eine Lösung von 320 g (0,75 Mol) 1-Aethyl-1,2,3,5,6,11-hexahydro-indolizino[8,7-b]indol-4-ium-1-propionsäuremethylester-perchlorat und 75 g wasserfreiem Kaliumacetat in 6 Litern Methanol wird in Gegenwart von 30 g Palladium-auf-Kohle-Katalysator (5%) während 3 Stunden bei normalem Druck hydriert. Das Reaktionsgemisch wird filtriert, das Filtrat im Vakuum eingedampft, der Rückstand in 4,5 Litern Methylenchlorid gelöst und mit 1,5 Litern 1-n.Natronlauge versetzt. Die organische Phase wird abgetrennt, 2 mal mit je 500 ml Wasser gewaschen, über Natriumsulfat getrocknet, filtriert und im Vakuum eingedampft. Der Rückstand wird in 1,5 Litern warmen Toluol gelöst, mit 1,5 Litern n-Hexan versetzt, worauf nach dem Abkühlen der cis-1-Aethyl-2,3,5,6,11,11b-hexahydro-1H-indolizino[8,7-b]indol-1-propionsäuremethylester der Formel

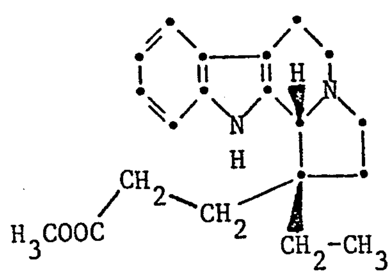

auskristallisiert; Smp. 162 - 163°.

Zur Ueberführung in das Methansulfonat löst man 32,6 g (0,1 Mol) der erhaltenen Verbindung in 250 ml Methanol und versetzt unter Rühren mit 9,6 g Methansulfonsäure. Nach Zusatz von 1,5 Litern Aether und Abkühlen kristallisiert das Methansulfonat der cis-Verbindung aus; Smp. 218-219°.

Die bei der Herstellung der Base erhaltene Mutterlauge wird im Vakuum völlig eingedampft, der Rückstand in 12 Litern n-Hexan suspendiert und während 30 Minuten bei Raumtemperatur gerührt. Nach dem Abfiltrieren wird die Lösung im Vakuum eingedampft, in 500 ml Aether

gelöst und mit 1 Liter n-Hexan versetzt. Nach dem Abkühlen kristallisiert trans-1-Aethyl-2,3,5,6,11,11b-hexahydro-1H-indolizino[8,7-b]indol-1-propionsäuremethylester aus; Smp. 118-121°.
Zur Ueberführung in das Methansulfonat löst man 32,6 g (0,1 Mol) dieser Verbindung in 1 Liter Aceton und versetzt die Lösung unter Rühren mit 9,6 g Methansulfonsäure. Nach dem Abkühlen kristallisiert das Methansulfonat der trans- Verbindung aus; Smp. 147-148°.

Das Ausgangsmaterial kann wie folgt erhalten werden:

a) Man rührt unter einer Stickstoffatmosphäre ein Gemisch von 300 g (1,25 Mol) des nach Beispiel 2d) erhaltenen 1-Aethyl-2,3,6,11-tetrahydro-5H-indolizino[8,7-b]indols und 300 g Acrylsäuremethylester in 4,5 Litern Methylenchlorid während 65 Stunden bei Raumtemperatur, dampft dann die Lösung im Vakuum ein, digeriert den roten öligen Rückstand zur Entfernung des überschüssigen Acrylsäuremethylesters 3 mal mit je 500 ml n-Hexan, worauf der 1-Aethyl-2,3,5,6-tetrahydro-1H-indolizino[8,7-b]indol-1-propionsäuremethylester der Formel

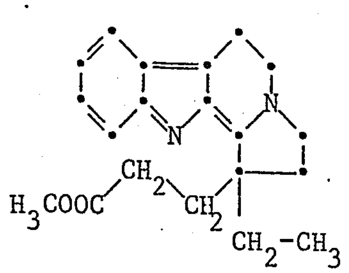

auskristallisiert;

Smp. 129-132°. Zur Ueberführung in das Perchlorat versetzt man bei 0 bis 5° eine Lösung von 324 g (1 Mol) dieser Verbindung in 5 Litern Methanol unter Rühren innerhalb von 30 Minuten mit 150 ml einer 70%igen wässrigen Perchlorsäurelösung und 500 ml Aether. Nach dem Abkühlen kristallisiert 1-Aethyl-1,2,3,5,6,11-hexahydro-indolizino[8,7-b]indol-4-ium-1-propionsäuremethylester-perchlorat der Formel

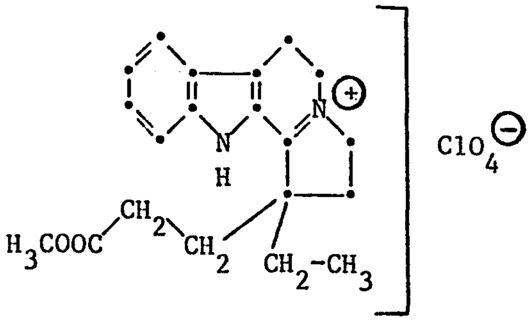

aus; Smp. 144-145°.

Aus der Hexan-Mutterlauge kann durch Aufarbeiten der 1-Aethyl-2,3-dihydro-1H-indolizino[8,7-b]indol-1-propionsäuremethylester der Formel

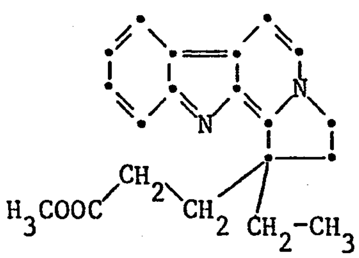

erhalten werden, der nach mehrmaligem Umkristallisieren aus Aether den Smp. 159-160° zeigt. Das Methansulfonat dieser Verbindung der Formel

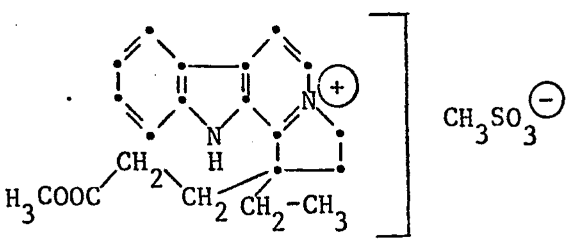

zeigt den Smp. 164-165° , (aus Aceton).

0089926

Beispiel 4: Man versetzt eine Lösung von 13,1 g (0,04 Mol) des nach Beispiel 3 erhaltenen cis-1-Aethyl-2,3,5,6,11,11b-hexahydro-1H-indolizino[8,7-b]indol-1-propionsäuremethylesters in 200 ml Aethylalkohol (95%) mit einer Lösung von 3,2 g Natriumhydroxid in 200 ml Aethylalkohol (95%) und kocht während 2 Stunden unter Rückfluss. Nach dem Abkühlen dampft man das Lösungsmittel am Vakuum ab, löst den Rückstand in 400 ml Wasser und neutralisiert die Lösung mit 2-n. wässriger Essigsäure, worauf nach dem Abkühlen cis-1-Aethyl-2,3,5,6,11,11b-hexahydro-1H-indolizino[8,7-b]indol-1-propionsäure vom Smp. 251-254° auskristallisiert.

Man mischt eine Lösung von 9,4 g (0,03 Mol) der erhaltenen cis-1-Aethyl-2,3,5,6,11,11b-hexahydro-1H-indolizino[8,7-b]indol-1-propionsäure in 50 ml Aethylalkohol (95%) mit einer Lösung von 1,2 g Natriumhydroxid in 100 ml Aethylalkohol (95%). Nach dem Eindampfen im Vakuum wird der Rückstand in 100 ml Methyläthylketon gelöst, die Lösung auf 0 bis 5° gekühlt und unter gutem Rühren innerhalb von 15 Minuten mit 3,75 g Brommethylmethyläther versetzt. Man rührt diese Lösung während 2 Stunden bei Raumtemperatur und dampft hierauf im Vakuum ein. Der Rückstand wird in 300 ml Methylenchlorid und 200 ml Wasser aufgenommen, die organische Phase abgetrennt und zweimal mit je 100 ml Wasser gewaschen, über Natriumsulfat getrocknet, filtriert und eingedampft. Nach Filtration über 50 g Kieselgel 60 (0,063 bis 0,200 mm, Merck) in Essigsäureäthylester erhält man cis-1-Aethyl-2,3,5,6,11,11b-hexahydro-1H-indolizino[8,7-b]indol-1-propionsäuremethoxymethylester vom Smp. 104-105°.

Beispiel 5: Man versetzt eine Lösung von 11,2 g (0,04 Mol) cis-D-Nor-Vincamon (hergestellt nach M. Mailand et al., Chem. Ber. 114, 1926 (1981)) in 350 ml Methanol mit 30 ml 2-n.Natronlauge und kocht während einer Stunde unter Rückfluss. Nach dem Abkühlen dampft man das Lösungsmittel im Vakuum ab, nimmt den Rückstand in 200 ml Wasser auf und neutralisiert die Lösung mit 2-n. Essigsäure, worauf beim Abkühlen cis-1-Aethyl-2,3,5,6,11,11b-hexahydro-1H-indolizino[8,7-b]

indol-1-essigsäure vom Smp. 243-245° (Zersetzung) auskristallisiert.

Man versetzt eine Suspension von 7,45 g (0,025 Mol) der erhaltenen cis-1-Aethyl-2,3,5,6,11,11b-hexahydro-1H-indolizino[8,7-b]indol-1-essigsäure in 400 ml Methanol unter Rühren tropfenweise mit 125 ml einer 1% ätherischen Diazomethanlösung (hergestellt nach T.J. de Boer, H.J. Backer: Org. Synth. Coll. Vol. IV, 250 (1963)). Die entstandene gelbe Lösung wird durch Zusatz einiger Tropfen Essigsäure entfärbt, im Vakuum auf 70 ml eingeengt, mit 6 ml einer 4,5-n.methanolischen Chlorwasserstofflösung schwach sauer gestellt, worauf nach Zusatz von 250 ml Aether beim Abkühlen cis-1-Aethyl-2,3,5,6,11,11b-hexahydro-1H-indolizino[8,7-b]indol-1-essigsäuremethylester-hydrochlorid vom Smp. 238-240° (Zersetzung) auskristallisiert. Smp. der freien Base (Monohydrat): 108-109°, (aus Aether/n-Hexan).

Die beschriebene Umsetzung entspricht folgender Reaktionsgleichung:

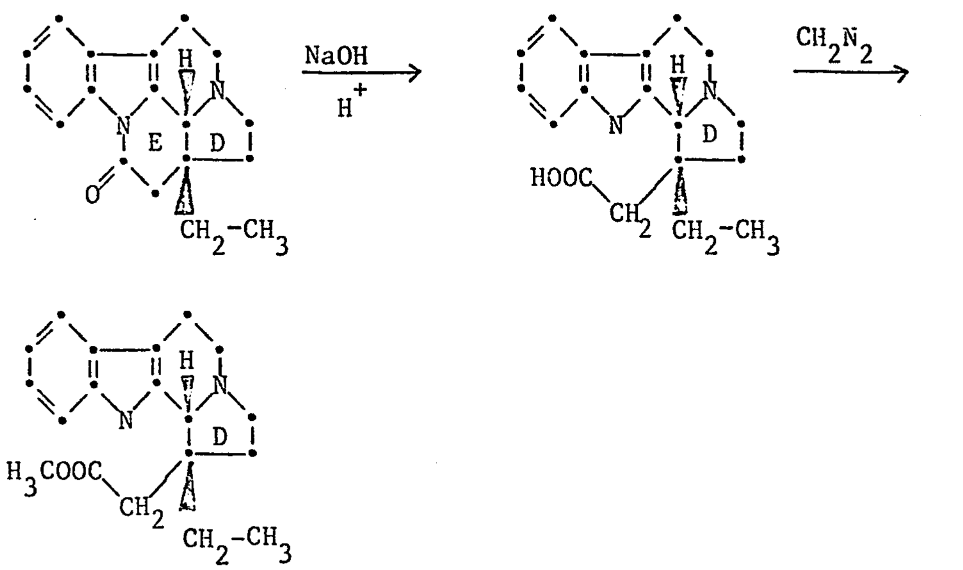


Beispiel 6: In eine Suspension von 3,8 g Lithiumaluminiumhydrid in 250 ml Tetrahydrofuran tropft man in einer Stickstoffatmosphäre unter Rühren innerhalb von 40 Minuten eine Lösung von 34,2 g (0,1 Mol) des nach Beispiel 1 erhaltenen Racemats des threo-cis-α-Hydroxy-1-Aethyl-2,3,5,6,11,11b-hexahydro-1H-indolizino[8,7-b]indol-1-propionsäuremethylesters vom Smp. 219-220° in 750 ml Tetrahydrofuran.

Nach 2 Stunden tropft man vorsichtig 4 ml Wasser, dann 4 ml einer 15% wässrigen Natriumhydroxidlösung und anschliessend weitere 12 ml Wasser zu, filtriert vom weissen Niederschlag ab und dampft das Lösungsmittel im Vakuum ab. Der Rückstand wird in 250 ml Methylenchlorid gelöst, 2 mal mit je 50 ml Wasser extrahiert, die organische Phase über Natriumsulfat getrockner, filtriert und im Vakuum eingedampft. Der Rückstand wird in 250 ml Methanol gelöst, worauf nach dem Abkühlen das threo-cis-3'-[1-Aethyl-2,3,5,6,11,11b-hexahydro-1H-indolizino[8,7-b]indol-1]-propan-1',2'-diol mit 1 Mol Kristall-Methanol vom Smp. 224-225° auskristallisiert.

Zur Ueberführung in das Cyclamat suspendiert man 15,7 g (0,05 Mol) dieser Verbindung in 50 ml Methanol und versetzt unter Rühren mit einer Lösung von 9 g N-Cyclohexylsulfaminsäure in 100 ml Methanol. Der entstandenen Lösung setzt man 300 ml Aether zu, worauf nach dem Abkühlen das threo-cis-3'-[1-Aethyl-2,3,5,6,11,11b-hexahydro-1H-indolizino-[8,7-b]-indol-1]-propan-1',2'-diol-cyclamat vom Smp. 177-179° auskristallisiert.

Die beschriebene Umsetzung entspricht folgender Reaktionsgleichung:

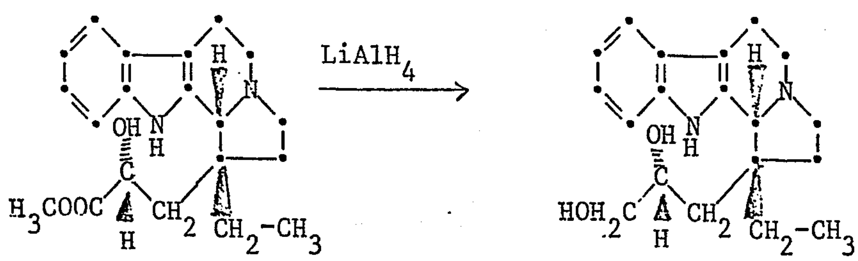


Beispiel 7: In eine Suspension von 3,8 g Lithiumaluminiumhydrid in 250 ml Tetrahydrofuran tropft man in einer Stickstoffatmosphäre unter Rühren innerhalb von 40 Minuten eine Lösung von 34,2 g (0,1 Mol) des nach Beispiel 1 erhaltenen erythro-cis-α-Hydroxy-1-äthyl-2,3,5,6,11,11b-hexahydro-1H-indolizino[8,7-b]indol-1-propionsäuremethylesters vom Smp. 183-185° in 750 ml Tetrahydrofuran.

Nach 2 Stunden tropft man vorsichtig 4 ml Wasser, dann 4 ml einer 15% wässrigen Natriumhydroxidlösung und anschliessend weitere 12 ml Wasser zu, filtriert vom weissen Niederschlag ab und dampft das Lösungsmittel im Vakuum ab. Der Rückstand wird in 250 ml Methylenchlorid gelöst, 2 mal mit je 50 ml Wasser extrahiert, die organische Phase über Natriumsulfat getrocknet, filtriert und im Vakuum eingedampft. Der Rückstand wird in 200 ml Methanol gelöst, worauf nach dem Abkühlen das erythro-cis-3'-[1-Aethyl-2,3,5,6,11,11b-hexahydro-1H-indolizino[8,7-b]indol-1]-1',2'-diol vom Smp. 206-208° auskristallisiert.

Zur Ueberführung in das Methansulfonat löst man 15,7 g (0,05 Mol) dieser Verbindung in 150 ml Methanol und versetzt diese Lösung unter Rühren mit 4,8 g Methansulfonsäure. Nach dem Eindampfen des Lösungsmittels im Vakuum wird der Rückstand in 400 ml Aceton gelöst, worauf nach dem Abkühlen das Methansulfonat der oben genannten erythro-cis-Verbindung vom Smp. 220-222° auskristallisiert.

Die beschriebene Umsetzung entspricht der im Beispiel 6 angegebenen Reaktionsgleichung.

Beispiel 8: In eine Suspension von 3,8 g Lithiumaluminiumhydrid in 250 ml Tetrahydrofuran tropft man in einer Stickstoffatmosphäre unter Rühren innerhalb von 40 Minuten eine Lösung von 34,2 g (0,1 Mol) des nach Beispiel 1 erhaltenen einen Isomeren des trans-α-Hydroxy-1-Aethyl-2,3,5,6,11,11b-hexahydro-1H-indolizino[8,7-b]indol-1-propionsäuremethylesters vom Smp. 153-154° in 750 ml Tetrahydrofuran. Nach 2 Stunden tropft man vorsichtig 4 ml Wasser, dann 4 ml einer 15% wässrigen Natriumhydroxidlösung und anschliessend weitere 12 ml Wasser zu, filtriert vom weissen Niederschlag ab und dampft das Lösungsmittel im Vakuum ab. Der Rückstand wird in 250 ml Methylenchlorid gelöst, 2 mal mit je 50 ml Wasser extrahiert, die organische Phase über Natriumsulfat getrocknet, filtriert und im Vakuum eingedampft. Der Rückstand wird in 150 ml Aether gelöst und mit 250 ml n-Hexan versetzt, worauf nach dem Abkühlen trans-3'-[1-Aethyl-2,3,5,6,11,11b-hexahydro-1H-indolizino[8,7-b]indol-1]-propan-1',2'-diol vom Smp. 167-169° auskristallisiert.

Zur Ueberführung in das Cyclamat löst man 15,7 g (0,05 Mol) dieser Verbindung in 100 ml Methanol und versetzt die erhaltene Lösung unter Rühren mit einer Lösung von 9 g N-Cyclohexylsulfaminsäure in 100 ml Methanol. Man dampft das Lösungsmittel im Vakuum ab und löst den Rückstand in 350 ml Aceton, worauf nach dem Abkühlen trans-3'-[1-Aethyl-2,3,5,6,11,11b-hexahydro-1H-indolizino[8,7-b]indol-1]-propan-1',2'-diol-cyclamat vom Smp. 158-160° auskristallisiert.

Die beschriebene Umsetzung entspricht folgender Reaktionsgleichung:

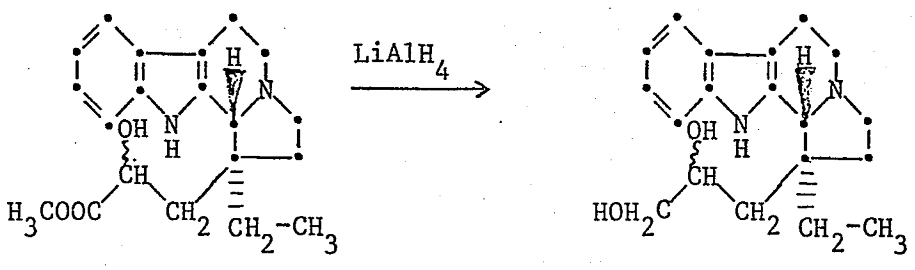


Beispiel 9: Man kocht eine Lösung von 15,45 g (0,05 Mol) des einen Isomeren des cis-α-Amino-D-nor-E-homo-vincamon vom Smp. 139-140° in 500 ml Methanol und 55 ml 1-n.Natronlauge während 1 Stunde unter Rückfluss, lässt erkalten, versetzt unter Kühlung und Rühren mit 55 ml 1-n.Salzsäure und dampft im Vakuum ein. Der Rückstand wird in 300 ml Methylenchlorid suspendiert, filtriert, eingedampft und der Rückstand erneut in 200 ml Methanol gelöst. Unter Rühren versetzt man diese Lösung mit 230 ml einer 1% ätherischen Diazomethanlösung (hergestellt nach: T.J. de Boer und H.J. Backer: Org. Synth. Coll.Vol. IV, 250 (1963)). Die entstandene gelbe Lösung wird durch Zusatz einiger Tropfen Essigsäure entfärbt und im Vakuum eingedampft. Nach Filtration über 250 g Kieselgel 60 (70-230 mesh, Merck) und

Kristallisation aus Aether erhält man das eine Isomere des cis-α-Amino-1-äthyl-2,3,5,6,11,11b-hexahydro-1H-indolizino[8,7-b]indol-1-propionsäuremethylesters der Formel

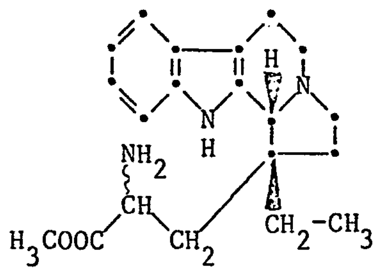

vom Smp. 143-146°; Smp. des Dihydrochlorids (als Sesquihydrat): 227-230°, (aus Methanol/Aether).

Das als Ausgangsmaterial benötigte Isomere des cis-α-Amino-D-nor-E-homo-vincamon vom Smp. 139-140° kann wie folgt hergestellt werden:

a) Man versetzt eine Lösung von 29,4 g (0,1 Mol) cis-D-Nor-E-Homo-Vincamon (hergestellt nach E. Bölsing et al., Chem. Ber. 114, 1932 (1981)) in 600 ml Toluol in einer Stickstoffatmosphäre unter Rühren mit 100 ml 90%igem tert.-Butylnitrit und anschliessend portionenweise mit 29 g Kalium-tertiär-butylat, wobei die Lösung sich auf 45° erwärmt. Man rührt noch eine Stunde, versetzt hierauf mit 1,5 Liter einer 5,4%igen wässrigen Ammoniumchloridlösung, rührt eine weitere Stunde bei Raumtemperatur, worauf α-Hydroximino-cis-D-nor-E-homo-vincamon vom Smp. 225-228° auskristallisiert (aus Methanol).

Die Umsetzung entspricht folgender Reaktionsgleichung:

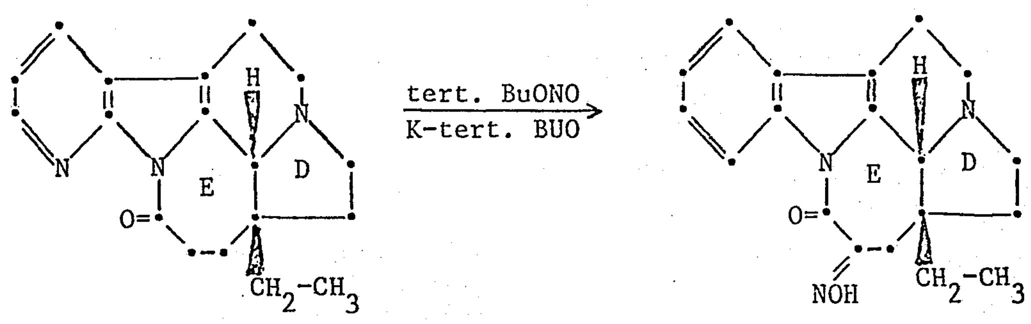


Zur Ueberführung in das Hydrochlorid suspendiert man 16,1 g (0,05 Mol) der erhaltenen Verbindung in 200 ml Methanol und versetzt unter Rühren mit 15 ml einer 3,5-n.methanolischen Chlorwasserstofflösung. Nach Zusatz von 400 ml Aether kristallisiert beim Abkühlen anti-α-Hydroximino-cis-D-nor-E-homo-vincamon-hydrochlorid vom 255-265° (Zersetzung) aus.

b) Eine Lösung von 14,4 g (0,04 Mol) des erhaltenen anti-α-Hydroximino-cis-D-nor-E-homo-vincamon-hydrochlorids in 1,5 Litern Methanol wird in Gegenwart von 5 g Palladium-auf-Kohle Katalysator (5%) während 40 Stunden bei normalem Druck hydriert. Das Reaktionsgemisch wird filtriert, das Filtrat im Vakuum eingedampft, der Rückstand in 300 ml Wasser gelöst und mit konz. wässriger Ammoniaklösung alkalisch gestellt. Nach 3 maliger Extraktion mit je 300 ml Methylenchlorid werden die vereinigten organischen Phasen einmal mit 150 ml Wasser gewaschen, über Natriumsulfat getrocknet, filtriert und im Vakuum eingedampft. Der Rückstand wird in 300 ml Wasser gelöst und mit konz. wässriger Ammoniaklösung alkalisch gestellt. Nach 3 maliger Extraktion mit je 300 ml Methylenchlorid werden die vereinigten organischen Phasen einmal mit 150 ml Wasser gewaschen, über Natriumsulfat getrocknet, filtriert und im Vakuum eingedampft. Der Rückstand wird in 30 ml Aether gelöst, worauf nach dem Abkühlen das eine Isomere des cis-α-Amino-D-nor-E-homo-vincamon der Formel

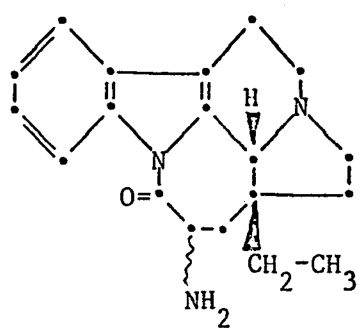

vom Smp. 139-140° auskristallisiert; Smp. des Dihydrochlorids: 290-293°. (Zersetzung)

Beispiel 10: Man kocht eine Lösung von 15,45 g (0,05 Mol) des einen Isomeren des trans-α-Amino-D-nor-E-homo-vincamon vom Smp. 125-127° in 500 ml Methanol und 55 ml 1-n. Natronlauge während 1 Stunde unter Rückfluss, lässt erkalten, versetzt unter Kühlung und Rühren mit 55 ml 1-n. Salzsäure und dampft das Reaktionsgemisch im Vakuum ein. Der Rückstand wird in 300 ml Methylenchlorid suspendiert, filtriert, das Filtrat eingedampft und der Rückstand erneut in 200 ml Methanol gelöst. Unter Rühren versetzt man diese Lösung mit 230 ml einer 1% ätherischen Diazomethanlösung, entfärbt die entstandene gelbe Lösung durch Zusatz einiger Tropfen Essigsäure und dampft im Vakuum ein. Der Rückstand wird in 200 ml Methanol gelöst, die Lösung unter Kühlen und Rühren mit 30 ml einer 3,5-n. methanolischen Chlorwasserstofflösung versetzt, worauf nach Zusatz von 150 ml Aether beim Abkühlen trans-α-Amino-1-äthyl-2,3,5,6,11,11b-hexahydro-1H-indolizino[8,7-b]indol-1-propionsäuremethylester-dihydrochlorid vom Smp. 235-238° (Zersetzung) auskristallisiert. Die daraus auf übliche Weise erhaltene freie Base stellt ein amorphes weisses Pulver dar.

Die Umsetzung entspricht folgender Reaktionsgleichung:

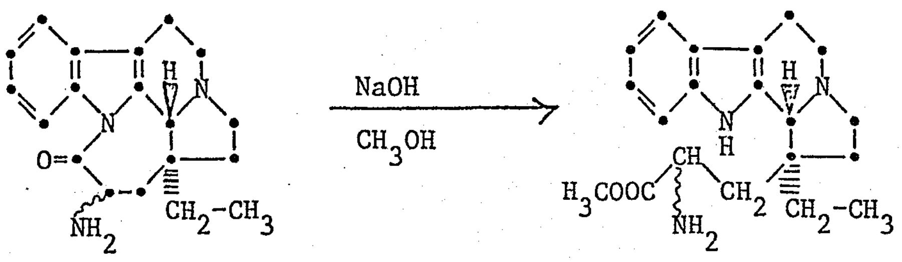


Das Ausgangsmaterial kann auf folgende Weise hergestellt werden:

a) Ein Gemisch von 49 g (0,15 Mol) des nach Beispiel 3 erhaltenen trans-1-Aethyl-2,3,5,6,11,11b-hexahydro-1H-indolizino[8,7b]indol-1-propionsäuremethylesters, 55 g Acetophenon und 8,5 g Kalium-tert.-butylat in 6 Litern Toluol wird in einer Stickstoffatmosphäre während 6 Stunden unter Rühren und Rückfluss gekocht, hierauf auf 0 bis 5° gekühlt und mit 1 Liter 0,5-n. Salzsäure versetzt. Die Toluolphase wird abgetrennt, zweimal mit je 300 ml Wasser gewaschen, die vereinigten wässrigen Phasen mit 2 Litern Methylenchlorid unterschichtet und mit konzentrierter wässriger Ammoniaklösung auf pH 8 eingestellt. Nach Phasentrennung wird die wässrige Phase noch dreimal mit je 750 ml Methylenchlorid extrahiert, die vereinigten organischen Phasen zweimal mit je 500 ml Wasser gewaschen, über Natriumsulfat getrocknet, filtriert und unter Vakuum eingedampft. Der Rückstand wird in 200 ml Methanol gelöst und mit 100 ml Aether versetzt, worauf nach Abkühlen trans-D-Nor-E-homo-Vincamon vom Smp. 131-132° auskristallisiert.

Die Umsetzung entspricht folgender Reaktionsgleichung:

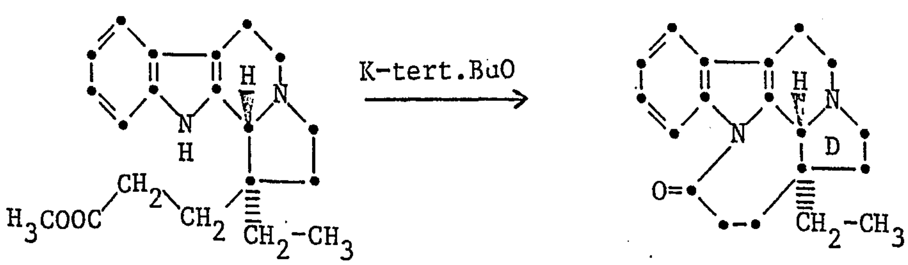

b) Man versetzt eine Lösung von 29,4 g (0,1 Mol) des erhaltenen trans-D-Nor-E-homo-Vincamon in Toluol in einer Stickstoffatmosphäre unter Rühren mit 100 ml 90%-igem tert.-Butylnitrit und anschliessend portionenweise mit 29 g Kalium-tert.-butylat, wobei sich die Lösung auf 45° erwärmt. Man rührt noch eine Stunde, versetzt hierauf mit 1,5 Litern einer 5,4% wässrigen Ammoniumchloridlösung und rührt eine weitere Stunde bei Raumtemperatur. Nach erfolgter Phasentrennung wird die wässrige Phase 3-mal mit je 300 ml Methylenchlorid extrahiert. Die vereinigten organischen Phasen wäscht man noch 2 mal mit je 250 ml Wasser, trocknet über Natriumsulfat, filtriert und dampft das Lösungsmittel im Vakuum ab. Der Rückstand wird in 300 ml heissem Methanol gelöst, worauf beim Abkühlen α-anti-Hydroxyimino-trans-D-Nor-E-homo-Vincamon vom Smp. 212-214° auskristallisiert.

Die Umsetzung entspricht folgender Reaktionsgleichung:

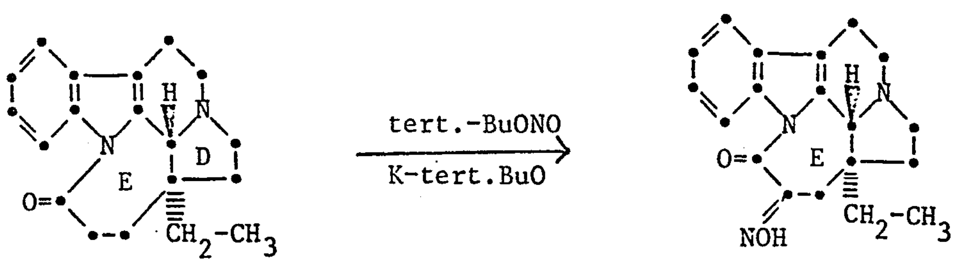


Zur Ueberführung in das Hydrochlorid suspendiert man 16,1 g (0,05 Mol) der erhaltenen Verbindung in 100 ml Methanol und versetzt unter Rühren mit 15 ml einer 3,5-n.methanolischen Chlorwasserstofflösung. Nach Zugabe von 200 ml Aether kristallisiert beim Abkühlen α-anti-Hydroximino-trans-D-Nor-E-homo-Vincamon-hydrochlorid vom Smp. 213-217° (Zersetzung) aus.

c) Eine Lösung von 14,4 g (0,0 Mol) des erhaltenen α-anti-Hydroimino-trans-D-Nor-E-homo-Vincamon-hydrochlorids in 500 ml Eisessig wird in Gegenwart von 5 g Palladium-auf-Kohle-Katalysator (5%) während 90 Stunden bei 25° bei normalem Druck hydriert. Das Reaktionsgemisch wird filtriert, das Filtrat im Vakuum eingedampft, der Rückstand in 300 ml Wasser gelöst und mit konz. wässriger Ammoniaklösung alkalisch eingestellt. Nach 3-maliger Extraktion mit je 300 ml Methylenchlorid werden die vereinigten organischen Phasen einmal mit 150 ml Wasser gewaschen, über Natriumsulfat getrocknet, filtriert und das Filtrat im Vakuum eingedampft. Der Rückstand wird in 80 ml Aether gelöst, worauf beim Abkühlen das eine Isomere des trans-α-Amino-D-Nor-E-homo-Vincamons vom Smp. 125-127° auskristallisiert.

Die Umsetzung entspricht folgender Reaktionsgleichung:

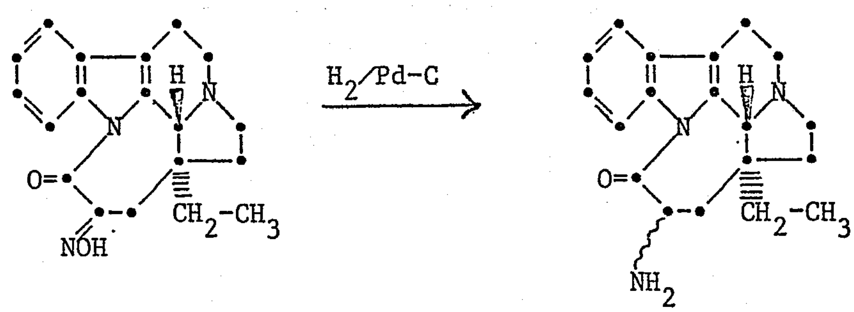


Beispiel 11: Eine Lösung von 386 g (0,75 Mol) 1-Aethyl-11-benzyl-1,2,3,5,6,11-hexahydro-indolizino[8,7-b]indol-4-ium-1-propionsäuremethylester-perchlorat und 75 g wasserfreies Kaliumacetat in 6,5 Litern Methanol wird analog der im Beispiel 3 beschriebenen Weise bis zur Aufnahme von 2 Moläquivalenten Wasserstoff hydriert und die Hydrierlösung, wie dort beschrieben, aufgearbeitet wonach man cis-1-Aethyl-2,3,5,6,11,11b-hexahydro-1H-indolizino[8,7-b]indol-1-propionsäuremethylester der dort angegebenen Formel vom Smp. 162-163° erhält. Die Aufarbeitung der Mutterlauge ergibt den im Beispiel 3 beschriebenen trans-1-Aethyl-2,3,5,6,11,11b-hexahydro-1H-indolizino[8,7-b]indol-1-propionsäuremethylester vom Smp. 118-121°.

Das Ausgangsmaterial kann wie folgt erhalten werden:

a) Analog der im Beispiel 2 b) beschriebenen Arbeitsweise setzt man 250 g (1 Mol) N-Benzyl-tryptamin (J.Med.Chem. 9, 793-94 (1966)) mit 170 g 2-Aethyl-4-chlor-butyrylchlorid um. Nach dem Aufarbeiten erhält man 3-Aethyl-N-[2-(3'-N-benzylindolyl)-äthyl]-2-pyrrolidon, welches als solches weiterverarbeitet wird.

b) Ein Gemisch von 346 g (1Mol) der erhaltenen Verbindung und 800 ml Phosphoroxychlorid wird analog der im Beispiel 2 c) beschriebenen Arbeitsweise umgesetzt, wobei man nach dem Aufarbeiten schliesslich das 1-Aethyl-11-benzyl-1,2,3,5,6,11-hexahydro-indolizino[8,7-b]indol-4-ium-perchlorat erhält, welches als solches weiterverarbeitet wird.

c) Eine Lösung von 214,3 g (0,5 Mol) des erhaltenen Produkts in 500 ml Dimethylsulfoxid und 1 Liter Toluol wird analog Beispiel 2 d) mit 670 ml n.Natronlauge behandelt. Nach dem Aufarbeiten erhält man 1-Aethyl-11-benzyl-2,3,6,11-tetrahydro-5H-indolizino[8,7-b]indol als Rohprodukt.

d) Unter einer Stickstoffatmosphäre rührt man ein Gemisch von 41,0 g (0,125 Mol) des nach Beispiel c) erhaltenen Rohprodukts, 400 ml Methylenchlorid, 30 g Acrylsäuremethylester und 5 ml Methanol während 65 Stunden bei Raumtemperatur, dampft anschliessend das Reaktionsgemisch im Vakuum ein und behandelt den öligen Rückstand zur Entfernung überschüssigen Acrylsäuremethylesters 3 mal mit je 50 ml n-Hexan, dekantiert die Hexanlösung ab, löst den Rückstand bei einer Temperatur von 0 bis 5° in 500 ml Methanol und fügt unter Rühren innerhalb von 10 Minuten 15 ml einer 70%igen wässrigen Perchlorsäurelösung und anschliessend 50 ml Aether zu. Durch Abkühlen des Reaktionsgemisches auf 0° kristallisiert nach längerem Stehen 1-Aethyl-11-benzyl-1,2,3,5,6,11-hexahydro-indolizino[8,7-b]indol-4-ium-1-propionsäure-methylester-perchlorat aus.

Beispiel 12: Eine Lösung von 334 g (0,75 Mol) α-Hydroximino-*cis*-1-äthyl-11-benzyl-2,3,5,6,11,11b-hexahydro-1H-indolizino[8,7-b]indol-1-propionsäuremethylester in 3 Litern Methanol wird in Gegenwart von

30 g Palladium-auf-Kohle-Katalysator bis zur beendeten Wasserstoffaufnahme hydriert. Nach dem Abfiltrieren des Katalysators und Eindampfen des Filtrats unter vermindertem Druck filtriert man über 1250 g Kieselgel 60 (70-230 mesh, Merck) und kristallisiert den Filterrückstand aus Aether um, wonach man das eine Isomere des cis-α-Amino-1-äthyl-2,3,5,6,11,11b-hexahydro-1H-indolizino[8,7-b]indol-1-propionsäuremethylesters vom Smp. 143-146° erhält.

Der als Ausgangsmaterial verwendete α-Hydroximino-cis-1-äthyl-11-benzyl-2,3,5,6,11,11b-hexahydro-1H-indolizino[8,7-b]indol-1-propionsäuremethylester kann wie folgt hergestellt werden:

a) Eine Lösung von 51,5 g (0,1 Mol) des nach Beispiel 11 d) hergestellten 1-Aethyl-11-benzyl-1,2,3,5,6,11-hexahydro-indolizino[8,7-b]indol-4-ium-1-propionsäuremethylester-perchlorat in 550 ml Tetrahydrofuran wird auf 0° gekühlt und unter einer Stickstoffatmosphäre mit 76,3 g (0,3 Mol) Lithium-tri-tert.butoxy-aluminiumhydrid versetzt. Nach 4-stündigem Rühren des Reaktionsgemisches bei 0° unter einer Stickstoffatmosphäre wird unter vermindertem Druck auf ein Volumen von etwa 150 ml eingeengt und die Lösung auf 2 Liter Eiswasser gegossen. Nach dem Absaugen wird der Filterrückstand mit 400 ml Tetrahydrofuran ausgekocht, abfiltriert und das Filtrat unter vermindertem Druck zur Trockne eingedampft, wonach man cis-1-Aethyl-11-benzyl-2,3,5,6,11,11b-hexahydro-1H-indolizino[8,7-b]indol-1-propionsäuremethylester als Rohprodukt erhält.

b) Man versetzt eine Lösung von 41,6 g (0,1 Mol) des erhaltenen Rohproduktes in 600 ml Toluol in einer Stickstoffatmosphäre unter Rühren mit 100 ml 90%igem tert.-Butylnitrit und anschliessend portionenweise mit 29 g Kalium-tert.-butylat. Nach weiterem Rühren während 1 Stunde fügt man 1,5 Liter einer 5,4%igen wässrigen Ammoniumchloridlösung zu, rührt noch eine weitere Stunde bei Raumtemperatur und engt anschliessend das Reaktionsgemisch weitgehend ein, versetzt den Rückstand mit 300 ml Wasser und filtriert den ausgefallenen α-Hydroximino-cis-1-äthyl-11-benzyl-2,3,5,6,11,11b-hexahydro-1H-indolizino[8,7-b]indol-1-propionsäuremethylester ab.

Beispiel 13: Zu einer Lösung von 48,0 g (0,1 Mol) cis-1-Aethyl-11-(p-tosyl)-2,3,5,6,11,11b-hexahydro-1H-indolizino[8,7-b]indol-1-propionsäuremethylester in 1 Liter Aethanol fügt man 240 ml einer 2-n.wässrigen Natriumhydroxidlösung und kocht das Gemisch während 60 Stunden unter einer Stickstoffatmosphäre unter Rückfluss. Nach dem Erkalten wird die erhaltene Lösung unter vermindertem Druck eingedampft, der Rückstand in 1200 ml Wasser gelöst und die Lösung mit 2-n wässriger Essigsäure neutralisiert, worauf nach dem Abkühlen cis-1-Aethyl-2,3,5,6,11,11b-hexahydro-1H-indolizino[8,7-b]indol-1-propionsäure vom Smp. 251-254° auskristallisiert.

Das Ausgangsmaterial kann wie folgt erhalten werden:

a) Man versetzt eine Lösung von 156 g (1 Mol) Indol-2-acetonitril in 2 Litern Methylenchlorid bei 0-5° unter Rühren in einer Stickstoffatmosphäre mit 2 Litern einer 2-n. wässrigen Natriumhydroxidlösung und 5 g Tetra-n-butylammonium-hydrogensulfat und anschliessend portionenweise mit 210 g p-Tosylchlorid. Nach weiterem Rühren das Reaktionsgemisches während 3 Stunden bei 0-5° lässt man auf Raumtemperatur erwärmen und rührt weitere 22 Stunden bei einer Temperatur von 22°, trennt die beiden Schichten, wäscht die organische Phase 3 mal mit je 500 ml Wasser, trocknet über Natriumsulfat und dampft die Lösung im Vakuum ein. Der erhaltene Schaum wird in 750 ml Methylenchlorid gelöst und die Lösung mit 3 Litern Aether versetzt, worauf nach dem Abkühlen 1-(p-Tosyl-indol)-3-acetonitril vom Smp. 162-163° auskristallisiert.

b) Eine Lösung von 190 g (0,61 Mol) 1-(p-Tosyl-indol)-3-acetonitril in 2 Litern einer 10%igen Lösung von Ammoniakgas in absolutem Aethanol wird in Gegenwart von 50 g eines Rhodium(0,5%)-auf-Aluminiumoxid-Katalysators bei Raumtemperatur und einem Druck von 4 bar während 24 Stunden hydriert. Nach dem Abfiltrieren im Vakuum löst man den erhaltenen Schaum in 1,5 Litern Aether, worauf nach dem Abkühlen 1-(p-Tosyl-indol)-3-(2-äthylamin) vom Smp. 122-124° auskristallisiert; Smp. des Hydrochlorids • 1/3 Hydrat: 205-207° (aus Aceton/Aether).

c) Analog der im Beispiel 2 b) beschriebenen Arbeitsweise setzt man 31,4 g (0,1 Mol) 1-(p-Tosyl-indol)-3-(2-äthylamin) mit 17 g 2-Aethyl-4-chlor-butyrylchlorid um. Nach dem Aufarbeiten erhält man 3-Aethyl-N-[2-(3'-p-tosyl-indol)-äthyl]-2-pyrrolidon, welches als Rohprodukt weiterverarbeitet wird.

d) Man setzt 41 g (0,1 Mol) der erhaltenen Verbindung analog der im Beispiel 2 c) beschriebenen Arbeitsweise mit 80 ml Phosphoroxychlorid um, wobei man nach dem Aufarbeiten und Umsetzen mit Natriumperchlorat das 1-Aethyl-11-(p-tosyl)-1,2,3,5,6,11-hexahydro-indolizino[8,7-b]indol-4-ium-perchlorat als Rohprodukt erhält, welches als solches weiterverarbeitet wird.

e) Eine Lösung von 49,3 g (0,1 Mol) der erhaltenen rohen Verbindung löst man in 100 ml Dimethylsulfoxid und 250 ml Toluol und fügt unter einer Stickstoffatmosphäre 134 ml 1-n. wässrige Natriumhydroxidlösung zu. Nach dem Verdünnen der Lösung mit 400 ml Toluol wird die organische Phase abgetrennt, mit Wasser gewaschen, über Kaliumcarbonat getrocknet und das Filtrat im Vakuum eingedampft, wonach man 1-Aethyl-11-(p-tosyl)-2,3,6,11 —tetrahydro-5H-indolizino[8,7-b]indol als Rohprodukt erhält.

f) Ein Gemisch von 39,2 g (0,1 Mol) des nach Beispiel c) erhaltenen Rohproduktes, 325 ml Methylenchlorid, 30 g Acrylsäuremethylester und 5 ml Methanol wird in einer Stickstoffatmosphäre während 70 Stunden bei Raumtemperatur gerührt und anschliessend das Reaktionsgemisch im Vakuum weitgehend eingedampft. Zur Entfernung überschüssigen Acrylsäuremethylesters behandelt man den Rückstand 3 mal mit je 70 ml n-Hexan, dekantiert die Hexanlösung ab, löst den Rückstand in 450 ml Methanol und fügt unter Rühren bei einer Temperatur von 0 bis 5° innerhalb von 10 Minuten 12 ml einer 70%igen wässrigen Perchlorsäurelösung und anschliessend 50 ml Aether zu. Nach dem Abkühlen auf 0° kristalliert nach längerem Stehen 1-Aethyl-11-(p-tosyl)-1,2,3,5,6,11-hexahydro-indolizino[8,7-b]indol-4-ium-1-propionsäuremethylester-perchlorat aus.

g) Eine Lösung von 434 g (0,75 Mol) der erhaltenen Verbindung wird genau analog der im Beispiel 11 beschriebenen Arbeitsweise reduziert,

wonach man beim Aufarbeiten der Hydrierlösung cis-1-Aethyl-11-(p-tosyl)-2,3,5,6,11,11b-hexahydro-1H-indolizino[8,7-b]indol-1-propionsäuremethylester erhält. Durch Aufarbeiten der angefallenen Mutterlauge erhält man die entsprechende trans-Verbindung, nämlich trans-1-Aethyl-11-(p-tosyl)-2,3,5,6,11,11b-hexahydro-1H-indolizino[8,7-b]indol-1-propionsäuremethylester.

Beispiel 14: Zu einer Lösung von 51,5 g (0,1 Mol) cis-1-Aethyl-11-(p-tosyl)-2,3,5,6,11,11b-hexahydro-1H-indolizino[8,7-b]indol-1-(α-chlor)-propionsäuremethylester in 240 ml Aethanol fügt man 110 ml 3-n.Natriumhydroxidlösung zu und kocht das Gemisch unter einer Stickstoffatmosphäre während 60 Stunden unter Rückfluss. Nach dem Erkalten dampft man die erhaltene Lösung zur Trockne ein, löst den Rückstand in 1100 ml Wasser und neutralisiert mit 2-n.Essigsäure, worauf nach dem Abkühlen cis-α-Hydroxy-1-äthyl-2,3,5,6,11,11b-hexahydro-1H-indolizino[8,7-b]indol-1-propionsäure als Gemisch der threo- und erythro-Form ausfällt.

Zur Trennung dieses Gemisches wird die erhaltene Verbindung mittels Diazomethan in ätherischer Lösung in den entsprechenden Methylester überführt, die Aetherlösung zur Trockne eingedampft, der Rückstand in 300 ml Methanol gelöst und 300 ml Aether zugefügt. Nach dem Abkühlen kristallisiert, wie im Beispiel 1 beschrieben, das Racemat des threo-cis-α-Hydroxy-1-äthyl-2,3,5,6,11,11b-hexahydro-1H-indolizino[8,7-b]indol-1-propionsäuremethylesters vom Smp. 219-220° aus.

Aus der Mutterlauge wird, wie im Beispiel 1 beschrieben, das Racemat der erythro-cis-Form dieser Verbindung als Hydrochlorid vom Smp. 232-234° isoliert. Durch Hydrolyse dieser beiden Verbindungen mittels 2-n.wässriger Natriumhydroxidlösung in äthanolischer Lösung wird jeweils das Racemat der threo- bzw. erythro-cis-α-Hydroxy-1-äthyl-2,3,5,6,11,11b-hexahydro-1H-indolizino[8,7-b]indol-1-propionsäure erhalten.

Das Ausgangsmaterial kann wie folgt erhalten werden:

a) 39,2 g (0,1 Mol) des nach Beispiel 13 e) erhaltenen 1-Aethyl-11-(p-tosyl)-2,3,6,11-tetrahydro-5H-indolizino[8,7-b]indols werden analog der im Beispiel 13 f) beschriebenen Arbeitsweise mit 42 g mit Hydrochinon stabilisiertem α-Chloracrylsäuremethylester (C.S. Marvel, J.C. Cowan: JACS 61, 3156-59 (1939)) umgesetzt. Nach dem Aufarbeiten und der im Beispiel 13 f) beschriebenen Umsetzung mit 12 ml einer 70%igen wässrigen Perchlorsäurelösung erhält man schliesslich 1-Aethyl-11-(p-tosyl)-1,2,3,5,6,11-hexahydro-indolizino[8,7-b]indol-4-ium-1-(α-chlor)-propionsäuremethylester-perchlorat.

b) 61,3 g (0,1 Mol) der erhaltenen rohen Verbindung werden entsprechend der im Beispiel 12 a) beschriebenen Arbeitsweise reduziert, wonach man nach entsprechendem Aufarbeiten cis-1-Aethyl-11-(p-tosyl)-2,3,5,6,11,11b-hexahydro-1H-indolizino[8,7-b]indol-1-(α-chlor)-propionsäuremethylester erhält, der als solcher weiterverarbeitet wird.

Beispiel 15: Eine Lösung von 317 g (0,75 Mol) 1-Vinyl-1,2,3,5,6,11-hexahydro-indolizino[8,7-b]indol-4-ium-1-propionsäuremethylester-perchlorat und 75 g wasserfreiem Kaliumacetat wird entsprechend der im Beispiel 3 beschriebenen Arbeitsweise hydriert und aufgearbeitet, wonach man den dort beschriebenen cis-1-Aethyl-2,3,5,6,11,11b-hexahydro-1H-indolizino[8,7-b]indol-1-propionsäuremethylester vom Smp. 162-163° erhält. Durch Aufarbeiten der angefallenen Mutterlauge erhält man den trans-1-Aethyl-2,3,5,6,11,11b-hexahydro-1H-indolizino[8,7-b]indol-1-propionsäuremethylester vom Smp. 118-121°.

Das Ausgangsmaterial kann man wie folgt erhalten:

a) 800 g (5 Mol) Tryptamin werden gemäss der im Beispiel 2 b) beschriebenen Arbeitsweise mit 710 g 4-Chlorbutyrylchlorid umgesetzt. Nach dem dort beschriebenen Aufarbeiten des Reaktionsgemisches erhält man N-[2-(3'-indol)-äthyl]-2-pyrrolidon als Rohprodukt.

b) Ein Gemisch von 114 g (0,5 Mol) der erhaltenen Verbindung und 400 ml Phosphoroxychlorid wird analog Beispiel 2 c) umgesetzt und aufgearbeitet, wonach man 1,2,3,5,6,11-Hexahydro-indolizino[8,7-b]indol-4-ium-perchlorat analog der im Beispiel 2 c) dargestellten Formel erhält.

c) In eine Lösung von 155,2 g (0,5 Mol) der gemäss b) erhaltenen Verbindung in 500 ml Dimethylsulfoxid und 1 Liter Toluol trägt man unter einer Stickstoffatmosphäre 635 ml 1-n.Natronlauge ein. Nach dem Verdünnen des Reaktionsgemisches mit 2 Litern Toluol wird die organische Phase abgetrennt, mit 350 ml Wasser gewaschen, über Natriumcarbonat getrocknet und die Lösung im Vakuum eingedampft, wonach man 2,3,6,11-Tetrahydro-indolizino[8,7-b]indol als Rückstand erhält.

d) Ein Gemisch von 21 g (0,1 Mol) der erhaltenen Verbindung wird gemäss der im Beispiel 3 a) beschriebenen Arbeitsweise mit 30 g Acrylsäuremethylester umgesetzt und das Reaktionsgemisch, wie angegeben, aufgearbeitet. Nach der beschriebenen Umsetzung mit 12 ml einer 70%igen wässrigen Perchlorsäurelösung erhält man schliesslich 1,2,3,5,6,11-Hexahydro-indolizino[8,7-b]indol-4-ium-1-propionsäuremethylester-perchlorat.

e) Gemäss der unter c) beschriebenen Arbeitsweise werden 198,2 g (0,5 Mol) der erhaltenen Verbindung in den 2,3,6,11-Tetrahydro-indolizino[8,7-b]indol-1-propionsäuremethylester umgewandelt, der als Rohprodukt erhalten und als solcher weiterverarbeitet wird.

f) 12 g Vinylbromid, 5 g Kaliumjodid und 29,6 g (0,1 Mol) der nach e) erhaltenen Verbindung werden zusammen mit 50 ml Metyhlenchlorid im Bombenrohr während 60 Stunden bei einer Temperatur von 55-60° gehalten. Nach beendeter Umsetzung werden die flüchtigen Teile abgedampft, der Rückstand in 150 ml Methanol gelöst und unter Rühren bei einer Temperatur von 0-5° innerhalb von 10 Minuten mit 12 ml einer 70%igen wässrigen Perchlorsäurelösung versetzt. Nach Zugabe von 60 ml Aether kristallisiert beim Abkühlen 1-Vinyl-1,2,3,5,6,11-hexahydro-indolizino[8,7-b]indol-4-ium-1-propionsäuremethylester-perchlorat aus.

Beispiel 16: Zu einer Lösung von 34,2 g (0,1 Mol) threo-cis-α-Hydroxy-1-äthyl-2,3,5,6,11,11b-hexahydro-1H-indolizino[8,7-b]indol-1-propionsäuremethylester in 300 ml Methylenchlorid und 25 ml Pyridin tropft

man unter Rühren in einer Stickstoffatmosphäre bei einer Temperatur von 0-5° eine Lösung von 16,9 g Benzoylchlorid in 150 ml Methylenchlorid innerhalb von 30 Minuten zu. Nach weiteren 2 Stunden giesst man die Lösung auf 300 ml Eiswasser und 100 ml 10%ige wässrige Natriumbicarbonatlösung, wäscht die organische Phase dreimal mit je 50 ml Wasser, trocknet über Natriumsulfat und dampft die Lösung im Vakuum ein. Nach dem Umkristallisieren des Rückstands aus Essigsäureäthylester erhält man threo-cis-α-Benzoyloxy-1-äthyl-2,3,5,6,11,11b-hexahydro- 1H -indolizino[8,7-b]indol-1-propionsäuremethylester vom Smp. 128-130°; Smp. des Salzes mit Benzoesäure: 142-144° (aus Essigsäureäthylester).

Beispiel 17: Entsprechend der im Beispiel 16 beschriebenen Arbeitsweise setzt man 32,2 g (0,1 Mol) erythro-cis-α-Hydroxy-1-äthyl-2,3,5,6,11,11b-hexahydro-1H-indolizino[8,7-b]indol-1-propionsäuremethylester mit 16,9 g Benzoylchlorid um. Nach dem Aufarbeiten wird der nach dem Abdampfen der organischen Phase erhaltene Rückstand aus Aceton-Aether umkristallisiert, wonach man erythro-cis-α-Benzoyloxy-1-äthyl-2,3,5,6,11,11b-hexahydro-1H-indolizino[8,7-b]indol-1-propionsäuremethylester vom Smp. 158-160° erhält.

Beispiel 18: Eine Lösung von 32,6 g (0,1 Mol) cis-1-Aethyl-2,3,5,6,11-11b-hexahydro-1H-indolizino[8,7-b]indol-1-propionsäuremethylester in 200 ml einer 6-n. methanolischen Ammoniaklösung wird im Bombenrohr während 24 Stunden auf 100° erhitzt. Nach dem Eindampfen des Reaktionsgemisches im Vakuum wird der Rückstand in 100 ml Methanol gelöst, worauf nach dem Abkühlen cis-1-Aethyl-2,3,5,6,11,11b-hexahydro-1H-indolizino[8,7-b]indol-1-propionsäureamid vom Smp. 202-204° auskristallisiert.

Beispiel 19: Eine Lösung von 34,1 g (0,1 Mol) cis-α-Amino-1-äthyl-2,3,5,6,11,11b-hexahydro-1H-indolizino[8,7-b]indol-1-propionsäuremethylester in 200 ml einer 6-n.methanolischen Ammoniaklösung wird im Bombenrohr während 24 Stunden auf 80° erhitzt. Nach dem Eindampfen im

Vakuum wird der Rückstand in 150 ml Methanol gelöst, worauf nach dem Abkühlen cis-α-Amino-1-äthyl-2,3,5,6,11,11b-hexahydro-1H.indolizino[8,7-b]indol-1-propionsäureamid vom Smp. 208-210° auskristallisiert.

Beispiel 20: Eine Lösung von 56,7 g (0,1 Mol) cis-1-Aethyl-11-(p-tosyl)-2,3,5,6,11,11b-hexahydro-1H-indolizino[8,7-b]indol-1-(α-äthoxycarbonylamino)-propionsäuremethylester in 260 ml Aethanol wird analog der im Beispiel 14 beschriebenen Verfahrensweise hydrolysiert. Nach dem Aufarbeiten erhält man das eine Isomere der cis-α-Amino-1-äthyl-2,3,5,6,11,11b-hexahydro-1H-indolizino[8,7-b]indol-1-propionsäure, deren Methylester, welcher durch Umsetzung der erhaltenen Carbonsäure mit Diazomethan in ätherischer Lösung erhalten wird, den Smp. 143-146° zeigt.

Das Ausgangsmaterial kann wie folgt erhalten werden:

Zu einer Lösung von 51,5 g (0,1 Mol) des nach Beispiel 14 b) erhaltenen cis-1-Aethyl-11-(p-tosyl)-2,3,5,6,11,11b-hexahydro-1H-indolizino[8,7-b]indol-1-(α-chlor)-propionsäuremethylesters in 120 ml wasserfreiem Dimethylformamid fügt man 12,2 g Kaliumcyanat und 22 ml Methanol zu und erhitzt das Gemisch während 2 Stunden unter kräftigem Rühren auf 100°, filtriert die nach dem Abkühlen ausgefallenen Salze ab, entfernt das Dimethylformamid unter vermindertem Druck, löst den Rückstand in 120 ml Aceton, filtriert von den ausgefallenen Salzen ab und engt das Filtrat im Vakuum ein, wonach man cis-1-Aethyl-11-(p-tosyl)-2,3,5,6,11,11b-hexahydro-1H-indolizino[8,7-b]indol-1-(α-äthoxycarbonylamino)-propionsäuremethylester erhält.

Beispiel 21: Analog den in der allgemeinen Beschreibung beschriebenen und in den vorhergehenden Beispielen illustrierten Verfahrensweisen können, unter Anwendung entsprechender Ausgangsstoffe und/oder Verfahrensweisen, auch folgende Verbindungen der Formel 1, deren N-Oxide oder Salze hergestellt werden:

a) cis-α-Methylthio-1-äthyl-2,3,5,6,11,11b-hexahydro-1H-indolizino-[8,7-b]indol-1-propionsäuremethylester;

b) cis-8-Brom-α-hydroxy-1-äthyl-2,3,5,6,11,11b-hexahydro-1H-indolizino[8,7-b]indol-1-propionsäuremethylester;

c) cis-9-Amino-α-hydroxy-1-äthyl-2,3,5,6,11,11b-hexahydro-1H-indolizino[8,7-b]indol-1-propionsäuremethylester;

d) trans-2'-Amino-3'-[1-äthyl-2,3,5,6,11,11b-hexahydro-1H-indolizino[8,7-b]indol]-propan-1'-ol;

e) 3'-(8,9-Methylendioxy-1-äthyl-2,3,5,6,11,11b-hexahydro-1H-indolizino[8,7-b]indol)-acrylsäuremethylester;

f) 1-n-Propyl-2,3,5,6,11,11b-hexahydro-1H-indolizino[8,7-b]indol-1-propionsäuremethylester;

g) 1-n-Butyl-2,3,5,6,11,11b-hexahydro-1H-indolizino[8,7-b]indol-1-propionsäuremethylester;

h) cis-8-Brom-α-hydroxy-1-äthyl-2,3,5,6,11,11b-hexahydro-1H-indolizino[8,7-b]indol-1-propionsäuredimethylamid;

i) cis-8-Brom-α-hydroxy-1-äthyl-2,3,5,6,11,11b-hexahydro-1H-indolizino[8,7-b]indol-1-propionsäuremorpholid;

j) trans-2'-Amino-3'-[8-brom-1-äthyl-2,3,5,6,11,11b-hexahydro-1H-indolizino[8,7-b]indol]-propan-1'-ol;

k) cis-α-Benzoylamino-1-äthyl-2,3,5,6,11,11b-hexahydro-1H-indolizino[8,7-b]indol-1-propionsäurethioäthylester;

l) cis-α-Aethoxy-2,3,5,6,11,11b-hexahydro-1H-indolizino[8,7-b]indol-1-propionsäure-3-phthalidylester;

m) cis-7-Aethoxycarbonylamino-1-äthyl-2,3,5,6,11,11b-hexahydro-1H-indolizino[8,7-b]indol-1-essigsäure-(2-hydroxypropyl)-ester;

n) trans-α-Pivaloyloxy-1-äthyl-2,3,5,6,11,11b-hexahydro-1H-indolizino[8,7-b]indol-1-propionsäure-(3,3,5-trimethyl-cyclohexyl)-ester;

o) cis-α-Hydroxy-1-äthyl-11-methyl-2,3,5,6,11,11b-hexahydro-1H-indolizino[8,7-b]indol-1-propionsäure-4-N-oxid;

p) cis-α-Dimethylamino-1-äthyl-10-methoxy-2,3,5,6,11,11b-hexahydro-1H-indolizino[8,7-b]indol-1-propionsäure-(2'-piperidinoäthyl)-ester.

q) cis-1-n-Propyl-8-acetoxy-11-methyl-2,3,5,6,11,11b-hexahydro-1H-indolizino[8,7-b]indol-1-propionsäure-(N'-nicotinoyl)-hydrazid;

r) trans-9-Nitro-2,3,5,6,11,11b-hexahydro-1H-indolizino[8,7-b]indol-1-propionsäure-(2-phenyläthyl)-amid;

s) cis-3'-[1-Aethyl-8-trifluormethyl-2,3,5,6,11,11b-hexahydro-1H-indolizino[8,7-b]indol-yl]-propan-1'-ol-3,4,5-trimethoxybenzoesäure-ester;

t) cis-α-p-Toluolsulfonyloxy-1-äthyl-8-benzyloxymethylen-2,3,5,6,11,11b-hexahydro-1H-indolizino[8,7-b]indol-1-propionsäure-[2-(4-methylpiperazino)-äthyl]-ester;

u) trans-2'-Aethylthio-3'-[1-äthyl-8-methyl-9-dimethylamino-2,3,5,6,11,11b-hexahydro-1H-indolizino[8,7-b]indol-yl]-propan-1-ol;

v) cis-α-Methyl-1-äthyl-2,3,5,6,11,11b-hexahydro-1H-indolizino[8,7-b]-indol-1-propionsäureamid;

w) cis-1-Aethyl-2,3,5,6,11,11b-hexahydro-1H-indolizino[8,7-b]-indol-1-propionsäuremethylester-$N^4$-ammonium-methojodid;

x) trans-3'-[1-Aethyl-2,3,5,6,11,11b-hexahydro-1H-indolizino[8,7-b]-indol-yl]-propan-1'-ol-2-trimethylammoniumchlorid;

y) cis-1-Aethyl-2,3,5,6,11,11b-hexahydro-1H-indolizino[8,7-b]indol-1-propionitril;

z) trans-8-Brom-1-äthyl-2,3,5,6,11,11b-hexahydro-1H-indolizino[8,7-b] indol-1-propionitril;

z') erythro-cis-α-Hydroxy-1-äthyl-2,3,5,6,11,11b-hexahydro-1H-indolizino[8,7-b]indol-1-propionsäuremorpholid;

z") erythro-cis-α-Hydroxy-1-äthyl-2,3,5,6,11,11b-hexahydro-1H-indolizino[8,7-b]indol-1-propionsäure-(4-methyl)-piperazid.

Beispiel 22: Tabletten enthaltend 20 mg an aktiver Substanz werden in folgender Zusammensetzung in üblicher Weise hergestellt:

Zusammensetzung:

| | |
|---|---|
| cis-1-Aethyl-2,3,5,6,11,11b-hexahydro-1H-indolizino-[8,7-b]indol-1-propionsäuremethylester-methansulfonat | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

cis-1-Aethyl-2,3,5,6,11,11b-hexahydro-1H-indolizino[8,7-b]indol-1-propionsäuremethylester-methansulfonat wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister geknetet, bis eine schwach plastische Masse entstanden ist. Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 23: Kapseln enthaltend 10 mg an aktiver Substanz werden wie folgt auf übliche Weise hergestellt:

Zusammensetzung:

| | |
|---|---|
| cis-1-Aethyl-2,3,5,6,11,11b-hexahydro-1H-indolizino-[8,7-b]indol-1-propionsäuremethylester-methansulfonat | 2500 mg |
| Talkum | 200 mg |
| Kolloidale Kieselsäure | 50 mg |

Herstellung:
Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse gefüllt.

Beispiel 24: 50,0 g cis-1-Aethyl-2,3,5,6,11,11b-hexahydro-1H-indolizino[8,7-b]indol-1-propionsäuremethylester-methansulfonat werden in destilliertem Wasser gelöst, die Lösung auf 5'000 ml aufgefüllt, und die sterilisierte Lösung in Ampullen von 5 ml abgefüllt, worin 1 ml 10 mg Wirkstoff enthält.

Beispiel 25: Anstelle der in den Beispielen 22 bis 24 als aktive Substanz verwendeten Verbindungen können auch folgende Verbindungen der Formel I, deren N-Oxide oder pharmazeutisch annehmbare nicht-toxische Säureadditionssalze als Wirkstoffe in Tabletten, Dragées, Kapseln, Ampullenlösungen etc. verwendet werden:

threo-cis-α-Hydroxy-1-äthyl-2,3,5,6,11,11b-hexahydro-1H-indolizino[8,7-b]indol-1-propionsäuremethylester,

erythro-cis-α-Hydroxy-1-äthyl-2,3,5,6,11,11b-hexahydro-1H-indolizino[8,7-b]indol-1-propionsäuremethylester,

α-Acetoxy-1-äthyl-2,3,5,6,11,11b-hexahydro-1H-indolizino[8,7-b]indol-1-propionsäuremethylester,

trans-1-Aethyl-2,3,5,6,11,11b-hexahydro-1H-indolizino[8,7-b]indol-1-propionsäuremethylester,

cis-1-Aethyl-2,3,5,6,11,11b-hexahydro-1H-indolizino[8,7-b]indol-1-propionsäure,

cis-1-Aethyl-2,3,5,6,11,11b-hexahydro-1H-indolizino[8,7-b]indol-1-essigsäuremethylester,

cis-3'-[1-Aethyl-2,3,5,6,11,11b-hexahydro-1H-indolizino[8,7-b]indol-1]-propan-1',2'-diol vom Smp. 224-225°,

cis-3'-[1-Aethyl-2,3,5,6,11,11b-hexahydro-1H-indolizino[8,7-b]indol-1]-propan-1',2'-diol vom Smp. 206-208°,

trans-3'-[1-Aethyl-2,3,5,6,11,11b-hexahydro-1H-indolizino[8,7-b]indol-1]-propan-1',2'-diol,

trans-α-Hydroxy-1-äthyl-2,3,5,6,11,11b-hexahydro-1H-indolizino[8,7-b]-indol-1-propionsäuremethylester,

trans-α-Amino-1-äthyl-2,3,5,6,11,11b-hexahydro-1H-indolizino[8,7-b]-indol-1-propionsäuremethylester,

cis-α-Amino-1-äthyl-2,3,5,6,11,11b-hexahydro-1H-indolizino[8,7-b]indol-1-propionsäuremethylester,

threo-cis-2-Benzoyloxy-1-äthyl-2,3,5,6,11,11b-hexahydro-1H-indolizino[8,7-b]indol-1-propionsäuremethylester,

erythro-cis-α-Benzoyloxy-1-äthyl-2,3,5,6,11,11b-hexahydro-1H-indolizino[8,7-b]indol-1-propionsäuremethylester,

cis-1-Aethyl-2,3,5,6,11,11b-hexahydro-1H-indolizino[8,7-b]indol-1-propionsäureamid,

cis-α-Amino-1-äthyl-2,3,5,6,11,11b-hexahydro-1-H-indolizino[8,7-b]indol-1-propionsäureamid,

sowie die im Beispiel 21 genannten Verbindungen, oder deren N-Oxide oder pharmazeutisch annehmbaren, nicht-toxischen Säureadditionssalze.

Patentansprüche

1. Pharmazeutische Präparate enthaltend Derivate des Indolizino[8,7-b]indols der Formel

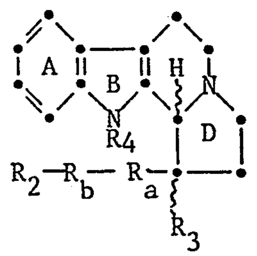

(I),

worin $R_a$ die Gruppe $-(CH_2)_m-$, worin m Null oder 1 ist, $R_b$ die Gruppe $-CH(R_1)-$ darstellt, worin $R_1$ Wasserstoff, Niederalkyl, gegebenenfalls verestertes oder veräthertes Hydroxy, Niederalkylthio, oder gegebenenfalls substituiertes Amino, $R_2$ gegebenenfalls verestertes oder veräthertes Hydroxymethyl oder gegebenenfalls funktionell abgewandeltes Carboxy, $R_3$ und $R_4$ jeweils Wasserstoff oder Niederalkyl bedeutet, oder anstelle der Gruppe $-(R_b-R_a)-$ die Gruppe -CH=CH- steht, und worin der carbocyclische Ring A unsubstituiert ist, oder unsubstituiertes oder substituiertes Niederalkyl, gegebenenfalls verestertes oder veräthertes Hydroxy, Nitro und/oder gegebenenfalls substituiertes Amino als Substituenten enthält, als Epimerengemische, reine Epimere, Racematgemische, Racemate, optische Antipoden, Gemische von cis- und trans-Isomeren, reine cis- oder trans-Isomere, N-Oxide oder Salze, insbesondere Säureadditionssalze, vor allem pharmazeutisch verwendbare, nichttoxische Säureadditionssalze davon, oder solche Verbindungen als quaternäre Ammoniumverbindungen oder deren Salze.

2. Pharmazeutische Präparate enthaltend Verbindungen der Formel I gemäss Anspruch 1, worin $R_a$, $R_b$ und m sowie die Gruppe der Formel $-(R_b-R_a)-$ die angegebenen Bedeutungen haben, $R_1$ Wasserstoff, Niederalkyl, Hydroxy, Halogen, Aroyloxy, Niederalkanoyloxy, Niederalkoxy, Phenylniederalkoxy, Niederalkylthio, Amino, N-Niederalkylamino, N,N-Diniederalkylamino, Niederalkylenamino, Oxaniederalkylenamino, Thianiederalkylenamino, Azaniederalkylenamino, worin das Azastickstoffatom unsubstituiert oder durch Niederalkyl substituiert sein kann, oder Niederalkanoylamino darstellt, $R_2$ für Hydroxymethyl, Aroyloxymethyl, Benzolsulfonyloxymethyl, Carboxy, Niederalkoxycarbonyl, Hydroxyniederalkoxycarbonyl, Niederalkoxy-niederalkoxycarbonyl, Halogenniederalkoxycarbonyl, Aminoniederalkoxycarbonyl, Niederalkylamino-niederalkoxycarbonyl, Diniederalkylamino-niederalkoxycarbonyl, Niederalkylenamino-niederalkoxycarbonyl, Oxaniederalkylen-aminoniederalkoxycarbonyl, Thianiederalkylenamino-niederalkoxycarbonyl, oder Azaniederalkylenamino-niederalkoxycarbonyl, worin das Azastickstoffatom unsubstituiert oder durch Niederalkyl substituiert sein kann, Niederalkylthiocarbonyl, gegebenenfalls durch Niederalkyl oder Niederalkoxy 1 bis 3-fach substituiertes Cycloalkyloxycarbonyl, Benzofuranyloxycarbonyl, 1,3-Dihydro-1-oxo-3-isobenzofuranyloxycarbonyl, Carbamoyl, N-Niederalkyl-carbamoyl, N,N-Diniederalkyl-carbamoyl, Niederalkylenamino-carbonyl, Oxaniederalkylenamino-carbonyl, Thianiederalkylenamino-carbonyl oder Azaniederalkylenamino-carbonyl steht, worin das Azastickstoffatom unsubstituiert oder durch Niederalkyl substituiert sein kann, Hydrazinocarbonyl, N,N'-Niederalkylhydrazinocarbonyl, N,N'-Niederalkanoylhydrazinocarbonyl, gegebenenfalls im Phenylteil durch Niederalkyl, Niederalkoxy, Halogen und/oder Nitro substituiertes N,N'-Benzoylhydrazinocarbonyl, N,N'-Nicotinoylhydrazinocarbonyl, oder die Cyanogruppe, $R_3$ und $R_4$ jeweils Wasserstoff oder Niederalkyl bedeuten, und worin der carbocyclische Ring A unsubstituiert ist oder Niederalkyl, Halogenniederalkyl, Hydroxyniederalkyl, Niederalkoxyniederalkyl, Hydroxy, Niederalkoxy, Phenylniederalkoxy, Niederalkyliden- oder Niederalkylendioxy, Halogen, Niederalkanoyloxy, Nitro, Amino, N-Niederalkylamino, N,N-Diniederalkylamino, Niederalkylenamino, Oxaniederalkylenamino, Thianiederalkylenamino, Azaniederalkylenamino, worin das Azastickstoffatom unsubstituiert oder durch Niederalkyl substituiert sein kann, und/oder Niederalkanoylamino jeweils 1-3fach als Substituenten enthält, als Epimerengemische, reine Epimere, Racematgemische, Racemate, optische Antipoden, Gemische von <u>cis</u>- und <u>trans</u>-Isomeren,

reine cis- oder trans-Isomere, N-Oxide oder Salze, insbesondere Säureadditionssalze, vor allem pharmazeutisch verwendbare, nicht-toxische Säureadditionssalze davon, oder solche Verbindungen als quaternäre Ammoniumverbindungen oder deren Salze.

3. Pharmazeutische Präparate enthaltend Verbindungen der Formel I gemäss Anspruch 1, worin $R_a$, $R_b$ und m sowie die Gruppe der Formel $-(R_b-R_a)-$ die angegebenen Bedeutungen haben, $R_1$ Wasserstoff, Hydroxy, Niederalkoxy, Aroyloxy, Niederalkanoyloxy, Amino, N-Niederalkylamino, N,N-Diniederalkylamino oder Niederalkanoylamino bedeutet, $R_2$ Hydroxymethyl, Aroyloxymethyl, Carboxy, Niederalkoxycarbonyl, Niederalkoxyniederalkoxycarbonyl, Niederalkylthiocarbonyl, Diniederalkylaminoniederalkoxycarbonyl, Carbamoyl, N-Niederalkylcarbamoyl, Hydrazinocarbonyl, N,N'-Benzoylhydrazinocarbonyl, N,N'-Nicotinoylhydrazinocarbonyl oder die Cyanogruppe, oder N,N-Diniederalkyl-carbamoyl darstellt, $R_3$ und $R_4$ jeweils Wasserstoff oder Niederalkyl ist, und worin der carbocyclische Ring A unsubstituiert oder durch Niederalkyl, Trifluormethyl, Hydroxy, Niederalkoxy, Phenylniederalkoxy, Niederalkylendioxy, Halogen, Niederalkanoyloxy, Nitro, Amino und/oder Niederalkanoylamino jeweils 1-3fach substituiert ist, wobei Niederalkoxy, Niederalkyl, Niederalkanoyl und Niederalkanoylamino jeweils bis und mit 4 Kohlenstoffatome enthalten,als Epimerengemische, reine Epimere, Racematgemische, Racemate, optische Antipoden, Gemische von cis- und trans-Isomeren, reine cis- oder trans-Isomere, N-Oxide oder Salze, insbesondere Säureadditionssalze, vor allem pharmazeutisch verwendbare, nicht-toxische Säureadditionssalze davon, oder solche Verbindungen als quaternäre Ammoniumverbindungen, worin die quartäre Gruppe Niederalkyl, Hydroxyniederalkyl, Halogenniederalkyl, Phenylniederalkyl oder Phenoxyniederalkyl ist, oder deren Salze.

4. Pharmazeutische Präparate enthaltend Verbindungen der Formel I gemäss Anspruch 1, worin $R_a$ , $R_b$ und m sowie die Gruppe der Formel $-(R_b-R_a)-$ die angegebenen Bedeutungen haben, $R_1$ Wasserstoff, Hydroxy, Benzoyloxy, Niederalkanoyloxy oder Amino bedeutet, $R_2$ für Hydroxymethyl,

im aromatischen Teil gegebenenfalls durch Niederalkyl, Niederalkoxy, Halogen oder funktionell abgewandeltes Carboxy substituiertes Benzoyloxymethyl, Carboxy, Niederalkoxycarbonyl, wie Methoxycarbonyl oder Aethoxycarbonyl, Niederalkoxy-niederalkoxycarbonyl wie Methoxy-methoxycarbonyl, Niederalkylthiocarbonyl, wie Aethylthiocarbonyl, Carbamoyl, Hydrazinocarbonyl, oder die Cyanogruppe steht, $R_3$ und $R_4$ jeweils Wasserstoff oder Niederalkyl bedeuten, und worin der carbocyclische Ring A unsubstituiert oder, vorzugsweise in den Stellungen 8 und/oder 9, durch Niederalkyl, z.B. Methyl, Hydroxy, Niederalkoxy, z.B. Methoxy, Phenylniederalkoxy, z.B. Benzyloxy, Niederalkylendioxy, z.B. Methylendioxy, und/oder Halogen, z.B. Brom, substituiert ist, wobei Niederalkyl und Niederalkoxy jeweils bis und mit vier Kohlenstoffatome enthalten, als Epimerengemische, reine Epimere, Racematgemische, Racemate, optische Antipoden, Gemische von cis- und trans-Isomeren, reine cis- oder trans-Isomere, N-Oxide oder Salze, insbesondere Säureadditionssalze, vor allem pharmazeutisch verwendbare, nicht-toxische Säureadditionssalze davon, oder solche Verbindungen als quaternäre Ammoniumverbindungen, worin die quartäre Gruppe Niederalkyl oder Phenylniederalkyl ist, oder deren Salze.

5. Pharmazeutische Präparate enthaltend Verbindungen der Formel I gemäss Anspruch 1, worin $R_a$ die Gruppe der Formel $-(CH_2)_m-$ bedeutet, wobei m für 1 steht, $R_b$ die Gruppe der Formel $-CH(R_1)-$ darstellt, wobei $R_1$ Wasserstoff, Hydroxy, Benzoyloxy oder Acetyloxy ist, $R_2$ Hydroxymethyl, gegebenenfalls durch Niederalkyl, wie Methyl, oder Niederalkoxy, wie Methoxy, oder Carboxy substituiertes Benzoylmethyl, z.B. 3,4,5-Trimethoxybenzoylmethyl, Carboxy, Niederalkoxycarbonyl, insbesondere Methoxycarbonyl oder Aethoxycarbonyl, Niederalkoxy-niederalkoxycarbonyl, wie Methoxy-methoxycarbonyl, Niederalkylthiocarbonyl wie Aethylthiocarbonyl, Carbamoyl oder die Cyanogruppe bedeutet, $R_3$ Wasserstoff oder Niederalkyl und $R_4$ Wasserstoff bedeutet, und worin der carbocyclische Ring A unsubstituiert, oder in 8- und/oder 9-Stellung durch Hydroxy oder Niederalkoxy, z.B. Methoxy, oder Methylendioxy und/oder Halogen, wie Brom, substituiert ist, wobei in diesen Verbindungen das Wasserstoffatom in 11b-Stellung und der Rest $R_3$, insbesondere Aethyl, in

1-Stellung in der trans- oder vorzugsweise in der cis-Konfiguration zueinander stehen, als Epimerengemische, reine Epimere, Racematgemische, Racemate, optische Antiopoden, Gemische von cis- und trans-Isomeren, reine cis- oder trans-Isomere, N-Oxide oder Salze, insbesondere Säureadditionssalze, vor allem pharmazeutisch verwendbare Säureadditionssalze davon, oder solche Verbindungen als quaternäre Ammoniumverbindungen, worin die quartäre Gruppe Niederalkyl, z.B. Methyl ist, oder deren Salze.

6. Pharmazeutische Präparate enthaltend als Verbindungen der Formel I gemäss Anspruch 1 die in den Beispielen beschriebenen neuen Verbindungen der Formel I.

7. Verbindungen der Formel I gemäss Anspruch 1, worin $R_a$ die Gruppe $-(CH_2)_m-$, worin m Null oder 1 ist, $R_b$ die Gruppe $-CH(R_1)-$ darstellt, worin $R_1$ Wasserstoff, Niederalkyl, gegebenenfalls verestertes oder veräthertes Hydroxy, Niederalkylthio, oder gegebenenfalls substituiertes Amino, $R_2$ gegebenenfalls verestertes oder veräthertes Hydroxymethyl oder gegebenenfalls funktionell abgewandeltes Carboxy, $R_3$ und $R_4$ jeweils Wasserstoff oder Niederalkyl bedeutet, oder anstelle der Gruppe $-(R_b-R_a)-$ die Gruppe -CH=CH- steht, und worin der carbocyclische Ring A unsubstituiert ist, oder unsubstituiertes oder substituiertes Niederalkyl, gegebenenfalls verestertes oder veräthertes Hydroxy, Nitro und/oder gegebenenfalls substituiertes Amino als Substituenten enthält, mit der Massgabe, dass in Verbindungen der Formel I, in welchen $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ Hydroxymethyl oder Benzyloxymethyl, $R_3$ die Aethylgruppe und $R_4$ Wasserstoff ist, der carbocyclische Ring A unsubstituiert ist, und mit der weiteren Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Aethyl, $R_2$ Hydroxymethyl oder Benzyloxymethyl, und $R_3$ und $R_4$ jeweils Wasserstoff bedeuten, der carbocyclische Ring A unsubstituiert

ist, und mit der weiteren Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ die Carboxymethoxygruppe oder die Cyanogruppe bedeutet, und $R_3$ und $R_4$ jeweils Wasserstoff ist, der carbocyclische Ring A unsubstituiert ist, als Epimerengemische, reine Epimere, Racematgemische, Racemate, optische Antipoden, Gemische von cis- und trans-Isomeren, reine cis- oder trans-Isomere, N-Oxide oder Salze, insbesondere Säureadditionssalze, vor allem pharmazeutisch verwendbare, nicht-toxische Säureadditionssalze davon, oder solche Verbindungen als quaternäre Ammoniumverbindungen, oder deren Salze.

8. Verbindungen der Formel I gemäss Anspruch 1, worin $R_a$, $R_b$ und m sowie die Gruppe der Formel $-(R_b-R_a)-$ die angegebenen Bedeutungen haben, $R_1$ Wasserstoff, Niederalkyl, Hydroxy, Halogen, Aroyloxy, Niederalkanoyloxy, Niederalkoxy, Phenylniederalkoxy, Niederalkylthio, Amino, N-Niederalkylamino, N,N-Diniederalkylamino, Niederalkylenamino, Oxaniederalkylenamino, Thianiederalkylenamino, Azaniederalkylenamino, worin das Azastickstoffatom unsubstituiert oder durch Niederalkyl substituiert sein kann, oder Niederalkanoylamino darstellt, $R_2$ für Hydroxymethyl, Aroyloxymethyl, Benzolsulfonyloxymethyl, Carboxy, Niederalkoxycarbonyl, Hydroxyniederalkoxycarbonyl, Niederalkoxy-niederalkoxycarbonyl, Halogen-niederalkoxycarbonyl, Aminoniederalkoxycarbonyl, Niederalkylamino-niederalkoxycarbonyl, Diniederalkylamino-niederalkoxycarbonyl, Niederalkylenamino-niederalkoxycarbonyl, Oxaniederalkylenamino-niederalkoxycarbonyl, Thianiederalkylenamino-niederalkoxycarbonyl, oder Azaniederalkylenamino-niederalkoxycarbonyl, worin das Azastickstoffatom unsubstituiert oder durch Niederalkyl substituiert sein kann, Niederalkylthiocarbonyl, gegebenenfalls durch Niederalkyl oder Niederalkoxy 1 bis 3fach substituiertes Cycloalkyloxycarbonyl, Benzofuranyloxycarbonyl, 1,3-Dihydro-1-oxo-3-isobenzofuranyloxycarbonyl, Carbamoyl, N-Niederalkyl-carbamoyl, N,N-Diniederalkyl-carbamoyl, Niederalkylenamino-carbonyl, Oxaniederalkylenamino-carbonyl, Thianiederalkylenamino-carbonyl oder Azaniederalkylenamino-carbonyl steht, worin das Azastickstoffatom unsubstituiert oder durch Niederalkyl substituiert sein kann, Hydrazinocarbonyl, N,N'-Niederalkylhydrazinocarbonyl, N,N'-Niederalkanoylhydrazinocarbonyl, gegebenenfalls im Phenylteil durch Niederalkyl, Niederalkoxy, Halogen und/oder Nitro substituiertes N,N'-Benzoylhydrazinocarbonyl, N,N'-Nicotinoylhydrazinocarbonyl, oder die Cyanogruppe, $R_3$ und $R_4$ jeweils Wasserstoff oder Niederalkyl bedeuten, und worin der carbocyclische Ring A unsubstituiert ist oder Niederalkyl, Halogenniederalkyl, Hydroxyniederalkyl, Niederalkoxyniederalkyl, Hydroxy, Niederalkoxy, Phenylniederalkoxy, Niederalkyliden- oder Niederalkylendioxy, Halogen, Niederalkanoyloxy, Nitro, Amino, N-Niederalkylamino, N,N-Diniederalkylamino, Niederalkylenamino, Oxaniederalkylenamino, Thianiederalkylenamino, Azaniederalkylenamino, worin das Azastickstoffatom unsubstituiert oder durch Niederalkyl substituiert sein kann, und/oder Niederalkanoylamino jeweils 1-3fach als Substituenten enthält, mit der Massgabe, dass in Verbindungen der Formel I, in welchen $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ Hydroxymethyl, $R_3$ die Aethylgruppe und $R_4$ Wasserstoff ist, der carbocyclische Ring A unsubstituiert ist, und mit der weiteren Massgabe, dass in Verbindungen der Formel I, worin $R_a$ die Gruppe der Formel $-(CH_2)_m$ bedeutet, worin m für 1 steht, $R_b$ die Gruppe der Formel $-CH(R_1)-$darstellt, worin $R_1$ Aethyl, $R_2$ Hydroxymethyl und $R_3$ und $R_4$ jeweils Wasserstoff bedeuten, der carbocyclische Ring A unsubstituiert ist, und mit der weiteren Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ die Carboxymethoxygruppe oder die Cyanogruppe bedeutet, und $R_3$ und $R_4$ jeweils Wasserstoff ist, der carbocyclische Ring A unsubstituiert ist, als Epimerengemische, reine Epimere, Racematgemische, Racemate, optische Antipoden, Gemische von <u>cis</u>- und <u>trans</u>-Isomeren, reine <u>cis</u>- oder <u>trans</u>-Isomere, N-Oxide oder Salze, insbesondere Säureaddtionssalze, vor allem pharmazeutisch verwendbare, nicht-toxische Säureadditionssalze davon oder solche Verbindungen als quaternäre Ammoniumverbindungen oder deren Salze.

9. Verbindungen der Formel I gemäss Anspruch 1, worin $R_a$, $R_b$ und m sowie die Gruppe der Formel $-(R_b-R_a)-$ die angegebenen Bedeutungen haben, $R_1$ Wasserstoff, Hydroxy, Niederalkoxy, Aroyloxy, Niederalkanoyloxy, Amino, N-Niederalkylamino, N,N-Diniederalkylamino oder Niederalkanoylamino bedeutet, $R_2$ Hydroxymethyl, Aroyloxymethyl, Carboxy, Niederalkoxycarbonyl, Niederalkoxy-niederalkoxycarbonyl, Niederalkylthiocarbonyl, Diniederalkylaminoniederalkoxycarbonyl, Carbamoyl, N-Niederalkylcarbamoyl, Hydrazinocarbonyl, N,N'-Benzoylhydrazinocarbonyl, N,N'-Nicotinoylhydrazinocarbonyl oder die Cyanogruppe, oder N,N-Diniederalkyl-carbamoyl darstellt, $R_3$ und $R_4$ jeweils Wasserstoff oder Niederalkyl ist, und worin der carbocyclische Ring A unsubstituiert oder durch Niederalkyl, Trifluormethyl, Hydroxy, Niederalkoxy, Phenylniederalkoxy, Niederalkylendioxy, Halogen, Niederalkanoyloxy, Nitro, Amino und/oder Niederalkanoylamino jeweils 1-3fach substituiert ist, wobei Niederalkoxy, Niederalkyl, Niederalkanoyl und Niederalkanoylamino jeweils bis und mit 4 Kohlenstoffatome enthalten, mit der Massgabe, dass in Verbindungen der Formel I, in welcher $R_a$ die Gruppe der Formel $-(CH_2)_m-$ bedeutet, worin m für 1 steht, $R_b$ eine Gruppe der Formel $-CH(R_1)-$ darstellt, worin $R_1$ Wasserstoff ist, $R_2$ Hydroxymethyl, $R_3$ die Aethylgruppe und $R_4$ Wasserstoff ist, der carbocyclische Ring A unsubstituiert ist, und mit der weiteren Massgabe,
dass in Verbindungen der Formel I, worin $R_a$ die Gruppe der Formel $-(CH_2)_m-$ bedeutet, worin m für 1 steht, $R_b$ eine Gruppe der Formel $-CH(R_1)-$ darstellt, worin $R_1$ Wasserstoff ist, $R_2$ die Carbomethoxygruppe oder die Cyanogruppe bedeutet, und $R_3$ und $R_4$ jeweils Wasserstoff ist, der carbocyclische Ring A unsubstituiert ist, als Epimerengemische, reine Epimeren, Racematgemische, Racemate, optische Antipoden, Gemische von cis- und trans-Isomeren, reine cis- oder trans-Isomere, N-Oxide oder Salze, insbesondere Säureadditionssalze, vor allem

pharmazeutisch verwendbare, nicht-toxische Säureadditionssalze davon, oder solche Verbindungen als quaternäre Ammoniumverbindungen, worin die quaternäre Gruppe Niederalkyl, Hydroxyniederalkyl, Halogenniederalkyl, Phenylniederalkyl oder Phenoxyniederalkyl ist, oder deren Salze.

10. Verbindungen der Formel I gemäss Anspruch 1, worin $R_a$, $R_b$ und m sowie die Gruppe der Formel $-(R_b-R_a)-$ die angegebenen Bedeutungen haben, $R_1$ Wasserstoff, Hydroxy, Benzoyloxy, Niederalkanoyloxy oder Amino bedeutet, $R_2$ für Hydroxymethyl, im aromatischen Teil gegebenenfalls durch Niederalkyl, Niederalkoxy, Halogen oder funktionell abgewandeltes Carboxy substituiertes Benzoyloxymethyl, Carboxy, Niederalkoxycarbonyl, wie Methoxycarbonyl oder Aethoxycarbonyl, Niederalkoxyniederalkoxycarbonyl wie Methoxy-methoxycarbonyl, Niederalkylthiocarbonyl, wie Aethylthiocarbonyl, Carbamoyl, Hydrazinocarbonyl oder die Cyanogruppe steht, $R_3$ und $R_4$ jeweils Wasserstoff oder Niederalkyl bedeuten, und worin der carbocyclische Ring A unsubstituiert oder, vorzugsweise in den Stellungen 8 und/oder 9, durch Niederalkyl, z.B. Methyl, Hydroxy, Niederalkoxy, z.B. Methoxy, Phenylniederalkoxy, z.B. Benzyloxy, Niederalkylendioxy, z.B. Methylendioxy, und/oder Halogen, z.B. Brom, substituiert ist, wobei Niederalkyl und Niederalkoxy jeweils bis und mit vier Kohlenstoffatome enthalten, mit der Massgabe, dass in Verbindungen der Formel I, in welchen $R_a$ die Gruppe der Formel $-(CH_2)_m-$ bedeutet, worin m für 1 steht, $R_b$ eine Gruppe der Formel $-CH(R_1)-$ darstellt, worin $R_1$ Wasserstoff ist, $R_2$ Hydroxymethyl, $R_3$ die Aethylgruppe und $R_4$ Wasserstoff ist, der carbocyclische Ring A unsubstituiert ist, und mit der weiteren Massgabe, dass in Verbindungen der Formel I, in welchen $R_a$ die Gruppe der Formel $-(CH_2)_m-$ bedeutet, worin m für 1 steht, $R_b$ eine Gruppe der Formel $-CH(R_1)-$ darstellt, worin $R_1$ Wasserstoff ist, $R_2$ die Carbomethoxygruppe oder die Cyanogruppe bedeutet, $R_3$ und $R_4$ jeweils Wasserstoff sind, der carbocyclische Ring A unsubstituiert ist, als Epimerengemische, reine Epimere, Racematgemische, Racemate, optische Antipoden, Gemische von cis- und trans-Isomeren, reine cis- oder trans-Isomere, N-Oxide oder Salze, insbesondere Säureadditionssalze, vor allem pharmazeutisch verwendbare, nicht-toxische Säureadditionssalze davon, oder solche Verbindungen als quaternäre Ammoniumverbindungen, worin die quaternäre Gruppe Niederalkyl oder Phenylniederalkyl ist, oder deren Salze.

11. Verbindungen der Formel I gemäss Anspruch 1, worin $R_a$ die Gruppe der Formel $-(CH_2)_m-$ bedeutet, wobei m für 1 steht, $R_b$ die Gruppe der Formel $-CH(R_1)-$ darstellt, wobei $R_1$ Wasserstoff oder Hydroxy, Benzoyloxy oder Acetyloxy ist, $R_2$ Hydroxymethyl, gegebenenfalls durch Niederalkyl, wie Methyl, oder Niederalkoxy, wie Methoxy oder Carboxy substituiertes Benzoyloxymethyl, z.B. 3,4,5-Trimethoxybenzoyloxymethyl, Carboxy, Niederalkoxycarbonyl, insbesondere Methoxycarbonyl oder Aethoxycarbonyl, Niederalkoxy-niederalkoxycarbonyl wie Methoxymethoxycarbonyl, Niederalkylthiocarbonyl wie Aethylthiocarbonyl, Carbamoyl oder die Cyanogruppe bedeutet, $R_3$ Wasserstoff oder Niederalkyl und $R_4$ Wasserstoff bedeutet, und worin der carbocyclische Ring A unsubstituiert oder in 8- und/oder 9-Stellung durch Hydroxy oder Niederalkoxy, z.B. Methoxy, oder Methylendioxy und/oder Halogen, wie Brom, substituiert ist, wobei in diesen Verbindungen das Wasserstoffatom in 11b-Stellung und der Rest $R_3$, insbesondere Aethyl, in der trans- oder vorzugsweise in der cis-Konfiguration zueinander stehen, mit der Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ Hydroxymethyl, $R_3$ die Aethylgruppe und $R_4$ Wasserstoff ist, der carbocyclische Ring A unsubstituiert ist, und mit der weiteren Massgabe, dass in Verbindungen der Formel I, in welchen $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ Methoxycarbonyl oder die Cyanogruppe bedeutet, $R_3$ und $R_4$ jeweils Wasserstoff ist, der carbocyclische Ring A unsubstituiert ist, als Epimerengemische, reine Epimere, Racematgemische, Racemate, optische Antipoden, Gemische von cis- und trans-Isomeren, reine cis oder trans-Isomere, N-Oxide oder Salze, insbesondere Säureadditionssalze,

vor allem pharmazeutisch verwendbare, nicht-toxische Säureadditionssalze davon, oder solche Verbindungen als quaternäre Ammoniumverbindungen, worin die quaternäre Gruppe Niederalkyl, z.B. Methyl ist, oder deren Salze.

12. Verbindungen der Formel I gemäss Anspruch 1, worin $R_a$ die Gruppe der Formel $-(CH_2)_m-$ bedeutet, wobei m für 1 steht, $R_b$ die Gruppe der Formel $-CH(R_1)-$ darstellt, wobei $R_1$ Wasserstoff, Hydroxy, Niederalkanoyloxy, wie Acetyloxy, oder Aroyloxy, wie Benzoyloxy ist, $R_2$ Carbamoyl, Carboxy oder Niederalkoxycarbonyl, wie Methoxycarbonyl, bedeutet, $R_3$ Niederalkyl, wie Aethyl, und $R_4$ Wasserstoff darstellt, und worin der carbocyclische Ring A unsubstituiert oder in 8- und/oder 9-Stellung durch Halogen, wie Brom, substituiert ist, wobei in diesen Verbindungen das Wasserstoffatom in 11b-Stellung und der Rest $R_3$, insbesondere Aethyl, in der trans-, vorzugsweise jedoch in der cis-Konfiguration zueinanderstehen und eine Hydroxygruppe $R_1$ in der threo-, vorzugsweise jedoch in der erythro-Form, bezogen auf die durch das in 11b-Stellung stehende Wasserstoffatom und die Niederalkylgruppe $R_3$, insbesondere Aethyl, gebildete cis-Konfiguration steht, als Epimerengemische, reine Epimere, Racematgemische, Racemate, optische Antipoden oder Salze, insbesondere Säureadditionssalze, vor allem pharmazeutisch verwendbare, nicht-toxische Säureadditionssalze davon.

13. threo-cis-α-Hydroxy-1-äthyl-2,3,5,6,11,11b-hexahydro-1H-indolizino[8,7-b]indol-1-propionsäuremethylester.

14. α-Acetoxy-1-äthyl-2,3,5,6,11,11b-hexahydro-1H-indolizino[8,7-b] indol-1-propionsäuremethylester.

15. cis-1-Aethyl-2,3,5,6,11,11b-hexahydro-1H-indolizino[8,7-b]indol-1-propionsäuremethylester.

16. trans-1-Aethyl-2,3,5,6,11,11b-hexahydro-1H-indolizino[8,7-b]indol-1-propionsäuremethylester.

17. cis-1-Aethyl-2,3,5,6,11,11b-hexahydro-1H-indolizino[8,7-b]indol-1-propionsäure.

18. cis-1-Aethyl-2,3,5,6,11,11b-hexahydro-1H-indolizino[8,7-b]indol-1-propionsäuremethoxymethylester.

19. cis-1-Aethyl-2,3,5,6,11,11b-hexahydro-1H-indolizino[8,7-b]indol-1-essigsäure.

20. cis-1-Aethyl-2,3,5,6,11,11b-hexahydro-1H-indolizino[8,7-b]indol-1-essigsäuremethylester.

21. threo-3-[1-Aethyl-2,3,5,6,11,11b-hexahydro-1H-indolizino[8,7-b]-indol]-propan-1',2'-diol.

22. erythro-cis-3'-[1-Aethyl-2,3,5,6,11,11b-hexahydro-1H.indolizino[8,7-b]indol-1]-propan-1',2'-diol.

23. trans-3'-[1-Aethyl-2,3,5,6,11,11b-hexahydro-1H-indolizino[8,7-b]-indol-1]-propan-1',2'-diol.

24. cis-α-Amino-1-äthyl-2,3,5,6,11,11b-hexahydro-1H-indolizino[8,7-b]-indol-1-propionsäuremethylester als Isomeres vom Smp. 143-146°.

25. trans-α-Amino-1-äthyl-2,3,5,6,11,11b-hexahydro-1H-indolizino-[8,7-b]indol-1-propionsäuremethylester.

26. erythro-cis-α-Hydroxy-1-äthyl-2,3,5,6,11,11b-hexahydro-1H-indolizino[8,7-b]indol-1-propionsäuremethylester.

27. trans-α-Hydroxy-1-äthyl-2,3,5,6,11,11b-hexahydro-1H-indolizino-[8,7-b]indol-1-propionsäuremethylester.

28. threo-cis-α-Benzoyloxy-1-äthyl-2,3,5,6,11,11b-hexahydro-1H-indolizino[8,7-b]-indol-1-propionsäuremethylester.

29. erythro-cis-α-Benzoyloxy-1-äthyl-2,3,5,6,11,11b-hexahydro-1H-indolizino[8,7-b]indol-1-propionsäuremethylester.

30. cis-1-Aethyl-2,3,5,6,11,11b-hexahydro-1H-indolizino[8,7-b]indol-1-propionsäureamid.

31. cis-α-Amino-1-äthyl-2,3,5,6,11,11b-hexahydro-1H-indolizino[8,7-b]-indol-1-propionsäureamid.

32. Salze von Verbindungen der Ansprüche 13-31.

33. Pharmazeutisch verwendbare, nicht toxische Säureadditionssalze von Verbindungen der Ansprüche 13-31.

34. Verbindungen der Formel I zur Anwendung in einem Verfahren zur therapeutischen Behandlung des menschlichen oder tierischen Körpers.

35. Verbindungen der Formel I als Mittel zur Bekämpfung von Vigilanzstörungen und zerebraler Leistungsinsuffizienz, insbesondere von Gedächtnisstörungen verschiedener Genese, wie senile Demenz, Multiinfarkt-Demenz oder Demenz vom Alzheimer-Typ, ferner von Folgezuständen von Hirntraumen oder Apoplexie.

36. Pharmazeutische Präparate enthaltend Verbindungen der Formel I gemäss den Ansprüchen 7-31 und 33.

37. Verwendung von Verbindungen der Formel I zur Herstellung von pharmazeutischen Präparaten.

38. Verfahren zur Herstellung von pharmazeutischen Präparaten, dadurch gekennzeichnet, dass man Verbindungen der Formel I gemäss den Ansprüchen 1-31 und 33, gegebenenfalls unter Beimischung von Hilfsstoffen, zu pharmazeutischen Präparaten verarbeitet.

39. Verfahren zur Herstellung von neuen Derivaten des Indolizino[8,7-b] indols der Formel

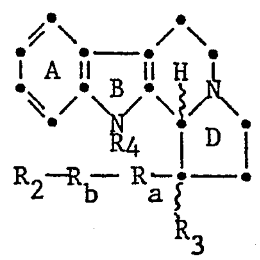

(I),

worin $R_a$ die Gruppe $-(CH_2)_m-$, worin m Null oder 1 ist, $R_b$ die Gruppe $-CH(R_1)-$ darstellt, worin $R_1$ Wasserstoff, Niederalkyl, gegebenenfalls verestertes oder veräthertes Hydroxy, Niederalkylthio, oder gegebenenfalls substituiertes Amino, $R_2$ gegebenenfalls verestertes oder veräthertes Hydroxymethyl oder gegebenenfalls funktionell abgewandeltes Carboxy, $R_3$ und $R_4$ jeweils Wasserstoff oder Niederalkyl bedeutet, oder anstelle

der Gruppe $-(R_b-R_a)-$ die Gruppe -CH=CH- steht, und worin der carbocyclische Ring A unsubstituiert ist, oder unsubstituiertes oder substituiertes Niederalkyl, gegebenenfalls verestertes oder veräthertes Hydroxy, Nitro und/oder gegebenenfalls substituiertes Amino als Substituenten enthält, mit der Massgabe, dass in Verbindungen der Formel I, in welchen $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ Hydroxymethyl, oder Benzyloxymethyl, $R_3$ die Aethylgruppe und $R_4$ Wasserstoff ist, der carbocyclische Ring A unsubstituiert ist, und mit der weiteren Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Aethyl, $R_2$ Hydroxymethyl oder Benzyloxymethyl, und $R_3$ und $R_4$ jeweils Wasserstoff bedeuten, der carbocyclische Ring A unsubstituiert ist, und mit der weiteren Massgabe, dass in Verbindungen der Formel I, worin $R_a$ die Gruppe der Formel $-(CH_2)_m-$ bedeutet, worin m für 1 steht, $R_b$ die Gruppe der Formel $-CH(R_1)-$ darstellt, worin $R_1$ Wasserstoff ist, $R_2$ die Carbomethoxygruppe oder die Cyanogruppe bedeutet, und $R_3$ und $R_4$ jeweils Wasserstoff sind, der carbocyclische Ring A unsubstituiert ist, als Epimerengemische, reine Epimere, Racematgemische, Racemate, optische Antipoden, Gemische von cis- und trans-Isomeren, reine cis- oder trans-Isomere, N-Oxide oder Salze davon, dadurch gekennzeichnet, dass man

a) in einer Verbindung der Formel

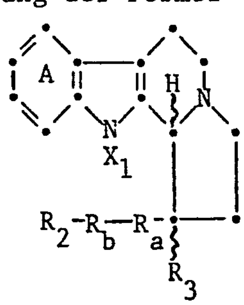

(II),

worin $X_1$ Wasserstoff oder einen abspaltbaren und durch Wasserstoff ersetzbaren Substituenten oder Niederalkyl bedeutet und gegebenenfalls Hydroxy- und/oder Aminogruppen durch einen abspaltbaren und durch Wasserstoff ersetztbaren Rest substituiert sind, mit der Massgabe, dass mindestens $X_1$ einen abspaltbaren und durch Wasserstoff ersetzbaren Substituenten bedeutet, oder mindestens Hydroxy- und/oder Aminogruppen durch einen abspaltbaren und durch Wasserstoff ersetzbaren Substituenten substituiert sind, oder in einem Salz davon, die von Wasserstoff verschiedene Gruppe $X_1$ und/oder Hydroxy- und/oder Aminogruppen substituierende, abspaltbare und durch Wasserstoff ersetzbare Substituenten abspaltet und durch Wasserstoff ersetzt, oder

b) in einer Verbindung der Formel

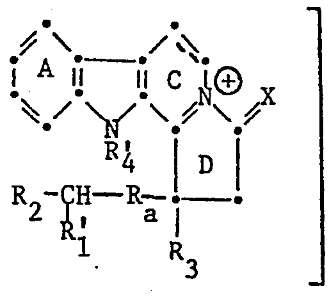

$M^{\ominus}$ (IV),

worin $M^{\ominus}$ das Anion einer Säure, z.B. das der Perchlorsäure ist, die Gruppe $>C=X$ für die Gruppe $>C=S$ oder $>CH_2$ steht, $R_1'$ die Bedeutung von $R_1$ hat, oder für eine durch eine Schutzgruppe geschützte, mittels Reduktion in eine freie Hydroxygruppe umwandelbare Hydroxygruppe steht, oder $R_1'$ einen Niederalkenylrest darstellt, oder gegebenenfalls anstelle der Gruppe $-CH(R_1')-$ der Rest der Formel $>C=CHR_1''$ (IVa), steht, worin $R_1''$ Wasserstoff oder einen ein Kohlenstoffatom weniger aufweisenden Niederalkylrest bedeutet, oder anstelle der Gruppe $-CH(R_1')-R_a-$ die Gruppe -CH=CH- (IVb) steht, und $R_4'$ Wasserstoff oder einen Niederalkenylrest bedeutet, die den Ringen C und D gemeinsamen Doppelbindung zur Kohlenstoff-Stickstoff-Einfachbindung, einen gegebenenfalls vorhandenen Niederalkenylrest $R_1'$ und/oder $R_4'$ zur Niederalkylgruppe $R_1$

bzw. $R_4$ und/oder gegebenenfalls die Gruppe IVa zur Gruppe $-\underset{R_1}{CH}-$,
und/oder gegebenenfalls die Gruppe IVb zur Gruppe $-CH_2-CH_2-$ (IVc), und/oder eine im Ring C gegebenenfalls vorhandene Doppelbindung zur Kohlenstoff-Kohlenstoff-Einfachbindung und/oder eine gegebenenfalls vorhandene Gruppe $>C=S$ zur Gruppe $>CH_2$ reduziert, und an Hydroxy- und/oder Aminogruppen gegebenenfalls stehende, unter den Bedingungen der Reduktion abspaltbare und durch Wasserstoff ersetzbare Schutzgruppen abspaltet und durch Wasserstoff ersetzt, oder,

c) eine Verbindung der Formel

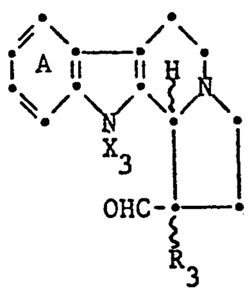

(V),

worin $X_3$ Niederalkyl oder eine unter den Bedingungen des Verfahrens abspaltbare und durch Wasserstoff ersetzbare Gruppe bedeutet, und Hydroxy- und/oder Aminogruppen gegebenenfalls in geschützter, z.B. mittels Solvolyse, wie Hydrolyse, spaltbarer Form vorliegen, mit einer Verbindung der Formel

$$X_o - CH_2 - R_2' \qquad \text{(Va)}$$

worin $X_o$ den Rest der Formel 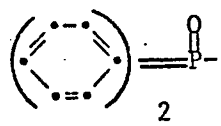 (Vb), oder den Rest der Formel 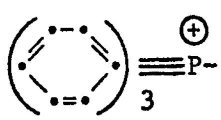 (Vc) bedeutet, der in quaternärer Form vorliegt, oder $X_o$ den Rest der Formel $(R_5O)_2\overset{O}{\overset{\|}{P}}-$ (Vd) darstellt, worin $R_5$ Niederalkyl, z.B. Aethyl ist, oder $X_o$ Halogen bedeutet, und $R_2'$ gegebenenfalls verestertes oder amidiertes Carboxy darstellt, umsetzt, und eine erhaltene Verbindung durch gleichzeitige oder nachfolgende Abspaltung der abspaltbaren und durch Wasserstoff ersetzbaren Gruppe $X_3$

und gegebenenfalls vorhandener Hydroxy- und/oder Aminoschutzgruppen in eine entsprechende Verbindung der Formel I umwandelt, worin anstelle der Gruppe $-(R_b-R_a)-$ der Rest -CH=CH- steht, oder

d) in einer Verbindung der Formel

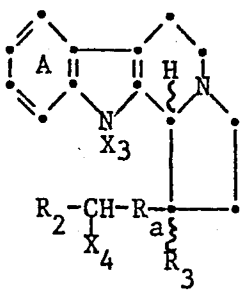

(VI),

worin $X_3$ Niederalkyl oder eine unter den Bedingungen des Verfahrens abspaltbare und durch Wasserstoff ersetzbare Gruppe bedeutet, und $X_4$ Halogen oder eine mit einer organischen Sulfonsäure veresterte Hydroxygruppe bedeutet, die Gruppe $X_4$ abspaltet und durch der Bedeutung von $R_1$ entsprechendes, gegebenenfalls substituiertes Amino, gegebenenfalls verestertes oder veräthertes Hydroxy oder Niederalkylthio ersetzt und gleichzeitig oder nachfolgend eine abspaltbare und durch Wasserstoff ersetzbare Gruppe $X_3$ abspaltet und durch Wasserstoff ersetzt, oder

e) eine Verbindung der Formel

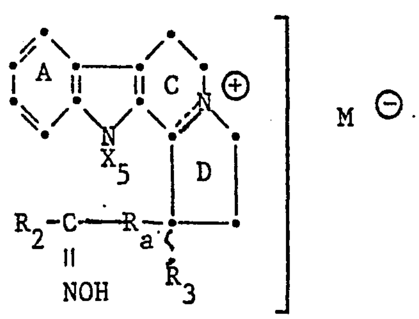

(VII),

worin $X_5$ Niederalkyl, Niederalkenyl oder eine unter den Bedingungen der Reduktion abspaltbare und durch Wasserstoff ersetzbare Gruppe bedeutet, die Gruppe $\rangle$C=NOH (VIIa) zur Gruppe $\rangle$CHNH$_2$ (VIIb), und eine gegebenenfalls vorhandene den Ringen C und D gemeinsame Doppelbindung, wobei in einem solchen Falle das den Ringen C und D gemeinsame N-Atom eine positive Ladung trägt und das Anion M die angegebene Bedeutung hat,

 

zur Kohlenstoff-Stickstoff-Einfachbindung, und/oder eine gegebenenfalls vorhandene Niederalkenylgruppe $X_5$ zur Niederalkylgruppe reduziert, und/oder eine gegebenenfalls vorhandene, unter den Bedingungen der Reduktion abspaltbare und durch Wasserstoff ersetzbare Gruppe $X_5$, und an Hydroxy- und/oder Aminogruppen gegebenenfalls stehende unter den Bedingungen der Reduktion abspaltbare und durch Wasserstoff ersetzbare Schutzgruppen abspaltet und durch Wasserstoff ersetzt, oder

f) in einer Verbindung der Formel

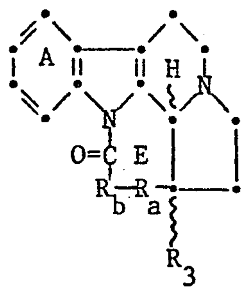

(VIII)

den Ring E mittels Alkoholyse, Aminolyse einschliesslich Ammonolyse oder Hydrolyse, unter Bildung einer gegebenenfalls veresterten oder amidierten Carboxygruppe $R_2$ öffnet, oder

g) in einer Verbindung der Formel

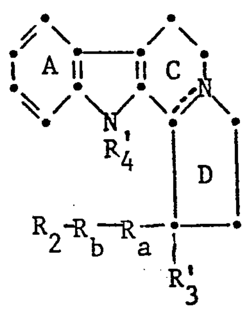

(IX),

worin $R_3'$ eine mittels Reduktion in eine Niederalkylgruppe $R_3$ überführbare Gruppe und $R_4'$ Wasserstoff oder Niederalkenyl darstellt, gegebenenfalls den Ringen C und D eine Doppelbindung gemeinsam ist, und in diesem Falle das dazugehörende N-Atom eine positive Ladung trägt und eine solche Verbindung als Salz vorliegt und Hydroxy- und/oder Aminogruppen gegebenenfalls durch mittels Reduktion abspaltbare und durch Wasserstoff ersetzbare Gruppen geschützt sind, die Gruppe $R_3'$ zur

Niederalkylgruppe $R_3$, eine gegebenenfalls vorhandene Niederalkenylgruppe $R_4'$ zur Niederalkylgruppe, eine gegebenenfalls vorhandene den Ringen C und D gemeinsame Doppelbindung zur Kohlenstoff-Stickstoff-Einfachbindung reduziert, und gegebenenfalls an Hydroxy- und/oder Aminogruppen stehende, unter den Bedingungen des Reduktionsverfahrens abspaltbare und durch Wasserstoff ersetzbare Schutzgruppen abspaltet und durch Wasserstoff ersetzt, oder

h) in einer Verbindung der Formel

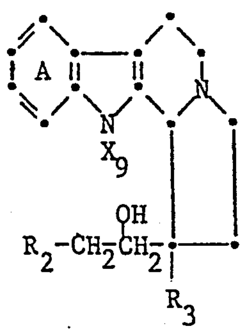

(X),

worin $X_9$ Wasserstoff, Niederalkyl oder eine unter den Bedingungen des Verfahrens abspaltbare und durch Wasserstoff ersetzbare Gruppe ist, die Gruppe der Formel $-CH_2-CH(OH)-$ mittels Wasserabspaltung in die Gruppe $-CH=CH-$ umwandelt, eine gegebenenfalls vorhandene unter den Bedingungen des Verfahrens abspaltbare und durch Wasserstoff ersetzbare Gruppe $X_9$ abspaltet und durch Wasserstoff ersetzt, gegebenenfalls vorhandene Hydroxy- und/oder Aminoschutzgruppen abspaltet und durch Wasserstoff ersetzt, oder

i) an eine Verbindung der Formel

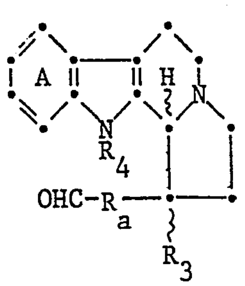

(XI)

Cyanwasserstoff oder eine in gleicher Weise reagierende, die zu bildende sekundäre Hydroxygruppe $R_1$ gegebenenfalls in geschützter Form

liefernde Verbindung anlagert, und die gebildete, gegebenenfalls in geschützter Form vorliegende sekundäre Hydroxygruppe in die freie Hydroxygruppe umwandelt, und, wenn erwünscht, eine erhaltene Verbindung der Formel I in eine andere Verbindung der Formel I umwandelt, und/oder, wenn erwünscht, ein erhaltenes Racematgemisch in die Racemate, oder ein erhaltenes Racemat in die optischen Antipoden, oder ein erhaltenes Gemisch von cis- und trans-Isomeren in die reinen cis- und trans-Isomeren, oder ein erhaltenes Epimerengemisch in die reinen Epimeren auftrennt, und/oder wenn erwünscht, eine erhaltene Verbindung der Formel I in ein Salz oder ein erhaltenes Salz in eine freie Verbindung oder ein anderes Salz oder ein N-Oxid überführt.

40. Die nach dem Verfahren des Anspruchs 39 erhältlichen Verbindungen.